# EUROPÄISCHE PATENTSCHRIFT

(11) **EP 0 328 026 B1**
(45) Veröffentlichungstag und Bekanntmachung des Hinweises auf die Patenterteilung: **28.04.1993**
(21) Anmeldenummer: 89102025.7
(22) Anmeldetag: 06.02.1989
(51) Int. Cl.: C07D 403/04, A61K 31/40

(54) **Substituierte Pyrrole**
Substituted pyrroles
Pyrroles substitués

(30) Priorität: 10.02.1988 GB 8803048; 25.11.1988 GB 8827565
(43) Veröffentlichungstag der Anmeldung: 16.08.1989
(73) Patentinhaber: F. HOFFMANN-LA ROCHE AG, 4002 Basel (CH)
(72) Erfinder: Davis, Peter David, Letchworth, Hertfordshire (GB); Hill, Christopher Huw, Knebworth, Hertfordshire (GB); Lawton, Geoffrey, Hitchin, Hertfordshire (GB)
(74) Vertreter: Lederer, Franz, Dr.

(56) Entgegenhaltungen:
- CHEMICAL ABSTRACTS, Band 104, Nr. 23, Juni 1986, Columbus, Ohio, USA, Seite 789, Spalte 1, Zusammenfassung Nr. 207579g
- CHEMICAL ABSTRACTS, Band 101, Nr. 7, 13. August 1984, Columbus, Ohio, USA, Seite 654, Spalte 2, Zusammenfassung Nr. 55460j
- CHEMICAL ABSTRACTS; Band 102, Nr. 1, 7. Januar 1985, Columbus, Ohio, USA, Seite 567, Spalte 1, Zusammenfassung Nr. 6236c
- HEMICAL ABSTRACTS, Band 98, Nr. 25, 20. Juni 1983, Columbus, Ohio, USA, Seite 585, Spalte 1, Zusammenfassung Nr. 215863t
- CHEMICAL ABSTRACTS, Band 93, Nr. 7, 18. August 1980, Columbus, Ohio, USA, Seite 503, Spalte 1, Zusammenfassung 66027r

## Beschreibung

Die vorliegende Erfindung betrifft substituierte Pyrrole. Insbesondere betrifft die Erfindung substituierte Pyrrole der allgemeinen Formel 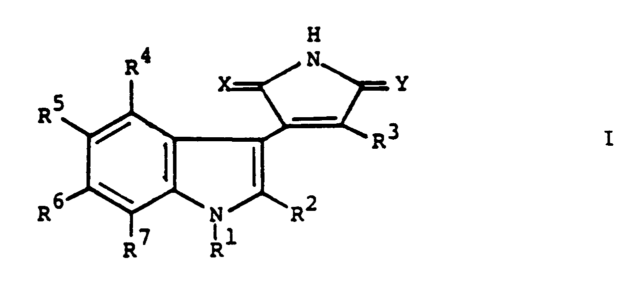
worin
- R¹: Wasserstoff, Alkyl, Aryl, Aralkyl, Alkoxyalkyl, Hydroxyalkyl, Haloalkyl, Aminoalkyl, Monoalkylaminoalkyl, Dialkylaminoalkyl, Trialkylaminoalkyl, Aminoalkylaminoalkyl, Azidoalkyl, Acylaminoalkyl, Acylthioalkyl, Alkylsulfonylaminoalkyl, Arylsulphonylaminoalkyl, Mercaptoalkyl, Alkylthioalkyl, Alkylsulfinylalkyl, Alkylsulfonylalkyl, Alkylsulfonyloxyalkyl, Alkylcarbonyloxyalkyl, Cyanoalkyl, Amidinoalkyl, Isothiocyanatoalkyl, Glucopyranosyl, Carboxyalkyl, Alkoxycarbonylalkyl, Aminocarbonylalkyl, Hydroxyalkylthioalkyl, Mercaptoalkylthioalkyl, Arylthioalkyl oder Carboxyalkylthioalkyl oder eine Gruppe der Formel 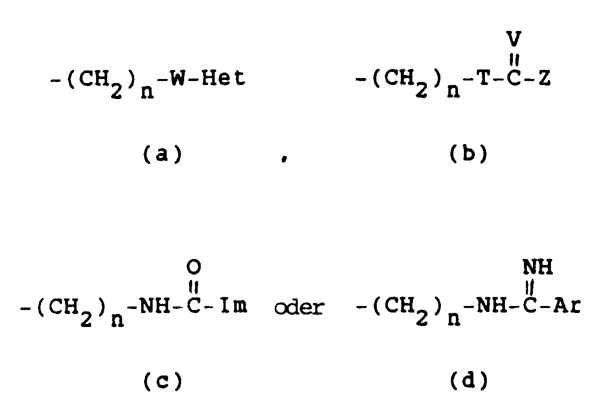 in der
- Het: eine heterocyclische Gruppe,
- W: NH, S oder eine Bindung,
- T: NH oder S,
- V: O, S, NH, NNO₂, NCN oder CHNO₂,
- Z: Alkylthio, Amino, Monoalkylamino oder Dialkylamino,
- Im: 1-Imidazolyl,
- Ar: Aryl, und
- n: eine Zahl von 2-6;
- R²: Wasserstoff, Alkyl, Aralkyl, Alkoxyalkyl, Hydroxyalkyl, Haloalkyl, Aminoalkyl, Monoalkylaminoalkyl, Dialkylaminoalkyl, Acylaminoalkyl, Alkylsulfonylaminoalkyl, Arylsulfonylaminoalkyl, Mercaptoalkyl, Alkylthioalkyl, Carboxyalkyl, Alkoxycarbonylalkyl, Aminocarbonylalkyl, Alkylthio oder Alkylsulfinyl;
- R³: eine carbocyclische oder heterocyclische aromatische Gruppe;
- R⁴, R⁵, R⁶ und R⁷: unabhängig voneinander Wasserstoff, Halogen, Alkyl, Hydroxy, Alkoxy, Aryloxy, Haloalkyl, Nitro, Amino, Acylamino, Monoalkylamino, Dialkylamino, Alkylthio, Alkylsulfinyl oder Alkylsulfonyl;
und eines von X und Y O und das andere O, S, (H,OH) oder (H,H) ist
mit der Bedingung, dass R¹ von Wasserstoff verschieden ist, falls R² Wasserstoff, R³ 3-Indolyl oder 6-Hydroxy-3-indolyl, R⁴, R⁵ und R⁷ Wasserstoff, R⁶ Wasserstoff oder Hydroxy und X und Y beide O sind, sowie falls R² Wasserstoff, R³ 3-Indolyl, R⁴, R⁵, R⁶ und R⁷ Wasserstoff, X (H,H) und Y O sind;
wobei eine heterocyclische Gruppe Het für eine gesättigte, teilweise ungesättigte oder eine aromatische 5- oder 6-gliedrige heterocyclische Gruppe steht, die gewünschtenfalls einen ankondensierten Benzolring enthält und unsubstituiert oder substituiert ist durch einen oder mehrere Substituenten aus der Gruppe von Halogen, Alkyl, Hydroxy, Alkoxy, Haloalkyl, Nitro, Amino, Acylamino, Mono- oder Dialkylamino, Alkylthio, Alkylsulfinyl und Alkylsufonyl,
eine heterocyclische aromatische Gruppe R³ wie weiter oben eine heterocyclische aromatische Gruppe Het definiert ist, und
eine carbocyclische aromatische Gruppe R³ für Phenyl oder Naphthyl steht, das gewünschtenfalls wie die obige Gruppe Het substituiert ist, und
Acyl allein oder in Kombination eine von einer Alkancarbonsäure mit bis zu 7 C-Atome oder von einer aromatischen Carbonsäure abgeleitete Gruppe ist, und
Alkyl allein oder in Kombination für eine geradkettige oder verzweigte Alkylgruppe mit bis zu 7 C-Atome steht,
sowie pharmazeutisch verwendbare Salze von sauren Verbindungen der Formel I mit Basen und von basischen Verbindungen der Formel I mit Säuren.

Die Erfindung betrifft die weiter oben definierten Verbindungen als solche sowie als therapeutisch aktive Substanzen; ein Verfahren zu ihrer Herstellung; Medikamente, die diese Verbindungen enthalten und die Herstellung dieser Medikamente; sowie die Verwendung der besagten Verbindungen bei der Behandlung oder der Prophylaxe von Krankheiten speziell von inflammatorischen, immunologischen, bronchopulmonären und cardiovaskulären Krankheiten, oder bei der Herstellung von Medikamenten gegen inflammatorische, immunologische, bronchopulmonäre und cardiovaskuläre Krankheiten.

Die Erfindung betrifft ferner ein Verfahren zur Herstellung eines Arzneimittels, wobei der Aktivstoff, nämlich eine der weiter oben definierten Verbindungen, zusammen mit einem pharmazeutisch geeigneten Trägermaterial und sonstigen Hilfsstoffen in eine für therapeutische Zwecke anwendbare Form gebracht wird, dadurch gekennzeichnet, dass man ein Heilmittel für die Behandlung von inflammatorischen, immunologischen, bronchopulmonären und cardiovaskulären Krankheiten herstellt, bestehend aus der Kombination von einem eine der besagten Verbindungen enthaltenden Präparat und der Information, welche in direktem Zusammenhang mit der Behandlung der besagten Krankheiten steht.

Im Rahmen der Erfindung bezeichnet "Alkyl" allein oder in Kombination eine geradkettige oder verzweigte Alkylgruppe mit bis zu 7, vorzugsweise bis zu 4 C-Atome, wie Methyl, Aethyl, Propyl, Isopropyl, Butyl, sek.Butyl, t-Butyl und Pentyl. Beispiele von Alkoxygruppen sind Methoxy, Aethoxy, Propoxy, Isopropoxy, Butoxy und t-Butoxy. Ein Haloalkyl kann eine oder mehrere Halogenatome enthalten. Ein Beispiel für eine solche Gruppe ist Chlormethyl und Trifluormethyl. Der Acylanteil von Acylamino, Acylaminoalkyl oder Acylthioalkyl ist von einer Alkancarbonsäure mit bis zu 7, vorzugsweise bis zu 4 C-Atome (z.B. Acetyl, Propionyl oder Butyryl) oder von einer aromatischen Carbonsäure (z.B. Benzoyl) abgeleitet. Der Ausdruck "Aryl" allein oder in Kombination, wie in Arylsulfonylaminoalkyl, Arylthioalkyl oder Aralkyl bezeichnet unsubstituiertes Phenyl oder Phenyl substituiert durch einen oder mehreren, vorzugsweise 1-3 Substituenten, wie Halogen, Alkyl, Hydroxy, Benzyloxy, Alkoxy, Haloalkyl, Nitro, Amino und Cyan. "Halogen" bezeichnet Fluor, Chlor, Brom oder Jod.

Beispiele von heterocyclischen Gruppen Het sind Imidazolyl, Imidazolinyl, Thiazolinyl, Pyridyl und Pyrimidinyl. Eine aromatische Stickstoffenthaltende Gruppe Het kann in Form eines N-Oxids vorliegen.

Beispiele von carbocyclischen aromatischen Gruppen R³ sind Phenyl, 2-, 3- oder 4-Chlorphenyl, 3-Bromphenyl, 2- oder 3-Methylphenyl, 2,5-Dimethylphenyl, 4-Methoxyphenyl, 2- oder 3-Trifluormethylphenyl, 2-, 3- oder 4-Nitrophenyl, 3- oder 4-Aminophenyl, 4-Methylthiophenyl, 4-Methylsulfinylphenyl, 4-Methylsulfonylphenyl und 1- oder 2-Naphthyl.

Eine heterocyclische aromatische Gruppe R³ kann eine 3-Indolylgruppe, insbesondere eine solche der Formel 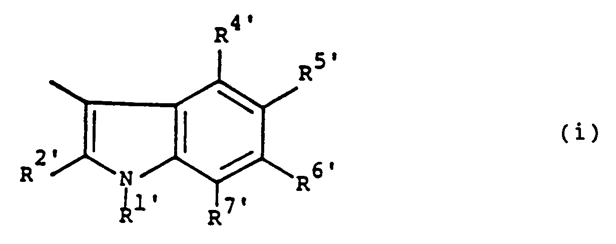
sein,
worin R^{1′}, R^{2′}, R^{4′}, R^{5′}, R^{6′} und R^{7′} eine der Bedeutungen von R¹, R², R⁴, R⁵, R⁶ und R⁷ in Formel I haben.

Beispiele von heterocyclischen aromatischen Gruppen R³ sind 2- oder 3-Thienyl, 3-Benzothienyl, 1-Methyl-2-pyrrolyl, 1-Benzimidazolyl, 3-Indolyl, 1- oder 2-Methyl-3-indolyl, 1-Methoxymethyl-3-indolyl, 1-(1-Methoxyäthyl)-3-indolyl, 1-(2-Hydroxypropyl)-3-indolyl, 1-(4-Hydroxybutyl)- -3-indolyl, 1-[1-(2-Hydroxyäthylthio)äthyl]-3-indolyl, 1-[1-(2-Mercaptoäthylthio)äthyl]-3-indolyl, 1-(1-Phenylthioäthyl)-3-indolyl, 1-[1-(Carboxymethylthio)äthyl]-3-indolyl und 1-Benzyl-3-indolyl.

In der Formel I bezeichnet R¹ vorzugsweise Alkyl, Aminoalkyl, Isothiocyanoatoalkyl oder eine Gruppe der Formel (b), in der T S, V NH und Z Amino oder in der T NH, V NH oder NNO₂ und Z Amino bezeichnet. Besonders bevorzugt sind Verbindungen der Formel I, worin R¹ Methyl, 3-Aminopropyl, 3-Isothiocyanatopropyl oder die weiter oben genannte Gruppe der Formel (b), in der n 3 ist. Vorzugsweise ist R² Wasserstoff. Vorzugsweise ist R³ Phenyl, monosubstituiert durch Halogen, insbesondere Chlor oder Brom, Alkyl, insbesondere Methyl, Alkoxy, insbesondere Methoxy, Haloalkyl, insbesondere Trifluormethyl, Nitro, Amino, Alkylthio, speziell Methylthio, Alkylsulfinyl, speziell Methylsulfinyl, oder Alkylsulfonyl, speziell Methylsulfonyl, oder eine Gruppe der Formel (i), insbesondere eine solche, in der R^{1′} Methyl, Methoxymethyl, 1-Methoxyäthyl, 2-Hydroxypropyl, 4-Hydroxybutyl, 1-(2-Hydroxyäthylthio)äthyl, 1-(2-Mercaptoäthylthio)äthyl, 1-Phenylthioäthyl oder 1-(Carboxymethylthio)äthyl, insbesondere Methyl, und R^{2′}, R^{4′}, R^{5′}, R^{6′} und R^{7′} Wasserstoff sind. Vorzugsweise sind R⁴, R⁵, R⁶ und R⁷ Wasserstoff.

Besonders bevorzugt sind die Verbindungen der Formel I, worin R¹ Methyl, 3-Aminopropyl, 3-Isothiocyanatopropyl oder eine Gruppe der Formel (b), in der T S, V NH, Z Amino und n die Zahl 3 ist oder in der T NH, V NH oder NNO₂, Z Amino und n die Zahl 3, R² Wasserstoff, R³ Phenyl monosubstituiert durch Chlor, Brom, Methyl, Methoxy, Trifluormethyl, Nitro, Amino, Methylsulfinyl oder Methylsulfonyl oder eine Gruppe der Formel (i), in der R^{1′} Methyl und R^{2′}, R^{4′}, R^{5′}, R^{6′} und R^{7′} sowie R⁴, R⁵, R⁶ und R⁷ Wasserstoff sind.

Speziell bevorzugt sind folgende Verbindungen:
3-(2-Chlorphenyl)-4-(1-methyl-3-indolyl)-1H-pyrrol-2,5-dion,
3-(1-Methyl-3-indolyl)-4-(2-nitrophenyl)-1H-pyrrol-2,5-dion,
3,4-Bis(1-methyl-3-indolyl)-1H-pyrrol-2,5-dion,
3-[1-(3-Aminopropyl)-3-indolyl]-4-(1-methyl-3-indolyl)-1H-pyrrol-2,5-dion,
3-[1-[3-(Amidinothio)propyl]-3-indolyl]-4-(1-methyl-3-indolyl)-1H-pyrrol-2,5-dion,
3-(1-Methyl-3-indolyl)-4-[1-[3-(2-nitroguanidino)propyl]-3-indolyl]-1H-pyrrol-2,5-dion und
3-[1-(3-Isothiocyanatopropyl)-3-indolyl]-4-(1-methyl-3-indolyl)-1H-pyrrol-2,5-dion.

Die Verbindungen der Formel I und ihre pharmazeutisch verwendbare Salze wie weiter oben definiert können dadurch hergestellt werden, dass man
a) zur Herstellung einer Verbindung der Formel I, in der X und Y O sind, eine Verbindung der Formel 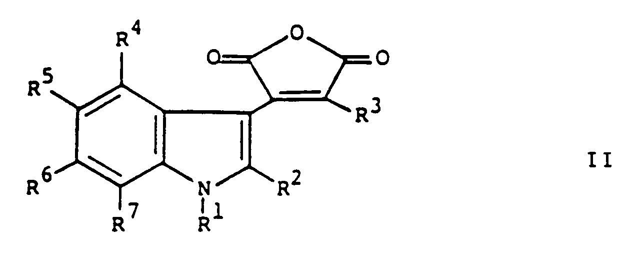 worin R¹ bis R⁷ die oben angegebene Bedeutung haben,
   mit Ammoniak unter Druck oder mit Hexamethyldisilazan und Methanol umsetzt, oder
(b) zur Herstellung einer Verbindung der Formel I, in der R¹ Wasserstoff und X und Y O sind, eine Verbindung der Formel 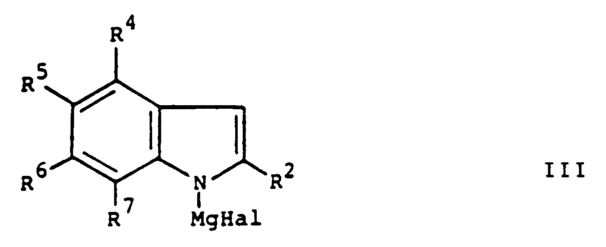 worin R² bis R⁷ die oben angegebene Bedeutung haben und Hal Halogen ist,
   mit einer Verbindung der Formel 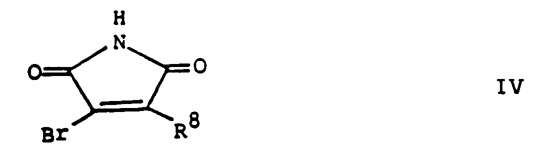 worin R⁸ die gleiche Bedeutung wie R³ hat oder Brom ist,
   umsetzt, oder
(c) zur Herstellung einer Verbindung der Formel I, in der R³ 1-Benzimidazolyl und X und Y O sind, eine Verbindung der Formel 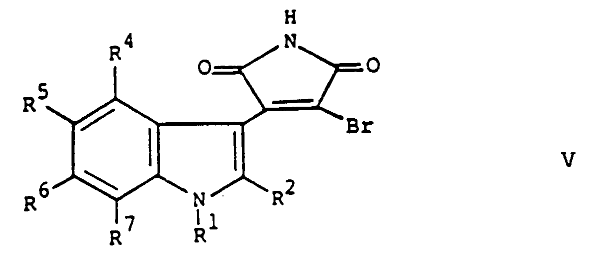 worin R¹ bis R⁷ die oben angegebene Bedeutung haben,
   mit einem Alkalimetallderivat des Benzimidazols umsetzt, oder
(d) zur Herstellung einer Verbindung der Formel I, in der eins von X und Y O und das andere S ist, eine Verbindung der Formel I, in der X und Y O sind, mit einem Sulfurierungsmittel umsetzt, oder
(e) zur Herstellung einer Verbindung der Formel I, in der eins von X und Y O und das andere (H,OH) ist, eine Verbindung der Formel I, in der X und Y O sind, mit einem komplexen Metallhydrid reduziert, oder
(f) zur Herstellung einer Verbindung der Formel I, in der eines von X und Y O und das andere (H,H) ist, eine Verbindung der Formel I, in der eins von X und Y O und das andere (H,OH) ist, katalytisch hydriert, oder
(g) zur Herstellung einer Verbindung der Formel I, in der R¹ Alkyl, Aralkyl, Alkoxyalkyl oder Hydroxyalkyl ist, eine Verbindung der Formel I, in der R¹ Wasserstoff ist, entsprechend N-substituiert, und
(h) erwünschtenfalls einen in einer Verbindung der Formel I vorhandenen reaktiven Substituenten funktionell abwandelt, und
(i) erwünschtenfalls eine saure Verbindung der Formel I in ein pharmazeutisch verwendbares Salz mit einer Base oder eine basische Verbindung der Formel I in ein pharmazeutisch verwendbares Salz mit einer Säure überführt.

Die Reaktion einer Verbindung der Formel II mit Ammoniak nach Verfahrensvariante a) kann man zweckmässig mittels wässrigem Ammoniak (vorzugsweise 33%iges wässrigem Ammoniak) und in Gegenwart eines wassermischbaren inerten organischen Lösungsmittels, wie Dimethylformamid (DMF) durchführen. Die Reaktion erfolgt vorzugsweise bei erhöhter Temperatur, z.B. zwischen etwa 100 und 150°C. Im allgemeinen beträgt die Reaktionsdauer zwischen etwa 0,5 und 5 Stunden.

Die Reaktion einer Verbindung der Formel II mit Hexamethyldisilazan und Methanol wird zweckmässig in einem inerten organischen Lösungsmittel, wie einem halogenierten Kohlenwasserstoff, z.B. Chloroform, Tetrachlorkohlenstoff oder Chlorbenzol, oder in einem aromatischen Kohlenwasserstoff, z.B. Benzol, Toluol oder Xylol, und bei erhöhter Temperatur, z.B. zwischen etwa 40 und 110°C durchgeführt.

Die Grignard-Reaktion zwischen einer Verbindung der Formel III und einer solchen der Formel IV kann man in an sich bekannter Weise durchführen, z.B. in einem inerten organischen Lösungsmittel, z.B. einem der weiter oben erwähnten aromatischen Kohlenwasserstoffe, und bei einer Temperatur zwischen etwa Raumtemperatur und Rückflusstemperatur des Reaktionsgemisches. Im allgemeinen beträgt die Reaktionsdauer einige Stunden, z.B. 18 Stunden bis einige Tagen, z.B. 5 Tage. Zweckmässigerweise wird eine Verbindung der Formel III in situ ausgehend von Indol oder einem substituierten Indol und einem geeigneten Alkylmagnesiumhalogenid, wie Methylmagnesiumbromid oder -jodid in an sich bekannter Weise hergestellt. Das Symbol Hal in den Verbindungen der Formel III bezeichnet vorzugsweise Brom oder Jod. Falls eine Verbindung der Formel III mit einer solchen der Formel IV, in der R⁸ Brom ist, umgesetzt wird, erhält man eine symmetrisch substituierte Verbindung der Formel I, d.h. eine solche, in der R³ eine Gruppe der Formel (i) ist, worin R^{1′}, R^{2′}, R^{4′}, R^{5′}, R^{6′} und R^{7′} die gleiche Bedeutung wie R¹, R², R⁴, R⁵, R⁶ bzw. R⁷ in der Verbindung der Formel III hat.

Herkömmliche Methoden können bei der Durchführung der Reaktion einer Verbindung der Formel V mit einem Alkalimetallderivat des Benzimidazols nach Variante c) angewendet werden. Die Reaktion wird zweckmässigerweise in einem inerten organischen Lösungsmittel, wie DMF durchgeführt. Die Reaktionstemperatur ist nicht kritisch. Die Reaktion wird jedoch vorzugsweise bei erhöhter Temperatur, z.B. etwa 45-95°C durchgeführt. Die Alkalimetalle, vorzugsweise Natriumderivate werden vorzugsweise in situ hergestellt durch Behandlung von Benzimidazol mit einer geeigneten Alkalimetallbase, wie einem Alkalimetallhydrid, z.B. Natriumhydrid.

Die Sulfurierung nach Variante d) wird zweckmässigerweise mittels Phosphorpentasulfid, Lawesson's Reagens [2,4-Bis(4-methoxyphenyl)-1,2-dithioxo -1,3,2,4-dithiaphosphetan; Bull. Soc. Chim. Belg. 87 (1978) 229-238] oder Davy-Reagens [2,4-Bis(Methylthio)-1,3,2,4-dithiadiphosphetan; Sulfur Lett. 1983, 1, 167] durchgeführt. Diese Reaktion erfolgt zweckmässigerweise in einem inerten organischen Lösungsmittel, wie einem aliphatischen oder cyclischen Aether, z.B. Dimethoxyäthan, oder einem aromatischen erwünschtenfalls halogenierten Kohlenwasserstoff, z.B. Benzol, Toluol oder Chlorbenzol, und bei erhöhter Temperatur speziell unter Rückfluss.

Die Reduktion nach Variante e) kann man in an sich bekannter Weise durchführen. Als komplexe Metallhydride verwendet man z.B. Diisobutylaluminiumhydrid, Natriumdihydro-bis(2-methoxyäthoxy)aluminat oder vorzugsweise ein Alkalimetallaluminiumhydrid, wie Lithiumaluminiumhydrid. Die Reduktion kann in einem inerten organischen Lösungsmittel, wie einem aliphatischen oder cyclischen Aether, wie Diäthyläther oder Tetrahydrofuran (THF) oder in einem Kohlenwasserstoff, wie Hexan, Benzol oder Toluol, zweckmässigerweise bei etwa Raumtemperatur erfolgen.

Die katalytische Hydrierung nach Variante f) kann man in an sich bekannter Weise durchführen, z.B. in Gegenwart eines Edelmetallkatalysators, wie einem Palladium- oder Platinkatalysator, z.B. Palladium/Kohlenstoff (Pd/C), und in einem inerten organischen Lösungsmittel, wie einem Alkanol, z.B. Methanol oder Aethanol, zweckmässigerweise bei etwa Raumtemperatur und unter Normaldruck.

Die N-Substitution einer Verbindung der Formel I, in der R¹ Wasserstoff ist, nach Variante g) kann man in an sich für die N¹-Substitution von Indolen bekannter Weise durchführen. Z.B. kann eine Hydroxyalkylgruppe R¹ in eine Verbindung der Formel I, in der R¹ Wasserstoff ist, dadurch eingeführt werden, dass man zuerst diese Verbindung in ein Alkalimetallderivat, z.B. das Natriumderivat überführt, z.B. mittels eines Alkalimetallhydrids, wie Natriumhydrid, und dann das erhaltene Derivat mit einem die Hydroxyalkylgruppe abgebenden Mittel, z.B. einem Alkylenoxid, wie Propylenoxid behandelt. Eine Alkoxyalkylgruppe R¹ kann man durch Behandlung einer Verbindung der Formel I, in der R¹ Wasserstoff ist, mit einem geeigneten Aldehyddialkylacetal in Gegenwart einer Säure, z.B. p-Toluolsulfonsäure, bei erhöhter Temperatur einführen. Ferner kann eine Verbindung der Formel I, in der R¹ Wasserstoff ist, mit einem Alkyl- oder Aralkylhalogenid in Gegenwart einer Base zu einer Verbindung der Formel I, in der R¹ Alkyl oder Aralkyl ist, umgesetzt werden.

Die funktionellen Modifikationen nach Variante h) lassen sich ebenfalls in an sich bekannter Weise durchführen. Z.B. kann man eine Nitrogruppe zur Aminogruppe reduzieren und letztere dann alkylieren oder acylieren. Eine Aminoalkylgruppe kann alkyliert, acyliert oder sulfonyliert werden. Eine Alkylthio- oder Alkylthioalkylgruppe kann man zur Alkylsulfinyl- bzw. Alkylsulfinylalkylgruppe und letztere gewünschtenfalls zur Alkylsulfonyl- bzw. Alkylsulfonylalkylgruppe oxidieren. Eine Alkoxycarbonylalkylgruppe kann zur Carboxyalkylgruppe verseift werden und letztere dann amidiert oder umestert werden. Eine Alkoxyalkylgruppe kann man zu einer Alkylthioalkyl- oder Arylthioalkylgruppe mittels eines Alkanthiols oder Thiophenols umsetzen. Eine Azidoalkylgruppe kann durch katalytische Hydrierung in eine Aminoalkylgruppe übergeführt und letztere wiederum funktionellen Modifikationen unterworfen werden. Z.B. kann man eine Aminoalkylgruppe mittels 1,1′-Thiocarbonyldiimidazol in eine Isothiocyanatoalkylgruppe umwandeln. Ferner kann man eine Amino-C₂₋₆-alkylgruppe überführen in eine Gruppe der Formel (a), in der W NH ist, durch Reaktion mit einem reaktionsfähigen Derivat einer geeigneten heterocyclischen Verbindung oder in eine Gruppe der Formel (b), in der
1. T NH, V NH und Z Amino ist, mittels 3,5-Dimethylpyrazol-1-carboxamidin,
2. T NH, V NNO₂ und Z Amino ist, mittels 3,5-Dimethyl-N²-nitro-1-pyrazol-1-carboxamid,
3. T NH, V NCN und Z Alkylthio ist, mittels eines Dialkyl-N-cyandithioiminocarbonats oder
4. T NH, V CHNO₂ und Z Alkylthio ist, mittels 1,1-Bis(alkylthio)-2-nitroäthylen.

Weiter kann man eine Amino-C₂₋₆-alkylgruppe in eine Gruppe der Formel (c) durch Reaktion mit 1,1′-Carbonyldiimidazol oder in eine Gruppe der Formel (d) durch Reaktion mit einem geeigneten Benzimidat überführen. Die Umwandlung einer Gruppe der Formel (b), in der T NH, V NCN oder CHNO₂ und Z Alkylthio ist, in eine entsprechende Gruppe der Formel (b), in der Z Amino oder Mono- oder Dialkylamino ist, kann man mittels Ammoniak oder einem Mono- oder Dialkylamin bewerkstelligen. Eine Isothiocyanatoalkylgruppe kann man in eine Gruppe der Formel (b), in der T NH, V S und Z Amino ist, durch Behandlung mit Ammoniak überführen.

Eine Alkylcarbonyloxyalkylgruppe kann man zur Hydroxyalkylgruppe verseifen und letztere in an sich bekannter Weise in eine Haloalkyl- oder eine Alkylsulfonyloxyalkylgruppe überführen. Eine Hydroxyalkylgruppe kann man auch in eine Aminoalkylaminoalkylgruppe überführen durch Behandlung mit Trifluormethansulfonsäureanhydrid gefolgt durch Reaktion mit einem geeigneten Diaminoalkan. Eine Hydroxy-C₂₋₆-alkylgruppe kann man zuerst mit Trifluormethansulfonsäureanhydrid und dann mit einer geeigneten heterocyclischen Verbindung, z.B. Pyridin, in eine Gruppe der Formel (a), in der W eine Bindung ist, umwandeln. Eine Alkylsulfonyloxyalkylgruppe kann beispielsweise in eine Mono- , Di- bzw. Trialkylaminoalkylgruppe mittels eines Mono-, Di- bzw. Trialkylamin; in eine Cyanalkylgruppe mittels eines Alkalimetallcyanids, in eine Alkylthioalkylgruppe mittels eines Alkalimetallalkanthiolat, oder in eine Acylthioalkylgruppe mittels eines Alkalimetallthioacylats umgewandelt werden.

Eine Alkylsulfonyloxy-C₂₋₆-alkylgruppe kann man auch mittels Thioharnstoff in eine Gruppe der Formel (b), in der T S, V NH und Z Amino ist, umwandeln. Ferner lässt sich eine Cyanalkylgruppe in eine Amidinoalkylgruppe mittels Ammoniak, eine Acylthioalkylgruppe in eine Mercaptoalkylgruppe mittels wässrigem Ammoniak, sowie eine Benzyloxy-arylgruppe in eine Hydroxy-arylgruppe durch Hydrogenolyse umwandeln. Ferner kann eine Gruppe der Formel (c) in eine solche der Formel (b), in der T NH, V O und Z Amino ist, mittels alkoholischem Ammoniak, umgewandelt werden. Selbstverständlich haben die obigen Umwandlungen lediglich beispielhaften Charakter und es können andere dem Fachmann bekannte Modifikationen durchgeführt werden.

Die Ueberführung einer sauren Verbindung der Formel I in ein pharmazeutisch verwendbares Salz nach Variante i) kann durch Behandlung mit einer geeigneten Base in an sich bekannter Weise durchgeführt werden. Geeignete Salze sind solche die von einer anorganischen Base, z.B. Natrium-, Kalium- oder Calciumsalze, oder von einer organischen Base, wie Aethylendiamin oder Mono- oder Diäthanolamin, abgeleitet sind. Die Umwandlung einer basischen Verbindung der Formel I in ein pharmazeutisch verwendbares Salz kann durch Behandlung mit einer geeigneten Säure in an sich bekannter Weise bewerkstelligt werden. Geeignete Salze sind solche, die von einer anorganischen Säure, z.B. Hydrochloride, Hydrobromide, Phosphate oder Sulfate, oder von einer organischen Säure, z.B. Acetate, Citrate, Fumarate, Tartrate, Maleate, Methansulfonate oder p-Toluolsulfonate, abgeleitet sind.

Die Ausgangsmaterialien der Formel II können durch Reaktion einer Verbindung der Formel 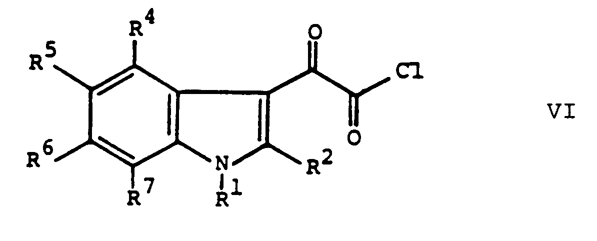
mit einer Verbindung der Formel

HOOC-CH₂-R³ VII

worin R¹ bis R⁷ die obige Bedeutung haben,
und gewünschtenfalls durch funktionelle Modifikation eines reaktionsfähigen Substituenten in einer erhaltenen Verbindung der Formel II hergestellt werden. Diese funktionellen Modifikationen können in der gleichen Weise durchgeführt werden, wie weiter oben beschrieben, für die funktionelle Modifikation von reaktionsfähigen Substituenten in einer Verbindung der Formel I.

Die Reaktion einer Verbindung der Formel VI mit einer Verbindung der Formel VII führt man vorzugsweise durch in Gegenwart eines Säure-bindenden Mittels, zweckmässigerweise einem tertiären Amin, wie einem Trialkylamin, z.B. Triäthyl- oder Diisopropyläthylamin, und in einem inerten organischen Lösungsmittel wie einem halogenierten aliphatischen Kohlenwasserstoff.

Die Verbindungen der Formel VI können hergestellt werden durch Umsetzung einer Verbindung der Formel 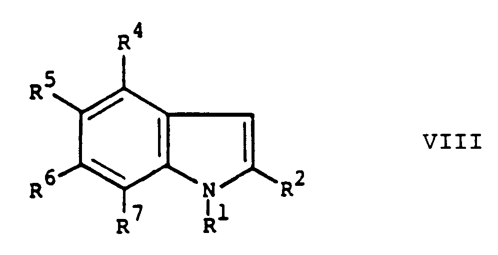
worin R¹ bis R⁷ die obige Bedeutung haben,
mit Oxalylchlorid, zweckmässigerweise in einem inerten organischen Lösungsmittel, wie einem halogenierten aliphatischen Kohlenwasserstoff, bei einer Temperatur zwischen etwa 0°C und Rückflusstemperatur des Lösungsmittels. Die resultierende Verbindung der Formel VI kann in situ mit einer Verbindung der Formel VII umgesetzt werden oder kann isoliert und, bevor man sie mit einer Verbindung der Formel VII umsetzt, gereinigt werden.

Wie weiter oben angegeben sind die Verbindungen der Formel III leicht zugänglich aus einem Indol oder einem geeignet substituierten Indol, d.h. einer Verbindung der Formel VIII, in der R¹ Wasserstoff ist, und aus einem geeigneten Alkylmagnesiumhalogenid, wie Methylmagnesiumbromid oder -jodid. So kann man eine Lösung einer Verbindung der Formel VIII in einem inerten organischen Lösungsmittel, wie einem aromatischen Kohlenwasserstoff, mit einer ätherischen Lösung des Alkylmagnesiumhalogenids bei etwa Raumtemperatur umsetzen.

Die Verbindungen der Formel IV, in der R⁸ die gleiche Bedeutung wie R³ hat, kann durch Bromierung einer Verbindung der Formel 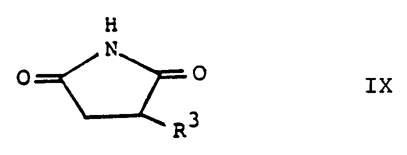
worin R³ die obige Bedeutung hat,
hergestellt werden.

Die Verbindungen der Formel V (oder diejenigen der Formel IV, worin R⁸ eine Gruppe der Formel (i) ist) können hergestellt werden durch Umsetzung einer Verbindung der Formel III mit Dibrommaleinimid, d.h. einer Verbindung der Formel IV, in der R⁸ Brom ist.

Die Bromierung einer Verbindung der Formel IX kann zweckmässigerweise mittels elementarem Brom gegebenenfalls in Gegenwart eines inerten organischen Lösungsmittels, z.B. eines aliphatischen Aethers, erfolgen. Die Bromierung wird vorzugsweise bei erhöhter Temperatur durchgeführt, z.B. bei 100-120°C, wenn kein Lösungsmittel verwendet wird, und unter Rückfluss, wenn man in einem Lösungsmittel arbeitet.

Die Reaktion einer Verbindung der Formel III mit Dibrommaleinimid kann in der gleichen Weise durchgeführt werden, wie weiter oben beschrieben, für die Verfahrensvariante b).

Die Pyrrole der Formel I und ihre pharmazeutisch verwendbaren Salze sind Inhibitoren der Proteinkinase; sie hemmen zellulare Prozesse, z.B. die Zellproliferation, und können in der Behandlung oder Prophylaxe von Krankheiten, z.B. von inflammatorischen Krankheiten, wie Arthritis, von Immunkrankheiten oder in Kombination mit Organtransplantationen, sowie in der Onkologie Verwendung finden. Sie hemmen die Infektion der Zellen durch Humanimmunodefekt-Viren und sind daher in der Behandlung von AIDS verwendbar. Sie hemmen auch die Kontraktion der glatten Muskulatur und können daher gegen cardiovaskuläre und bronchopulmonäre Krankheiten verwendet werden. Ferner sind sie auch in der Asthmatherapie verwendbar.

Die Aktivität der vorliegenden Verbindungen in der Hemmung der Proteinkinase C kann man z.B. anhand des in BBRC 19 (1979) 1218 beschriebenen Versuchssystems nachweisen. Die IC₅₀-Werte in der nachstehenden Tabelle sind diejenigen Konzentrationen der Testsubstanz, die die durch die Proteinkinase induzierte Inkorporation von ³²P aus [γ-³²P]ATP in das Histon um 50% reduziert.

**Tabelle**

| Verbindung | IC₅₀ |
|---|---|
| 3-[1-(2-Carbamoyläthyl)-3-indolyl]-4-(1-methyl-3-indolyl)-1H-pyrrol-2,5-dion | 0,5 µM |
| 3-(5-Amino-1-methyl-3-indolyl)-4-(1-methyl-3-indolyl)-1H-pyrrol-2,5-dion | 0,6 µM |
| 3-(1-Methyl-3-indolyl)-4-(3-(methylphenyl)-1H-pyrrol-2,5-dion | 1,0 µM |
| 3-[1-[3-(Amidinothio)propyl]-3-indolyl]-4-(1-methyl-3-indolyl)-1H-pyrrol-2,5-dion | 0,010 µM |
| 3-(1-Methyl-3-indolyl)-4-[1-[3-(2-nitroguanidino)propyl]-3-indolyl]-1H-pyrrol-2,5-dion | 0,025 µM |
| 3-[1(3-Isothiocyanatopropyl)-3-indolyl]-4-(1-methyl-3-indolyl)-1H-pyrrol-2,5-dion | 0,008 µM |

Die Pyrrole der Formel I und ihre Salze können als Medikamente verwendet werden, z.B. in Form von pharmazeutischen Präparaten, die man oral, z.B. in Form von Tabletten, Dragées, Hart- oder Weichgelatinekapseln, Lösungen, Emulsionen oder Suspensionen, verabreichen kann. Sie können auch rektal, z.B. in Form von Suppositorien, oder parenteral, z.B. in Form von Injektionslösungen verabreicht werden. Für die Herstellung von pharmazeutischen Präparaten können diese Verbindungen mit therapeutisch inerten anorganischen und organischen Träger verarbeitet werden. Beispiele von solchen Trägern für Tabletten, Dragées und Hartgelatinekapseln sind Lactose, Maisstärke oder Derivate davon, Talk, Stearinsäure oder dessen Salze. Geeignete Träger für Weichgelatinekapseln sind pflanzliche Oele, Wachse, Fette und halbfeste oder flüssige Polyole. Je nach der Natur des Wirkstoffes erübrigen sich jedoch Träger im Fall von Weichgelatinekapseln. Geeignete Träger für die Herstellung von Lösungen und Sirupen sind Wasser, Polyole, Saccharose, Invertzucker und Glucose. Geeignete Träger für Injektionslösungen sind Wasser, Alkohole, Polyole, Glycerol und pflanzliche Oele. Geeignete Träger für Suppositorien sind pflanzliche oder gehärtete Oele, Wachse, Fette und halbflüssige Polyole.

Die pharmazeutischen Präparate können auch Konservierungsmittel, Lösungsmittel, Stabilisierungsmittel, Netzmittel, Emulgiermittel, Süsstoffe, Farbstoffe, Geschmacksmittel, Salze zur Veränderung des osmotischen Druckes, Puffer, Ueberzugsmittel oder Antioxidantien, sowie gegebenenfalls andere therapeutische Wirkstoffe enthalten.

Wie weiter oben angegeben können die Pyrrole der Formel I und ihre Salze in der Behandlung oder Prophylaxe von Krankheiten speziell von inflammatorischen, immunologischen, bronchopulmonären und cardiovaskulären Krankheiten verwendet werden. Die Dosierung kann in weiten Bereichen variieren, liegt jedoch im allgemeinen bei oraler Verabreichung an Erwachsenen im Bereich von etwa 5 bis 500 mg/Tag, obwohl letzterer Wert, falls nötig, erhöht werden kann. Die tägliche Dosis kann in einer Einzeldosis oder in mehreren Dosen verabreicht werden.

### Beispiel 1

0,4 g 3-(1-Methyl-3-indolyl)-4-(1-methyl-5-nitro-3-indolyl)furan -2,5-dion werden mit 3 ml DMF und 20 ml einer 33%igen wässrigen Ammoniaklösung behandelt und 3,5 Stunden bei 140°C erhitzt. Das abgekühlte Gemisch wird filtriert und der Rückstand wird mit Wasser gewaschen und getrocknet. Man erhält 0,29 g 3-(1-Methyl-3-indolyl)-4-(1-methyl-5-nitro-3-indolyl) -1H-pyrrol-2,5-dion, Smp. 282-284°C.

Das Ausgangsfurandion wird wie folgt hergestellt:
0,7 g 1-Methyl-5-nitroindol-3-glyoxylylchlorid in 20 ml Dichlormethan werden mit 0,85 ml Triäthylamin und 0,5 g 1-Methylindol-3-ylessigsäure behandelt. Das Gemisch wird 16 Stunden stehengelassen und dann eingeengt. Der Rückstand wird an Silicagel mit 50% Aethylacetat in Petroleumäther chromatographiert. Man erhält 0,42 g Furandion, Smp. 220-221°C.

### Beispiel 2

56 mg 3-(1-Methyl-3-indolyl)-4-(1-naphthyl)furan-2,5-dion werden mit 5 ml DMF und 5 ml einer 33%igen wässrigen Ammoniaklösung behandelt und das Gemisch wird 5 Stunden auf 130°C erhitzt. Der gebildete Niederschlag wird abfiltriert, mit Wasser gewaschen und getrocknet. Man erhält 53 mg 3-(1-Methyl-3-indolyl)-4-(1-naphthyl)-1H-pyrrol-2,5-dion, Smp. 258-260°C.

Das Ausgangsfurandion wird wie folgt hergestellt:
Zu 1,1 g 1-Methylindol-3-glyoxylylchlorid in 30 ml Dichlormethan werden 1,65 ml Triäthylamin und dann eine Lösung von 0,93 g 1-Naphthylessigsäure in 20 ml Dichlormethan zugesetzt. Nach 16-stündigem Rühren wird das Gemisch eingeengt und der Rückstand wird an Silicagel mit Dichlormethan gereinigt. Man erhält 295 mg des Furandions, Smp. 217-219°C.

### Beispiel 3

0,30 g 3-(1-Methyl-3-indolyl)-4-(3-methylphenyl)furan-2,5-dion werden mit 8 ml DMF und 60 ml einer 33%igen wässrigen Ammoniaklösung behandelt und 5 Stunden bei 150°C erhitzt und dann abkühlen gelassen. Der gebildete Niederschlag wird abfiltriert, mit Wasser gewaschen und getrocknet. Man erhält 162 mg 3-(1-Methyl-3-indolyl)-4-(3-methylphenyl)-1H-pyrrol -2,5-dion, Smp. 243°C.

Das Ausgangsfurandion wird wie folgt hergestellt:
1,5 g 1-Methylindol-3-glyoxylylchlorid in 30 ml Dichlormethan werden bei 0°C mit 2,17 ml Triäthylamin und 1,02 g 3-Methylphenylessigsäure behandelt. Das Gemisch wird auf Raumtemperatur erwärmen gelassen und über Nacht gerührt. Siliciumdioxid wird hinzugefügt und das Lösungsmittel wird abgedampft. Das Siliciumdioxid und adsorbierte Produkte werden an Silicagel mit 20% Aethylacetat und Petroleumäther gereinigt. Man erhält 307 mg des Furandions, Smp. 158-160°C.

### Beispiel 4

160 mg 3-(1-Benzothiophen-3-yl)-4-(1-methyl-3-indolyl)furan -2,5-dion werden mit 2 ml DMF und 20 ml einer 33%igen wässrigen Ammoniaklösung behandelt und das Gemisch wird 5 Stunden bei 140°C erhitzt. Das abgekühlte Gemisch wird filtriert und der Rückstand wird mit Wasser gewaschen und getrocknet. Der Feststoff wird an Silicagel mit 50% Aethylacetat in Petroleumäther gereinigt. Man erhält 20 mg 3-(1-Benzothiophen-3-yl)-4-(1-methyl-3-indolyl)-1H-pyrrol-2,5-dion, Smp. 250-255°C.

Das Ausgangsfurandion wird wie folgt hergestellt:
1,0 g 1-Methylindol-3-glyoxylylchlorid in 20 ml Dichlormethan werden mit 1,6 ml Triäthylamin und einer Lösung von 0.87 g 1-Benzothiophen-3-ylessigsäure in Dichlormethan behandelt. Das Gemisch wird 16 Stunden bei Raumtemperatur stehengelassen, dann eingeengt und der Rückstand wird an Silicagel mit 50% Aethylacetat in Hexan chromatographiert. Man erhält 0,33 g des Furandions, Smp. 165°C.

### Beispiel 5

0,28 g 3-(1-Methyl-3-indolyl)-4-(3-thienyl)furan-2,5-dion werden mit 10 ml DMF und 40 ml einer 33%igen wässrigen Ammoniaklösung behandelt. Das Gemisch wird 4 Stunden bei 140°C erhitzt. Die abgekühlte Lösung wird in 150 ml Wasser geschüttet und der entstandene Niederschlag wird abfiltriert und getrocknet. Man erhält 0,15 g 3-(1-Methyl-3-indolyl)-4-(3-thienyl)-1H-pyrrol-2,5-dion, Smp. 211-212°C.

Das Ausgangsfurandion wird wie folgt hergestellt:
1,1 g 1-Methylindol-3-glyoxylylchlorid in 10 ml Dichlormethan werden mit 1,65 ml Triäthylamin und einer Lösung von 0,71 g 3-Thiophenessigsäure in Dichlormethan behandelt. Nach 2-stündigem Rühren bei Raumtemperatur wird das Gemisch eingeengt und der Rückstand an Silicagel mit Dichlormethan gereinigt. Man erhält 0,42 g Furandion, Smp. 162-164°C.

### Beispiel 6

0,17 g 3-(5-Amino-1-methyl-3-indolyl)-4-(1-methyl-3-indolyl)furan -2,5-dion werden mit 4 ml DMF und 30 ml einer 33%igen wässrigen Ammoniaklösung behandelt und das Gemisch wird 4 Stunden bei 140°C erhitzt. Die abgekühlte Lösung wird filtriert und der Rückstand wird mit Wasser gewaschen. Man erhält 0,08 g 3-(5-Amino-1-methyl-3-indolyl)-4-(1-methyl-3-indolyl) -1H-pyrrol-2,5-dion, Smp. 254-256°C.

Das Ausgangsfurandion wird wie folgt hergestellt:
0,2 g 3-(1-Methyl-3-indolyl)-4-(1-methyl-5-nitro-3-indolyl)furan -2,5-dion in 50 ml THF wird über 0,2 g 10%igem Pd/C 23 Stunden hydriert. Das Gemisch wird filtriert und das Lösungsmittel wird abgedampft. Man erhält 0,17 g Furandion, Smp. 130-134°C.

### Beispiel 7

0,050 g des Produkts von Beispiel 6 werden 1 Stunde bei Raumtemperatur mit 10 ml Essigsäureanhydrid behandelt. Der Ueberschuss an Anhydrid wird abgedampft. Man erhält 0,039 g 3-(5-Acetamido-1-methyl-3-indolyl)-4-(1-methyl-3-indolyl) -1H-pyrrol-2,5-dion, Smp. 276-279°C.

### Beispiel 8

0,058 g 3-(5-Hydroxy-1-methyl-3-indolyl)-4-(1-methyl-3-indolyl)furan -2,5-dion werden mit 1,5 ml DMF und 20 ml einer 33%igen wässrigen Ammoniaklösung behandelt und das Gemisch wird 3 Stunden bei 140°C erhitzt. Das Lösungsmittel wird von der abgekühlten Lösung abgedampft und der Rückstand wird mit Wasser vermischt. Der entstandene Feststoff wird abfiltriert und getrocknet. Man erhält 0,018 g 3-(5-Hydroxy-1-methyl-3-indolyl)-4-(1-methyl-3-indolyl)-1H-pyrrol-2,5-dion, Smp. 284-287°C.

Das Ausgangsfurandion wird wie folgt hergestellt:
7,85 g 5-Methoxy-1-methylindol-3-glyoxylylchlorid in 100 ml Dichlormethan werden mit 10,8 ml Triäthylamin gefolgt durch 5,86 g 1-Methylindol-3-ylessigsäure behandelt. Nach 16 Stunden wird das Gemisch eingeengt und der Rückstand wird an Silicagel mit 1% Methanol in Dichlormethan chromatographiert.

0,10 g des erhaltenen 3-(5-Methoxy-1-methyl-3-indolyl)-4-(1-methyl -3-indolyl)furan-2,5-dion (Smp. 234-237°C) werden mit 3 ml Pyridin und 0,40 g Pyridinhydrochlorid behandelt und das Gemisch wird 3 Stunden bei 220°C erhitzt. Das Gemisch wird zwischen Dichlormethan und Wasser aufgeteilt und die organische Phase wird mit Wasser und dann mit 0,5M Salzsäure gewaschen. Die organische Phase wird getrocknet und eingeengt. Der Rückstand wird an Silicagel mit 1% Methanol in Dichlormethan chromatographiert. Man erhält 0,058 g des erwünschten Furandions, Smp. 128-132°C.

### Beispiel 9

Eine Lösung von 800 mg 3-(1-Methyl-3-indolyl)-4-(1-methyl-2-pyrrolyl)furan-2,5-dion in 6 ml DMF und 50 ml einer 33%igen wässrigen Ammoniaklösung wird 3 Stunden bei 130°C erhitzt. Der Niederschlag wird abfiltriert und getrocknet. Man erhält 400 mg 3-(1-Methyl-3-indolyl)-4-(1-methyl-2-pyrrolyl) -1H-pyrrol-2,5-dion, Smp. 248-250°C.

Das Ausgangsfurandion wird wie folgt hergestellt:
An 6,4 g 1-Methylindol-3-glyoxylylchlorid in 120 ml Dichlormethan und 8,0 ml Triäthylamin werden 4,0 g 1-Methylpyrrol-2-essigsäure unter einer Stickstoffatmosphäre zugesetzt. Nach 16 Stunden Rühren wird das Lösungsmittel abgedampft. Der Rückstand wird an Silicagel mit Aethylacetat/Petroleumäther (1:2) gereinigt. Man erhält 800 mg Furandion, Smp. 163-165°C.

### Beispiel 10

1,4 ml Acetaldehyddimethylacetal und 10 mg p-Toluolsulfonsäure werden einer Lösung von 250 mg 3,4-Bis(3-indolyl)-1H-pyrrol-2,5-dion in 40 ml Chloroform zugesetzt. Das entstandene Gemisch wird 18 Stunden unter Stickstoff bei Rückflusstemperatur erhitzt. Die erhaltene Lösung wird eingedampft und der Rückstand wird an Silicagel mit Aethylacetat/Petroleumäther (1:2) gereinigt. Umkristallisation aus Chloroform/Hexan ergibt 165 mg 3,4-Bis[1-(1-methoxyäthyl)-3-indolyl]-1H-pyrrol-2,5-dion, Smp. 222-224°C.

### Beispiel 11

220 mg Thiophenol und 1 Tropfen konzentrierte Salzsäure werden einer Lösung von 150 mg des Produkts von Beispiel 10 in 40 ml Dichlormethan zugesetzt. Die Lösung wird 2 Stunden unter Stickstoff gerührt. Das Lösungsmittel wird abgedampft und der Rückstand wird aus Diäthyläther/Hexan umkristallisiert. Man erhält 190 mg 3,4-Bis[1-(1-phenylthioäthyl)-3-indolyl]-1H-pyrrol -2,5-dion, Smp. 102-105°C.

### Beispiel 12

Eine Lösung von 4,12 g 2-Methylindol in 75 ml Benzol wird mit 9,2 ml einer 3M Lösung von Methylmagnesiumjodid in Diäthyläther behandelt und die resultierende Lösung wird 0,5 Stunden unter Stickstoff gerührt. 2,0 g Dibrommaleimid werden zugesetzt und das Gemisch wird 14 Stunden unter Rückfluss erhitzt. Die abgekühlte Lösung wird eingedampft, in 200 ml Dichlormethan gelöst und mit 100 ml 2M Salzsäure angesäuert. Die organische Schicht wird abgetrennt, mit 100 ml Wasser gewaschen, getrocknet und eingedampft. Der Rückstand wird mit Dichlormethan vermischt und der erhaltene Feststoff wird aus Aceton/Wasser umkristallisiert. Man erhält 1,1 g 3,4-Bis(2-methyl-3-indolyl)-1H-pyrrol-2,5-dion, Smp. 311-313°C.

### Beispiel 13

20 ml einer 1M Lösung von LiAlH₄ in Diäthyläther wird einer Lösung von 1,0 g 3,4-Bis(3-indolyl)-1H-pyrrol-2,5-dion in 140 ml THF zugesetzt. Das Gemisch wird 18 Stunden unter Stickstoff gerührt. Das Gemisch wird auf 0°C abgekühlt, mit 50 ml Wasser versetzt, dann auf pH 2 mit 2M Salzsäure angesäuert und mit Aethylacetat extrahiert. Die organischen Extrakte werden mit gesättigter Natriumbicarbonatlösung gewaschen, getrocknet und eingedampft. Der Rückstand wird an Silicagel mit 5-10% Methanol in Dichlormethan gereinigt. Das erste Eluat wird mit Aethylacetat/Hexan vermischt. Man erhält 175 mg 3,4-Bis(3-indolyl)-3-pyrrolin-2-on, Smp. 290-293°C (Zers.). Das zweite Eluat wird aus Aethylacetat/Chloroform kristallisiert. Man erhält 490 mg 5-Hydroxy-3,4-bis(3-indolyl)-3-pyrrolin2-on, Smp. 250°C (Zers.).

Das Ausgangspyrroldion wird wie folgt hergestellt:
Eine Lösung von 18,72 g Indol in 240 ml Benzol wird mit 48 ml einer 3M Lösung von Methylmagnesiumjodid in Diäthyläther behandelt und 0,5 Stunden unter Stickstoff gerührt. 10,2 g Dibrommaleimid werden zugesetzt und das Gemisch wird 65 Stunden unter Rückfluss erhitzt, dann abgekühlt und eingedampft. Der Rückstand wird zwischen Dichlormethan und 2M Salzsäure aufgeteilt und das unlösliche Material wird abfiltriert. Das Dichlormethanextrakt wird abgetrennt und getrocknet und das Lösungsmittel wird abgedampft. Das Produkt wird an Silicagel mit Aethylacetat/Petroleumäther gereinigt. Man erhält 6,0 g des Pyrroldions, Smp. 252-253°C nach Ausfällung aus Methanol/Wasser.

### Beispiel 14

820 mg Lawesson's-Reagens werden einer Lösung von 330 mg 3,4-Bis(3-indolyl)-1H-pyrrol-2,5-dion in 50 ml Dimethoxyäthan zugesetzt und das Gemisch wird 1 Stunde unter Rückfluss erhitzt. 410 mg Lawesson's-Reagens werden dann zugesetzt und das Gemisch wird 1 Stunde unter Rückfluss erhitzt. Das Lösungsmittel wird abgedampft und der Rückstand wird an Silicagel mit Aethylacetat/Hexan (1:4) gereinigt. Umkristallisation aus Diäthyläther/Hexan ergibt 30 mg 5-Thioxo-3,4-bis(3-indolyl)-3-pyrrolin-2-on, Smp. 254-257°C.

### Beispiel 15

260 mg einer 60%igen Dispersion von Natriumhydrid in Mineralöl werden einer Lösung von 295 mg Benzimidazol in 10 ml DMF hinzugefügt und das Gemisch wird 0,5 Stunden unter Stickstoff gerührt. 582 mg 3-Brom-4-(3-indolyl)-1H-pyrrol-2,5-dion werden zugesetzt und das Gemisch wird 18 Stunden bei 50°C erhitzt. Eine Lösung von 767 mg Benzimidazol und 260 mg Natriumhydrid in 10 ml DMF werden zugesetzt und das Gemisch wird 18 Stunden unter Stickstoff bei 90°C erhitzt. Das Lösungsmittel wird abgedampft und der Rückstand wird zwischen Dichlormethan und 2M Salzsäure verteilt. Der Niederschlag wird an Silicagel mit Aethylacetat/Petroleumäther gereinigt. Umkristallisation aus Aethylacetat ergibt 25 mg 3-(1-Benzimidazolyl)-4-(3-indolyl)-1H-pyrrol-2,5-dion, Smp. 310-320°C.

Das Ausgangspyrroldion wird wie folgt hergestellt:
Eine Lösung von 2,34 g Indol in 25 ml Benzol wird mit 13,4 ml einer 3M Lösung von Methylmagnesiumbromid in Diäthyläther behandelt. Die Lösung wird eine halbe Stunde gerührt und dann einer Lösung von 5,12 g Dibrommaleimid in 75 ml Benzol zugesetzt. Das Gemisch wird 16 Stunden gerührt, dann eingedampft und der Rückstand wird zwischen Dichlormethan und 2M Salzsäure aufgeteilt. Der Niederschlag wird abfiltriert und mit Diäthyläther vermischt. Man erhält 1,8 g des Pyrroldions, Smp. 204-205°C nach Umkristallisation aus Aethylacetat/Petroleumäther.

### Beispiel 16

Eine Lösung von 804 mg 3-(1-Methyl-3-indolyl)-4-[1-methyl-2-(methylthio) -3-indolyl]furan-2,5-dion in 12 ml DMF und 50 ml einer 33%igen wässrigen Ammoniaklösung wird 2 Stunden bei 130°C erhitzt. Das Produkt wird abfiltriert und getrocknet. Man erhält 675 mg 3-(1-Methyl-3-indolyl)-4-[1-methyl-2-(methylthio) -3-indolyl]-1H-pyrrol-2,5-dion, Smp. 281-283°C.

Das Ausgangsfurandion wird wie folgt hergestellt:
1,40 g Oxalylchlorid werden einer Lösung von 1,77 g 1-Methyl-2-methylthioindol in 45 ml Dichlormethan bei 0°C zugesetzt. Die Lösung wird auf Raumtemperatur erwärmen gelassen und das Lösungsmittel wird abgedampft. Einer Lösung des erhaltenen Produkts in Dichlormethan werden 2,02 g Triäthylamin und 1,89 g 1-Methylindol-3-ylessigsäure unter einer Stickstoffatmosphäre zugesetzt. Nach 18 Stunden Rühren wird das Lösungsmittel abgedampft. Der Rückstand wird an Silicagel mit Aethylacetat/Hexan gereinigt. Man erhält 1,32 g des Furandions, Smp. 230-232°C nach Umkristallisation aus Dichlormethan/Hexan.

### Beispiel 17

270 mg m-Chlorperbenzoesäure werden unter Rühren einer Lösung von 500 mg des Produkts von Beispiel 16 in 250 ml Dichlormethan bei 0°C zugesetzt. Die entstandene Lösung wird 1 Stunde bei 0°C gerührt und dann mit gesättigter Natriumbicarbonatlösung und Wasser gewaschen. Die Lösung wird getrocknet und eingedampft. Der Rückstand wird mit Methanol vermischt. Man erhält 505 mg 3-(1-Methyl-3-indolyl)-4-[1-methyl-2-(methylsulfinyl) -3-indolyl]-1H-pyrrol-2,5-dion, Smp. 300°C.

### Beispiel 18

Eine Lösung von 4,9 g Indol in 50 ml Benzol wird mit 19 ml einer 3M Lösung von Methylmagnesiumjodid in Diäthyläther behandelt und 15 Minuten unter Stickstoff gerührt. 3,5 g 3-Brom-4-phenyl-1H-pyrrol-2,5-dion werden zugesetzt und das Gemisch wird 18 Stunden gerührt. Das Lösungsmittel wird abgedampft und der Rückstand wird in 250 ml Dichlormethan und 50 ml 2M Salzsäure aufgelöst. Die organischen Extrakte werden mit Wasser gewaschen, getrocknet und eingedampft. Der Rückstand wird an Silicagel mit Aethylacetat/Petroleumäther gereinigt. Das Mischen mit Dichlormethan und die Umkristallisation aus Methanol ergeben 1,40 g 3-(3-Indolyl)-4-phenyl-1H-pyrrol-2,5-dion, Smp. 256°C.

Das Ausgangspyrroldion wird wie folgt hergestellt:
5,0 g Phenylsuccinimid werden bei 100°C erhitzt und 3,1 ml Brom werden über einer Zeitspanne von 1 Stunde zugesetzt. Dann wird 15 Minuten bei 120°C erhitzt. Nach Abkühlen werden 25 ml Wasser zugesetzt und das Gemisch wird 10 Minuten gerührt und dann das Produkt abfiltriert. Umkristallisieren aus Aethanol/Wasser ergibt 3,55 g des Pyrroldions, Smp. 181°C.

### Beispiel 19

0,6 ml einer 3M Lösung von Methylmagnesiumbromid in Diäthyläther wird unter Stickstoff einer Suspension von 105 mg Indol in 20 ml Benzol zugesetzt. Das Gemisch wird 0,5 Stunden gerührt. 100 mg 3-Brom-4-(5-methoxy-3-indolyl)-1H-pyrrol-2,5-dion werden zugesetzt und das Gemisch wird 5 Tage unter Rückfluss erhitzt. Nach Abkühlen wird der Rückstand aufgeteilt zwischen Dichlormethan und 2M Salzsäure. Die organischen Extrakte werden mit Wasser gewaschen, getrocknet und eingedampft. Der Rückstand wird an Silicagel mit 1% Methanol in Dichlormethan gereinigt und dann mit 50% Methanol/0,1% Trifluoressigsäure/Wasser an Spherisorb gereinigt. Man erhält 3 mg 3-(3-Indolyl)-4-(5-methoxy-3-indolyl)-1H-pyrrol-2,5-dion, Smp. 280°C.

Das Ausgangspyrroldion wird wie folgt hergestellt:
4,0 ml einer 3M Lösung von Methylmagnesiumbromid in Diäthyläther werden einer Lösung von 2,00 g 5-Methoxyindol in 25 ml Benzol unter Stickstoff zugesetzt. Die resultierende Lösung wird 0,5 Stunden gerührt. Nach Zusatz von 0,87 g Dibrommaleimid wird das Gemisch 24 Stunden unter Rückfluss erhitzt. Nach Abkühlen wird das Lösungsmittel abgedampft und der Rückstand wird zwischen Dichlormethan und 2M Salzsäure aufgeteilt. Die organischen Extrakte werden mit Wasser gewaschen, getrocknet und eingedampft. Der Rückstand wird an Silicagel mit 5% Methanol in Dichlormethan, 2% Methanol in Dichlormethan und Aethylacetat/Petroleumäther (1:2) gereinigt. Man erhält 100 mg des Pyrroldions, Smp. 225°C (Zers.).

### Beispiel 20

1,4 ml einer 3M Lösung von Methylmagnesiumjodid in Diäthyläther werden einer Lösung von 360 mg Indol in 20 ml Benzol unter Stickstoff zugesetzt. Nach 10-minütigem Rühren bei Raumtemperatur werden 300 mg 3-Brom-4-(4-nitrophenyl)-1H-pyrrol-2,5-dion zugesetzt und das Gemisch wird 4 Tage unter Rückfluss erhitzt. Nach Abkühlen wird die Lösung eingedampft und der Rückstand wird zwischen Dichlormethan und 2M Salzsäure aufgeteilt. Die organische Phase wird mit Wasser gewaschen, getrocknet und eingedampft. Der Rückstand wird an Silicagel mit Dichlormethan und 1% Methanol in Dichlormethan und dann an Hypersil mit 20% Methanol/Wasser gereinigt. Man erhält 3 mg 3-(3-Indolyl)-4-(4-nitrophenyl)-1H-pyrrol-2,5-dion, Smp. 125°C (Zers.).

Das Ausgangspyrroldion wird wie folgt hergestellt:
Einer Lösung von 2,33 g p-Nitrophenylsuccinimid in 150 ml Diäthyläther werden 1,2 ml Brom zugesetzt. Die Lösung wird 4 Tage unter Rückfluss erhitzt, wobei zusätzliche 1,2 ml Brom zugesetzt werden. Nach Abkühlen des Gemisches wird mit gesättigter Natriumthiosulfatlösung und Wasser gewaschen, dann getrocknet und eingedampft. Der Rückstand wird an Silicagel mit Diäthyläther/Petroleumäther gereinigt. Umkristallisation aus Toluol ergibt 350 mg des Pyrroldions, Smp. 165°C.

### Beispiel 21

Eine Lösung von 200 mg 3-[1-(3-Acetoxypropyl)-3-indolyl]-4-(1-methyl -3-indolyl)furan-2,5-dion in 1 ml DMF und 2 ml 33%igem wässrigem Ammonia wird 2 Stunden bei 100°C erhitzt. 50 ml Wasser werden zugesetzt und der resultierende Feststoff wird abfiltriert, getrocknet und aus Aethylacetat umkristallisiert. Man erhält 85 mg 3-[1-(3-Hydroxypropyl)-3-indolyl]-4-(1-methyl -3-indolyl)-1H-pyrrol-2,5-dion, Smp. 185-187°C.

Das Ausgangsfurandion wird wie folgt hergestellt:
367 µl Oxalylchlorid werden einer Lösung von 868 mg 1-(3-Acetoxypropyl)indol in 10 ml Dichlormethan bei 0°C zugesetzt. Die Lösung wird 3 Stunden gerührt und das Lösungsmittel wird dann abgedampft. Der Rückstand wird in Dichlormethan und Triäthylamin aufgelöst und 756 mg 1-Methylindol-3-ylessigsäure werden unter Stickstoff zugesetzt. Nach 18-stündigem Rühren wird das Lösungsmittel abgedampft und der Rückstand wird an Silicagel mit Aethylacetat/Petroleumäther gereinigt. Umkristallisation aus Aethylacetat/Hexan ergibt 290 mg des Furandions, Smp. 94-96°C.

### Beispiel 22

Eine Lösung von 200 mg 3-[1-(2-Methoxycarbonyläthyl)-3-indolyl]-4-(1-methyl -3-indolyl)furan-2,5-dion in 1 ml DMF und 2 ml 33%igem wässrigem Ammoniak wird 0,75 Stunden bei 100°C erhitzt. 30 ml Aethylacetat werden der abgekühlten Lösung zugesetzt und die organische Phase wird abgetrennt und mit gesättigter Natriumbicarbonatlösung gewaschen. Die organische Phase wird getrocknet und das Lösungsmittel wird abgedampft. Umkristallisation aus Aethylacetat/Petroleumäther ergibt 40 mg 3-[1-(2-Carbamoyläthyl)-3-indolyl]-4-(4-methyl-3-indolyl) -1H-pyrrol-2,5-dion, Smp. 243-247°C.

Das Ausgangsfurandion wird wie folgt hergestellt:
Eine Lösung von 622 µl Oxalylchlorid und 1,5 g 1-[2-(Methoxycarbonyl)äthyl]indol in 20 ml Dichlormethan wird 10 Minuten bei 0°C und dann 2 Stunden bei Raumtemperatur gerührt. Das Lösungsmittel wird abgedampft. Der Rückstand wird in Dichlormethan gelöst. 2,03 ml Triäthylamin und 1,4 g 1-Methylindol-3-ylessigsäure werden unter Stickstoff zugesetzt. Nach 18-stündigem Rühren wird das Lösungsmittel abgedampft und der Rückstand wird an Silicagel mit Dichlormethan und dann mit Aethylacetat/Petroleumäther gereinigt. Umkristallisation aus Aethylacetat/Petroleumäther ergibt 590 mg des Furandions, Smp. 150-152°C.

### Beispiel 23

Eine Lösung von 150 mg 3-[1-(2-Carboxyäthyl)-3-indolyl]-4-(1-methyl -3-indolyl)furan-2,5-dion in 1 ml DMF und 2 ml einer 33%igen wässrigen Ammoniaklösung wird 1 Stunde bei 100°C erhitzt. Die abgekühlte Lösung wird eingedampft und der Rückstand wird aus Aethylacetat/Petroleumäther kristallisiert. Man erhält 90 mg 3-[1-(2-Carboxyäthyl)-3-indolyl]-4-(1-methyl -3-indolyl)-1H-pyrrol-2,5-dion, Smp. 256-258°C.

Das Ausgangsfurandion wird wie folgt hergestellt:
Eine Lösung von 200 mg 3-[1-(2-Methoxycarbonyläthyl)-3-indolyl]-4-(1-methyl -3-indolyl)furan-2,5-dion in 4 ml Aethanol wird 1 Stunde mit 180 mg KOH erhitzt. Das Lösungsmittel wird abgedampft und der Rückstand wird mit 2M Salzsäure angesäuert und mit Dichlormethan extrahiert. Die organische Phase wird abgetrennt, getrocknet und eingedampft. Der Rückstand wird mit Aethylacetat vermischt. Man erhält 170 mg des Furandions, Smp. 222-224°C.

### Beispiel 24

Eine Lösung von 40 mg des Produkts von Beispiel 23 in 5 ml Methanol wird 4 Stunden mit 10 mg p-Toluolsulfonsäure unter Rückfluss erhitzt. Das Lösungsmittel wird abgedampft und der Rückstand wird aus Aethylacetat kristallisiert. Man erhält 25 mg 3-[1-(2-Methoxycarbonyläthyl)-3-indolyl]-4-(1-methyl -3-indolyl)-1H-pyrrol-2,5-dion, Smp. 209-211°C.

### Beispiel 25

Eine Lösung von 2,50 g 3-[1-(3-Azidopropyl)-3-indolyl]-4-(1-methyl -3-indolyl)furan-2,5-dion in 13 ml DMF und 18 ml einer 33%igen wässrigen Ammoniaklösung wird 4 Stunden bei 140°C erhitzt. Das Produkt wird aus der abgekühlen Lösung abfiltriert. Man erhält 2,27 g 3-[1-(3-Azidopropyl)-3-indolyl]-4-(1-methyl -3-indolyl)-1H-pyrrol-2,5-dion, Smp. 222-224°C.

Das Ausgangsfurandion wird wie folgt hergestellt:
a) Einer Lösung von 23,4 g Indol in 200 ml DMF werden bei 10°C 22,4 g Kaliumhydroxid und 101 g 1,3-Dibrompropan zugesetzt. Das Gemisch wird 3 Tage unter Stickstoff gerührt. Der entstandene Feststoff wird abfiltriert und das Filtrat wird eingedampft. Der Rückstand wird an Silicagel mit 5% Diäthyläther in Petroleumäther chromatographiert. Man erhält 14,7 g 1-(3-Brompropyl)indol.
b) 4,2 ml Oxalylchlorid werden einer Lösung von 11,75 g 1-(3-Brompropyl)indol in 125 ml Dichlormethan bei 0°C zugesetzt. Die Lösung wird 2 Stunden gerührt und das Lösungsmittel wird dann abgedampft. Der Rückstand wird in Dichlormethan gelöst und mit 17,4 ml Diisopropyläthylamin und 9,45 g 1-Methylindol-3-ylessigsäure unter Stickstoff behandelt. Nach 3-tägigem Rühren wird das Lösungsmittel abgedampft und der Rückstand wird an Silicagel mit Dichlormethan gereinigt. Umkristallisation aus Aethylacetat/Petroleumäther ergibt 5,09 g 3-[1-(3-Brompropyl)-3-indolyl]-4-(1-methyl -3-indolyl)furan-2,5-dion, Smp. 168-170°C.
c) Eine Lösung von 2,8 g des Produkts von b) in 50 ml DMF wird 2 Stunden bei Raumtemperatur und dann 2 Stunden bei 60°C mit 1,25 g Natriumazid gerührt. Das Lösungsmittel wird abgedampft und der Rückstand wird zwischen Dichlormethan und Wasser verteilt. Die organische Phase wird mit Wasser gewaschen, getrocknet und eingedampft. Kristallisation aus Aethylacetat ergibt 2,5 g des erwünschten Furandions, Smp. 154-156°C.

### Beispiel 26

a) Eine Lösung von 1,9 g des Produkts von Beispiel 25 in 300 ml Aethylacetat wird 3 Tage über 190 mg 10%igem Pd/C hydriert. Die Lösung wird filtriert und das Filtrat wird eingedampft. Der resultierende Niederschlag wird abfiltriert und getrocknet. Man erhält 1,57 g 3-[1-(3-Aminopropyl)-3-indolyl]-4-(1-methyl-3-indolyl) -1H-pyrrol-2,5-dion, Smp. 195-197°C.
b) 1,3 g des Produkts von a) werden in 500 ml Aethylacetat aufgenommen und mit einer gesättigten Lösung von Chlorwasserstoff in Aethylacetat behandelt. Das Gemisch wird 2 Stunden gerührt und dann filtriert. Man erhält 1,5 g 3-[1-(3-Aminopropyl)-3-indolyl]-4-(1-methyl-3-indolyl) -1H-pyrrol-2,5-dionhydrochlorid, Smp. 215-220°C.

### Beispiel 27

40 mg einer 60%igen Suspension von Natriumhydrid in Mineralöl werden einer Lösung von 327 mg 3,4-Bis(3-indolyl)-1H-pyrrol-2,5-dion in 5 ml DMF bei 0°C unter Stickstoff zugesetzt. Nach 0,5 Stunden wird das Gemisch auf -20°C abgekühlt und 108 mg Trimethylsilylchlorid werden zugesetzt. Das Gemisch wird auf Raumtemperatur erwärmen gelassen, dann auf 0°C abgekühlt und dann werden zusätzliche 80 mg Natriumhydrid zugesetzt. Nach 0,5 Stunden bei 0°C werden 116 mg Propylenoxid zugesetzt und das Gemisch wird über Nacht gerührt. 5 ml Wasser werden zugesetzt und das Gemisch wird mit Dichlormethan extrahiert. Die organische Phase wird getrocknet und eingedampft. Der Rückstand wird an Silicagel mit Aethylacetat/Petroleumäther gereinigt. Umkristallisation aus Diäthyläther/Petroleumäther ergibt 30 mg 3,4-Bis[1-(2-hydroxypropyl)-3-indolyl]-1H-pyrrol -2,5-dion, Smp. 133-135°C.

### Beispiel 28

3,4-Bis(1-methoxymethyl-3-indolyl)-1H-pyrrol-2,5-dion, Smp. 178-182°C wird hergestellt im zu Beispiel 10 analoger Weise.

### Beispiel 29

In zu Beispiel 11 analoger Weise werden hergestellt:
3,4-Bis[1-[1-(1-hydroxyäthylthio)äthyl]-3-indolyl]-1H-pyrrol-2,5-dion, Smp. 191-194°C;
3,4-Bis[1-[1-(2-mercaptoäthylthio)äthyl]-3-indolyl]-1H-pyrrol-2,5-dion, Smp. 97-99°C; und
3,4-Bis[1-[1-(carboxymethylthio)äthyl]-3-indolyl]-1H-pyrrol-2,5-dion, Smp. 111-114°C.

### Beispiel 30

In zu Beispiel 16 analoger Weise werden hergestellt:
3,4-Bis(1-methyl-3-indolyl)-1H-pyrrol-2,5-dion, Smp. 355°C;
3-(4-Methoxy-1-methyl-3-indolyl)-4-(1-methyl-3-indolyl)-1H-pyrrol-2,5-dion, Smp. 288-290°C;
3-(1-Methyl-5-methylthio-3-indolyl)-4-(1-methyl-3-indolyl)-1H-pyrrol-2,5-dion, Smp. 260°C;
3-(6-Methoxy-1-methyl-3-indolyl)-4-(1-methyl-3-indolyl)-1H-pyrrol-2,5-dion, Smp. 267°C;
3-(7-Methoxy-1-methyl-3-indolyl)-4-(1-methyl-3-indolyl)-1H-pyrrol-2,5-dion, Smp. 255°C;
3,4-Bis(1-benzyl-3-indolyl)-1H-pyrrol-2,5-dion, Smp. 108°C; und
3-(5-Chlor-1-methyl-3-indolyl)-4-(1-methyl-3-indolyl)-1H-pyrrol-2,5-dion, Smp. 270-271°C.

### Beispiel 31

3-(1-Methyl-5-methylsulfinyl-3-indolyl)-4-(1-methyl-3-indolyl)-1H-pyrrol-2,5-dion, Smp. 292°C wird in zu Beispiel 17 analoger Weise hergestellt.

### Beispiel 32

In zu Beispiel 21 analoger Weise werden hergestellt:
3-[1-(4-Hydroxybutyl)-3-indolyl]-4-(1-methyl-3-indolyl)-1H-pyrrol-2,5-dion, Smp. 185-188°C;
3-(1-alpha-D-Glucopyranosyl-3-indolyl)-4-(1-methyl-3-indolyl)-1H-pyrrol-2,5-dion, Smp. 210-215°C;
3,4-Bis[1-(4-hydroxybutyl-3-indolyl)]-1H-pyrrol-2,5-dion, Smp. 192-193°C; und
3-[1-(5-Hydroxypentyl)-3-indolyl]-4-(1-methyl-3-indolyl)-1H-pyrrol-2,5-dion, Smp. 179-181°C.

### Beispiel 33

3-[1-(4-Carbamoylbutyl)-3-indolyl]-4-(1-methyl-3-indolyl)-1H-pyrrol-2,5-dion, Smp. 247-249°C wird in zu Beispiel 22 analoger Weise hergestellt.

### Beispiel 34

In zu Beispiel 23 analoger Weise werden hergestellt:
3-[1-(3-Carboxypropyl)-3-indolyl]-4-(1-methyl-3-indolyl)-1H-pyrrol-2,5-dion, Smp. 238-240°C; und
3-[1-(4-Carboxybutyl)-3-indolyl]-4-(1-methyl-3-indolyl)-1H-pyrrol-2,5-dion, Smp. 234-238°C.

### Beispiel 35

In zu Beispiel 24 analoger Weise werden hergestellt:
3-[1-[3-(Methoxycarbonyl)propyl]-3-indolyl]-4-(1-methyl-3-indolyl)-1H-pyrrol-2,5-dion, Smp. 208-210°C; und
3-[1-[4-(Methoxycarbonyl)butyl]-3-indolyl]-4-(1-methyl-3-indolyl)-1H-pyrrol-2,5-dion, Smp. 138-140°C.

### Beispiel 36

In zu Beispiel 25 analoger Weise werden hergestellt:
3-[1-(4-Azidobutyl)-3-indolyl]-4-(1-methyl-3-indolyl)-1H-pyrrol-2,5-dion, Smp. 196-198°C; und
3-[1-(5-Azidopentyl)-3-indolyl]-4-(1-methyl-3-indolyl)-1H-pyrrol-2,5-dion, Smp. 170-172°C.

### Beispiel 37

In zu Beispiel 1 analoger Weise werden hergestellt:
3-(1-Benzyl-3-indolyl)-4-(1-methyl-3-indolyl)-1H-pyrrol-2,5-dion, Smp. 261-262°C;
3-(5-Methoxy-1-methyl-3-indolyl)-4-(1-methyl-3-indolyl)-1H-pyrrol-2,5-dion, Smp. 240-245°C;
3-(5-Benzyloxy-1-methyl-3-indolyl)-4-(1-methyl-3-indolyl)-1H-pyrrol-2,5-dion, Smp. 215-218°C;
3-(1-Methyl-3-indolyl)-4-(1-methyl-7-nitro-3-indolyl)-1H-pyrrol-2,5-dion, Smp. 264-266°C;
3-(1-Methyl-3-indolyl)-4-(1-methyl-6-nitro-3-indolyl)-1H-pyrrol-2,5-dion, Smp. 285-287°C;
3-(1-Methyl-3-indolyl)-4-(1,5-dimethyl-3-indolyl)-1H-pyrrol-2,5-dion, Smp. 283-285°C;
3-(1,7-Dimethyl-3-indolyl)-4-(1-methyl-3-indolyl)-1H-pyrrol-2,5-dion, Smp. >300°C;
3-(6-Chlor-1-methyl-3-indolyl)-4-(1-methyl-3-indolyl)-1H-pyrrol-2,5-dion, Smp. 280-282°C;
3-(1-Methyl-3-indolyl)-4-(1-methyl-4-nitro-3-indolyl)-1H-pyrrol-2,5-dion, Smp. 315-316°C; und
3-(1,4-Dimethyl-3-indolyl)-4-(1-methyl-3-indolyl)-1H-pyrrol-2,5-dion, Smp. 292-293°C.

### Beispiel 38

In zu Beispiel 3 analoger Weise werden hergestellt:
3-(1-Methyl-3-indolyl)-4-phenyl-1H-pyrrol-2,5-dion, Smp. 243°C (Zers.);
3-(4-Methoxyphenyl)-4-(1-methyl-3-indolyl)-1H-pyrrol-2,5-dion, Smp. 262°C;
3-(4-Chlorphenyl)-4-(1-methyl-3-indolyl)-1H-pyrrol-2,5-dion, Smp. 268-270°C;
3-(1-Methyl-3-indolyl)-4-[4-(methylthio)phenyl]-1H-pyrrol-2,5-dion, Smp. 266-267°C;
3-(1-Methyl-3-indolyl)-4-(2-nitrophenyl)-1H-pyrrol-2,5-dion, Smp. 230-231°C;
3-(4-Aminophenyl)-4-(1-methyl-3-indolyl)-1H-pyrrol-2,5-dion, Smp. 297°C;
3-(1-Methyl-3-indolyl)-4-(3-nitrophenyl)-1H-pyrrol-2,5-dion, Smp. 248°C;
3-(3-Chlorphenyl)-4-(1-methyl-3-indolyl)-1H-pyrrol-2,5-dion, Smp. 224-225°C;
3-(1-Methyl-3-indolyl)-4-(2-methylphenyl)-1H-pyrrol-2,5-dion, Smp. 245-247°C;
3-(3-Bromphenyl)-4-(1-methyl-3-indolyl)-1H-pyrrol-2,5-dion, Smp. 219-220°C;
3-(2,5-Dimethylphenyl)-4-(1-methyl-3-indolyl)-1H-pyrrol-2,5-dion, Smp. 262-263°C;
3-(2-Chlorphenyl)-4-(1-methyl-3-indolyl)-1H-pyrrol-2,5-dion, Smp. 238-239°C;
3-(1-Methyl-3-indolyl)-4-(2-trifluormethylphenyl)-1H-pyrrol-2,5-dion, Smp. 237-238°C; und
3-(1-Methyl-3-indolyl)-4-(3-trifluormethylphenyl)-1H-pyrrol-2,5-dion, Smp. 187-188°C.

### Beispiel 39

3-(1-Methyl-3-indolyl)-4-(2-naphthyl)-1H-pyrrol-2,5-dion, Smp. 289°C (Zers.) wird in zu Beispiel 2 analoger Weise hergestellt.

### Beispiel 40

3-(1-Methyl-3-indolyl)-4-(2-thienyl)-1H-pyrrol-2,5-dion, Smp. 244-246°C wird in zu Beispiel 5 analoger Weise hergestellt.

### Beispiel 41

In zu Beispiel 6 analoger Weise werden hergestellt:
3-(7-Amino-1-methyl-3-indolyl)-4-(1-methyl-3-indolyl)-1H-pyrrol-2,5-dion, Smp. >300°C;
3-(6-Amino-1-methyl-3-indolyl)-4-(1-methyl-3-indolyl)-1H-pyrrol-2,5-dion, Smp. 264-267°C; und
3-(3-Aminophenyl)-4-(1-methyl-3-indolyl)-1H-pyrrol-2,5-dion, Smp. 259°C.

### Beispiel 42

In zu Beispiel 7 analoger Weise werden hergestellt:
3-(7-Acetamido-1-methyl-3-indolyl)-4-(1-methyl-3-indolyl)-1H-pyrrol-2,5-dion, Smp. >300°C; und
3-(6-Acetamido-1-methyl-3-indolyl)-4-(1-methyl-3-indolyl)-1H-pyrrol-2,5-dion, Smp. >300°C.

### Beispiel 43

In zu Beispiel 17 analoger Weise werden hergestellt:
3-(1-Methyl-3-indolyl)-4-[4-(methylsulfonyl)phenyl]-1H-pyrrol-2,5-dion, Smp. 265°C; und
3-(1-Methyl-3-indolyl)-4-[4-(methylsulfinyl)phenyl]-1H-pyrrol-2,5-dion, Smp. 256-258°C.

### Beispiel 44

In zu Beispiel 1 analoger Weise werden hergestellt:
3-(1-Methyl-3-indolyl)-4-(1,2-dimethyl-3-indolyl)-1H-pyrrol-2,5-dion, Smp. 305-306°C; und
3-(1-Methyl-2-indolyl)-4-(1-methyl-3-indolyl)-1H-pyrrol-2,5-dion, Smp. >300°C.

### Beispiel 45

In zu Beispiel 3 analoger Weise werden hergestellt:
3-(1-Methyl-3-indolyl)-4-(2,3-dimethylphenyl)-1H-pyrrol-2,5-dion, Smp. 275-276°C;
3-(3,5-Dichlorphenyl)-4-(1-methyl-3-indolyl)-1H-pyrrol-2,5-dion, Smp. 197-200°C;
3-(2,3,6-Trichlorphenyl)-4-(1-methyl-3-indolyl)-1H-pyrrol-2,5-dion, Smp. 306-309°C; und
3-(2,6-Dichlorphenyl)-4-(1-methyl-3-indolyl)-1H-pyrrol-2,5-dion, Smp. 285-286°C.

### Beispiel 46

Ein Gemisch von 163 mg 3-(1-Indolyl)-4-(1-methyl-3-indolyl)furan-2,5-dion, 2,6 g Hexamethyldisilazan, 0,6 g Methanol und 50 ml Toluol wird 1 Stunde bei 40°C und dann 1 Stunde bei 110°C gerührt. Flüchtiges Material wird abgedampft und der Rückstand wird an Silicagel mit 10% Methanol in Dichlormethan chromatographiert. Man erhält 75 mg 3-(1-Indolyl)-4-(1-methyl-3-indolyl)-1H-pyrrol-2,5-dion, Smp. 235-236°C.

Das Ausgangsfurandion wird wie folgt hergestellt:
876 mg Indol-1-ylessigsäure in 50 ml Dichlormethan wird mit 1,65 ml Diisopropyläthylamin und dann mit einer Lösung von 1,10 g 1-Methylindol-3-glyoxylylchlorid in 50 ml Dichlormethan behandelt. Das Gemisch wird 3 Stunden gerührt und dann eingeengt. Der Rückstand wird an Silicagel mit Dichlormethan chromatographiert. Man erhält 430 mg des Furandions, Smp. 164-166°C.

### Beispiel 47

3-(3-Benzofuranyl)-4-(1-methyl-3-indolyl)-1H-pyrrol-2,5-dion, Smp. 183-185°C wird in zu Beispiel 46 analoger Weise hergestellt.

### Beispiel 48

200 mg des Produkts von Beispiel 26b) in 10 ml DMF werden mit einer Lösung von 0,85 mg 1,1′-Thiocarbonyldiimidazol in 2 ml THF behandelt und das Gemisch wird 16 Stunden gerührt. Die Lösungsmittel werden abgedampft und der Rückstand wird an Silicagel mit 10% Methanol in Dichlormethan chromatographiert. Man erhält 129 mg 3-[1-(3-Isothiocyanatopropyl)-3-indolyl]-4-(1-methyl-3-indolyl)-1H-pyrrol-2,5-dion, Smp. 219-221°C.

### Beispiel 49

Eine Lösung von 100 mg des Produkts von Beispiel 26b) in 10 ml DMF wird mit einer Lösung von 40 mg 1,1′-Carbonyldiimidazol in 2 ml THF behandelt. Das Gemisch wird 16 Stunden gerührt. Die Lösungsmittel werden abgedampft und der Rückstand wird an Silicagel mit Chloroform/Methanol/Essigsäure/Wasser (60:18:2:3) chromatographiert. Man erhält 90 mg 3-[1-[3-(1-Imidazolylcarboxamido)propyl]-3-indolyl] -4-(1-methyl-3-indolyl)-1H-pyrrol-2,5-dion, Smp. 145-148°C.

### Beispiel 50

Eine Suspension von 500 mg des Produkts von Beispiel 26b) in 100 ml Aethanol wird einem Gemisch von 116 mg Natriumcarbonat und 177 mg Dimethyl-N-cyanodithioiminocarbonat zugesetzt. Nach 16 Stunden werden zusätzliche 160 mg Dimethyl-N-cyanodithioiminocarbonat zugesetzt und das Gemisch wird 2 Tage weitergerührt. Das Lösungsmittel wird abgedampft und der Rückstand wird an Silicagel mit Dichlormethan und dann Aethylacetat chromatographiert. Man erhält 120 mg 3-[1-[(3-Cyano-2-methylisothioureido)propyl]-3-indolyl] -4-(1-methyl-3-indolyl)-1H-pyrrol-2,5-dion, Smp. 236-238°C.

### Beispiel 51

Eine Lösung von 200 mg des Produkts von Beispiel 26a) in 10 ml DMF wird mit einer Lösung von 83 mg 1,1-Bis(methylthio)-2-nitroäthylen in 10 ml Acetonitril behandelt und das Gemisch wird 3 Tage bei 85°C erhitzt. Abdampfen des Lösungsmittels und Chromatographie des Rückstandes an Silicagel mit 10% Methanol in Dichlormethan ergeben 154 mg 3-(1-Methyl-3-indolyl)-4-[1-[3-[[1-(methylthio) -2-nitrovinyl]amino]propyl]-3-indolyl]-1H-pyrrol-2,5-dion, Smp. 144-146°C.

### Beispiel 52

Eine Lösung von 175 mg des Produkts von Beispiel 49 in 10 ml DMF wird mit 10 ml einer äthanolischen Ammoniaklösung behandelt. Das Gemisch wird 3 Stunden gerührt und dann werden die Lösungsmittel abgedampft. Der Rückstand wird aus Aethanol kristallisiert. Der entstandene Feststoff wird an 1%-20% Methanol in Dichlormethan gereinigt. Man erhält 43 mg 3-(1-Methyl-3-indolyl)-4-[1-(3-ureidopropyl)-3-indolyl] -1H-pyrrol-2,5-dion, Smp. 248-250°C.

### Beispiel 53

Eine Lösung von 150 mg 3,5-Dimethylpyrazol-1-carboxamidinenitrat in 10 ml Aethanol wird mit 200 mg des Produkts von Beispiel 26a) behandelt und das Gemisch wird 3 Tage unter Rückfluss erhitzt. Das Lösungsmittel wird abgedampft und der Rückstand wird an Silicagel mit Dichlormethan/Methanol/Essigsäure/Wasser (60:18:2:3) chromatographiert. Man erhält 53 mg 3-[1-(3-Guanidinopropyl)-3-indolyl]-4-(1-methyl-3-indolyl) -1H-pyrrol-2,5-dionnitrat, Smp. 179-181°C.

### Beispiel 54

Eine Lösung von 0,411 g 3-[1-(3-Acetoxypropyl)-3-indolyl]-4-(2-nitrophenyl)furan -2,5-dion in 50 ml Chloroform wird mit einem Gemisch von 1,53 g 1,1,1,3,3,3-Hexamethyldisilazan und 0,3 g Methanol behandelt und das Gemisch wird 1 Stunde bei 60°C erhitzt. Zusätzliche 1,53 g Hexamethyldisilazan und 0,3 g Methanol werden zugesetzt und das Erhitzen wird über Nacht fortgesetzt, bevor man zusätzliche 1,53 g Hexamethyldisilazan und 0,3 g Methanol zusetzt. Das Gemisch wird 1 Stunde weiter erhitzt. Die Lösungsmittel werden abgedampft und der Rückstand wird an Silicagel mit 5% Methanol in Dichlormethan chromatographiert. Man erhält 130 mg 3-[1-(3-Acetoxypropyl)-3-indolyl]-4-(2-nitrophenyl) -1H-pyrrol-2,5-dion, Smp. 77-78°C.

Das Ausgangsfurandion wird wie folgt hergestellt:
3,2 g Oxalylchlorid werden einer Lösung von 5,0 g 1-(3-Acetoxypropyl)indol in 100 ml Dichlormethan bei 0°C zugesetzt. Das Gemisch wird auf Raumtemperatur erwärmen gelassen und 3 Stunden gerührt bevor man es eindampft. Der entstandene Feststoff wird mit Dichlormethan und 5,5 g Triäthylamin und dann mit 3,9 g 2-Nitrophenylessigsäure behandelt. Das Gemisch wird 16 Stunden gerührt und die Lösungsmittel werden abgedampft. Der Rückstand wird an Silicagel mit Aethylacetat chromatographiert. Man erhält das Furandion.

### Beispiel 55

Eine Lösung von 40 mg Natriumhydroxid in 5 ml Aethanol wird einer Lösung von 400 mg des Produkts von Beispiel 54 in 10 ml Aethanol zugesetzt. Nach 3 Stunden Rühren wird das Lösungsmittel unter vermindertem Druck entfernt und der Rückstand wird an Silicagel mit 10% Methanol in Dichlormethan chromatographiert. Man erhält 190 mg 3-[1-(3-Hydroxypropyl)-3-indolyl]-4-(2-nitrophenyl)-1H-pyrrol-2,5-dion, Smp. 193-195°C.

### Beispiel 56

Eine Lösung von 128 mg des Produkts von Beispiel 51 in 30 ml Aethanol wird mit 10 ml einer gesättigten Lösung von Ammoniak in Aethanol behandelt und das Gemisch wird 3 Stunden bei 80°C erhitzt. Das Lösungsmittel wird unter vermindertem Druck entfernt und der Rückstand wird an Silicagel mit 10% Methanol in Dichlormethan chromatographiert. Man erhält 39 mg 3-[1-[3-(1-Amino-2-nitrovinylamino)propyl]-3-indolyl] -4-(1-methyl-3-indolyl)-1H-pyrrol-2,5-dion, Smp. 206-209°C (Zers.).

### Beispiel 57

Eine Suspension von 100 mg des Produkts von Beispiel 48 in 10 ml Aethanol wird mit 4 ml DMF und dann mit 10 ml einer gesättigten Lösung von Ammoniak in Aethanol behandelt. Das Gemisch wird 1 Stunde bei Raumtemperatur belassen und das Lösungsmittel wird dann abgedampft. Der Rückstand wird aus Aethanol kristallisiert. Man erhält 18 mg 3-(1-Methyl-3-indolyl)-4-[1-(3-thioureidopropyl)-3-indolyl]-1H-pyrrol-2,5-dion, Smp. 166-168°C (Zers.).

### Beispiel 58

190 mg Methansulfonsäureanhydrid werden einer Lösung von 399 mg des Produkts des Beispiel 21 und 1 ml Pyridin in 40 ml Dichlormethan zugesetzt. Nach 2 Stunden werden zusätzliche 40 ml Methansulfonsäureanhydrid und 1 ml Pyridin zugesetzt und es wird 16 Stunden weiter gerührt.

Das Gemisch wird mit Wasser gewaschen, getrocknet und eingedampft. Kristallisation aus Aethylacetat/Hexan ergibt 350 mg 3-(1-Methyl-3-indolyl)-4-[1-[3-(methylsulfonyloxy)propyl] -3-indolyl]-1H-pyrrol-2,5-dion, Smp. 202-204°C.

### Beispiel 59

7 mg einer 80%igen Dispersion von Natriumhydrid in Mineralöl werden in eine abgekühlte Lösung von 23 mg 2-Mercaptoimidazol in 10 ml DMF zugesetzt. Das Gemisch wird 0,5 Stunden unter Abkühlung gerührt. 100 mg des Produkts von Beispiel 58 werden zugesetzt und das Gemisch wird 2 Stunden unter Abkühlung gerührt. Das Gemisch wird dann auf Raumtemperatur erwärmen gelassen und über Nacht gerührt. Die Lösungsmittel werden unter vermindertem Druck entfernt und der Rückstand wird an Silicagel mit 10% Methanol in Dichlormethan chromatographiert. Man erhält 20 mg 3-[1-[3-(2-Imidazolylthio)propyl]-3-indolyl]-4-(1-methyl -3-indolyl)-1H-pyrrol-2,5-dion, Smp. 130-132°C.

### Beispiel 60

In zu Beispiel 59 analoger Weise erhält man aus 27 mg 2-Mercaptothiazolin und 100 mg des Produkts von Beispiel 58, 18 mg 3-(1-Methyl-3-indolyl)-4-[1-[3-(2-thiazolin-2-ylthio)propyl]-3-indolyl]-1H-pyrrol-2,5-dion, Smp. 170-173°C.

### Beispiel 61

In zu Beispiel 59 analoger Weise erhält man aus 25 mg 2-Mercaptopyrimidin und 100 mg des Produkts von Beispiel 58, 45 mg 3-(1-Methyl-3-indolyl)-4-[1-[3-(2-pyrimidinylthio)propyl] -3-indolyl]-1H-pyrrol-2,5-dion, Smp. 125-127°C.

### Beispiel 62

Einer Lösung von Natriummethoxid, hergestellt aus 51 mg Natrium und 20 ml Methanol, werden 315 mg 2-Mercaptopyridin-N-oxid und 200 mg des Produkts von Beispiel 58 zugesetzt. Das Gemisch wird 16 Stunden bei 55°C erhitzt. Die Lösungsmittel werden unter vermindertem Druck abgedampft und der Rückstand wird mit Aethylacetat vermischt. Der erhaltene Feststoff wird mit 2N Natriumhydroxid und dann mit Wasser gewaschen. Chromatographie an Silicagel mit 1% Methanol in Dichlormethan ergibt 49 mg 2-[3-[3-(1-Methyl-3-indolyl)-2,5-dioxo-1H-pyrrol-4-yl]-1-indolyl]propylthio]pyridin-1-oxid, Smp. 165-167°C.

### Beispiel 63

100 mg des Produkts von Beispiel 50 werden mit 30 ml Aethanol, 2 ml DMF und 40 ml gesättigter Ammoniaklösung in Aethanol bei 100°C 16 Stunden erhitzt. Die Lösungsmittel werden unter vermindertem Druck entfernt und der Rückstand wird an Silicagel mit 1% Methanol in Dichlormethan chromatographiert. Man erhält 10 mg 3-[1-[3-(2-Cyanoguanidino)propyl]-3-indolyl]-4-(1-methyl -3-indolyl)-1H-pyrrol-2,5-dion, Smp. 168-170°C.

### Beispiel 64

Eine Lösung von 100 mg 3-[1-(3-Aminopropyl)-3-indolyl]-4-(1-methyl-3-indolyl) -1H-pyrrol-2,5-dionacetat, enthaltend einen zusätlichen Aequivalent Essigsäure, in 10 ml Dimethylsulfoxid (DMSO) wird mit 35 mg Natriumbicarbonat und 36 mg 2-Chlor-3-nitropyridin behandelt. Das Gemisch wird 1 Stunde bei 60°C und dann 2 Stunden bei 100°C erhitzt. Die Lösung wird abkühlen gelassen, Wasser wird zugesetzt und der Niederschlag wird abfiltriert und auf Silicagel mit 1-5% Methanol in Dichlormethan chromatographiert. Das Produkt wird durch Kristallisation aus Hexan/Aethylacetat gereinigt. Man erhält 60 mg 3-(1-Methyl-3-indolyl)-4-[1-[3-(3-nitro -2-pyridylamino)propyl]-3-indolyl]-1H-pyrrol-2,5-dion, Smp. 218-220°C.

### Beispiel 65

In zu Beispiel 64 analoger Weise erhält man aus 100 mg des Produkts von Beispiel 26a) und 36 mg 2-Chlor-5-nitropyridin, 45 mg 3-(1-Methyl-3-indolyl)-4-[1-[3-(5-nitro-2-pyridylamino)propyl]-3-indolyl]-1H-pyrrol-2,5-dion, Smp. 245-247°C.

### Beispiel 66

Ein Gemisch von 100 mg 3-[1-(3-Aminopropyl)-3-indolyl]-4-(1-methyl-3-indolyl) -1H-pyrrol-2,5-dionacetat, enthaltend einen zusätzlichen Aequivalent Essigsäure, 75 mg 2-Chlorpyrimidin und 100 mg Natriumcarbonat in 100 ml DMSO wird 2 Stunden bei 80°C erhitzt. 50 ml Wasser werden der abgekühlten Lösung zugesetzt und der Niederschlag wird abfiltriert und an Silicagel mit 5% Methanol in Dichlormethan chromatographiert. Man erhält 60 mg 3-(1-Methyl-3-indolyl)-4-[1-[3-(2-pyrimidinylamino)propyl]-3-indolyl]-1H-pyrrol-2,5-dion, Smp. 214-215°C.

### Beispiel 67

Eine Lösung von 560 mg des Produkts von Beispiel 26a), enthaltend zwei Aequivalente Essigsäure, in 20 ml DMF wird mit 180 mg Natriumbicarbonat und 300 mg Methyl-4-benzyloxybenzimidathydrochlorid bei Raumtemperatur 24 Stunden behandelt. Das Lösungsmittel wird abgedampft und der Rückstand wird an Silicagel mit 1%-10% Methanol in Dichlormethan chromatographiert. Man erhält 275 mg 3-[1-[3-(4-Benzyloxy-α-iminobenzylamino)propyl] -3-indolyl]-4-(1-methyl-3-indolyl)-1H-pyrrol -2,5-dionhydrochlorid, Smp. 254-256°C.

Das Methyl-4-benzyloxybenzimidathydrochlorid wird wie folgt hergestellt:
Eine Lösung von 560 mg 4-Benzyloxybenzonitril in 16 ml THF und 0,2 ml Methanol wird auf 0°C abgekühlt, mit Salzsäure gesättigt und 16 Stunden bei 4°C gehalten. Der Niederschlag wird abfiltriert, mit Diäthyläther gewaschen und getrocknet. Man erhält 357 mg Methyl-4-benzyloxybenzimidathydrochlorid, Smp. 179-180°C.

### Beispiel 68

93 mg Methansulfonsäureanhydrid werden einer Lösung von 0,46 mMol des Produkts von Beispiel 55 in 25 ml Dichlormethan zugesetzt. 0,5 ml Pyridin werden zugesetzt und das Gemisch wird 0,5 Stunden gerührt, dann mit Wasser gewaschen, getrocknet und eingeengt. Chromatographie des Rückstandes an Silicagel mit 10% Methanol in Dichlormethan ergibt 160 mg 3-[1-[3-(Methylsulfonyloxy)propyl]-3-indolyl] -4-(2-nitrophenyl)-1H-pyrrol-2,5-dion, Smp. 177-178°C.

### Beispiel 69

50 mg Thioharnstoff werden einer auf Rückflusstemperatur erwärmten Lösung von 150 mg des Produkts von Beispiel 68 in 15 ml Aethanol zugesetzt. Das Gemisch wird 1 Stunde erhitzt und das Lösungsmittel wird dann abgedampft. Der Rückstand wird an Silicagel mit 20% Methanol in Dichlormethan chromatographiert. Man erhält 10 mg 3-[1-[3-(Amidinothio)propyl]-3-indolyl]-4-(2-nitrophenyl)-1H-pyrrol-2,5-dionmethansulfonat, Smp. 164-165°C.

### Beispiel 70

150 mg 3-(1-Methyl-3-indolyl)-4-(1-phenyl-3-indolyl)furan-2,5-dion werden mit 3 ml DMF und 10 ml einer 33%igen wässrigen Ammoniaklösung 4 Stunden bei 80°C behandelt. Das Gemisch wird abgekühlt und mit Aethylacetat extrahiert. Die Aethylacetatextrakte werden getrocknet und eingeengt. Der Rückstand wird aus Aethylacetat/Hexan kristallisiert. Man erhält 120 mg 3-(1-Methyl-3-indolyl)-4-(1-phenyl-3-indolyl) -1H-pyrrol-2,5-dion, Smp. 135.137°C.

Das Ausgangsfurandion wird wie folgt hergestellt:
0,7 g Oxalylchlorid werden einer Lösung von 1,0 g 1-Phenylindol in 50 ml Dichlormethan bei 0°C zugesetzt. Nach Erwärmen auf Raumtemperatur und 16-stündigem Rühren wird das Lösungsmittel unter vermindertem Druck entfernt. Das erhaltene Produkt wid mit 50 ml Dichlormethan, 1,4 g Triäthylamin und 1,0 g 1-Methylindol-3-ylessigsäure behandelt und das Gemisch wird 4 Stunden gerührt. Das Lösungsmittel wird abgedampft und der Rückstand wird an Silicagel mit Aethylacetat/Hexan (2:1) chromatographiert. Man erhält 190 mg des Furandions, Smp. 94-96°C.

### Beispiel 71

Eine Lösung von 50 mg des Produkts von Beispiel 50 in 10 ml DMF wird mit 4 ml einer 33%igen Lösung von Methylamin in Aethanol behandelt. Das Gemisch wird 16 Stunden gerührt und die Lösungsmittel werden unter vermindertem Druck entfernt. Der Rückstand wird an Silicagel mit Aethylacetat chromatographiert. Man erhält 46 mg 3-[1-[3-(2-Cyano-3-methylguanidino)propyl]-3-indolyl] -4-(1-methyl-3-indolyl)-1H-pyrrol-2,5-dion, Smp. 190-193°C.

### Beispiel 72

100 mg des Produkts von Beispiel 50 werden mit 10 ml Aethanol, 10 ml DMF und 20 ml einer 40%igen wässrigen Diäthylaminlösung 16 Stunden behandelt. Die Lösungsmittel werden unter vermindertem Druck entfernt und der Rückstand wird an Silicagel mit 10% Methanol in Dichlormethan chromatographiert. Man erhält 53 mg 3-[1-[3-(2-Cyano-3,3-dimethylguanidino)propyl]-3-indolyl] -4-(1-methyl-3-indolyl)-1H-pyrrol-2,5-dion, Smp. 150-153°C.

### Beispiel 73

Eine Lösung von 107 mg des Produkts von Beispiel 67 in 30 ml Aethanol wird mit 10 mg 10%igem Pd/C behandelt und 16 Stunden unter Wasserstoff geschüttelt. Das Lösungsmittel wird unter vermindertem Druck entfernt und der Rückstand wird an Silicagel mit 1%-10% Methanol in Dichlormethan chromatographiert. Das erhaltene Produkt wird durch Auflösung in Methanol, Filtration, Konzentration des Filtrats, Mischen des Rückstandes mit Aethylacetat, Filtrierung und Trocknen des Filtrierungsrückstandes gereinigt. Man erhält 22 mg 3-[1-[3-(4-Hydroxy-α-iminobenzylamino)propyl] -3-indolyl]-1H-pyrrol-2,5-dion, Smp. >300°C.

### Beispiel 74

150 mg des Produkts von Beispiel 58 und 50 mg 2-Imidazolidinthion werden 24 Stunden in 5 ml Aethanol unter Rückfluss erhitzt. Das Lösungsmittel wird abgedampft und der Rückstand wird an Silicagel mit 10%-25% Methanol in Dichlormethan chromatographiert. Man erhält 50 mg 3-[1-[3-(2-Imidazolin-2 ylthio)propyl]-3-indolyl]-4-(1-methyl-3-indolyl)-1H-pyrrol-2,5-dionmethansulfonat, Smp. 134-136°C.

### Beispiel 75

0,5 g 3-[1-(3-Aminopropyl)-3-indolyl]-4-(1-methyl-3-indolyl) -1H-pyrrol-2,5-dionacetat, 110 mg Natriumbicarbonat und 242 mg 3,5-Dimethyl-N²-nitro-1-pyrazol-1-carboxamidin werden 4 Stunden in 25 ml Aethanol unter Rückfluss erhitzt. Das Lösungsmittel wird unter vermindertem Druck entfernt und der Rückstand wird an Silicagel mit 1%-5% Methanol in Dichlormethan chromatographiert. Man erhält 500 mg 3-(1-Methyl-3-indolyl)-4-[1-[3-(2-nitroguanidino)propyl] -3-indolyl]-1H-pyrrol-2,5-dion, Smp. 268-270°C (Zers.).

### Beispiel 76

0,5 ml Pyridin und 115 mg Methansulfonylchlorid werden einer Lösung von 50 mg des Produkts von Beispiel 26a) in 35 ml Dichlormethan zugesetzt. Die entstandene Lösung wird über Nacht gerührt. 2 ml Pyridin werden zugesetzt und das Gemisch wird 8 Stunden unter Rückfluss erhitzt. Das abgekühlte Gemisch wird mit 2M Salzsäure, gesättigter Natriumbicarbonatlösung und Wasser gewaschen. Die Lösung wird getrocknet und eingedampft. Man erhält einen Feststoff, der aus Dichlormethan/Diäthyläther/Hexan umkristallisiert wird. Man erhält 40 mg 3-(1-Methyl-3-indolyl)-4-[1-[3-(methylsulfonamido)propyl] -3-indoly]-1H-pyrrol-2,5-dion, Smp. 135-138°C.

### Beispiel 77

3-[1-[3-(Benzolsulfonamido)propyl]-3-indolyl]-4-(1-methyl-3-indolyl)-1H-pyrrol-2,5-dion, Smp. 125-128°C wird in zu Beispiel 76 analoger Weise hergestellt.

### Beispiel 78

30 µl Benzoylchlorid werden einer Lösung von 50 mg des Produkts von Beispiel 26a) in 40 ml Dichlormethan zugesetzt. 200 µl Pyridin werden dann zugesetzt und das Gemisch wird 5 Stunden gerührt. Das Gemisch wird dann mit 2M Salzsäure und mit gesättigter Natriumbicarbonatlösung gewaschen, getrocknet und eingedampft. Kristallisieren des Rückstandes aus Aethylacetat/Petroleumäther ergibt 45 mg 3-[1-(3-Benzamidopropyl)-3-indolyl]-4-(1-methyl-3-indolyl)-1H-pyrrol-2,5-dion, Smp. 138-140°C.

### Beispiel 79

102 mg Essigsäureanhydrid werden einer Lösung von 50 mg des Produkts von Beispiel 26a) in 40 ml Dichlormethan zugesetzt. Die entstandene Lösung wird 1 Stunde gerührt. Das Gemisch wird mit 2M Salzsäure und gesättigter Natriumbicarbonatlösung gewaschen, getrocknet und eingedampft. Kristallisieren des Rückstandes aus Dichlormethan/Hexan ergibt 45 mg 3-[1-(3-Acetamidopropyl)-3-indolyl]-4-(1-methyl-3-indolyl) -1H-pyrrol-2,5-dion, Smp. 132-136°C.

### Beispiel 80

Ein Gemisch von 300 mg des Produkts von Beispiel 58 und 2 ml einer 33%igen Lösung von Dimethylamin in Aethanol wird 1 Stunde bei 90°C erhitzt. Das Gemisch wird eingedampft, der Rückstand wird in 50 ml Aethylacetat gelöst, die Lösung wird mit gesättigter Natriumbicarbonatlösung gewaschen und mit Salzsäure in Aethylacetat behandelt. Das Lösungsmittel wird abgedampft und der Rückstand wird an Silicagel mit Dichlormethan/Methanol/Essigsäure/Wasser (60:18:2:3) gereinigt. Kristallisieren aus Methanol/Aethylacetat ergibt 40 mg 3-[1-(3-Dimethylamino)propyl]-3-indolyl]-4-(1-methyl -3-indolyl)-1H-pyrrol-2,5-dionhydrochlorid, Smp. 268-270°C.

### Beispiel 81

Ein Gemisch von 173 mg des Produkts von Beispiel 58 und 2 ml einer 33%igen Lösung von Trimethylamin in Aethanol wird 3 Stunden bei 90°C erhitzt. Das Lösungsmittel wird abgedampft und der Rückstand wird an Silicagel mit Dichlormethan/Methanol/Essigsäure/Wasser (60:18:2:3) gereinigt. Mischen mit Aethylacetat ergibt 75 mg Trimethyl-[3-[3-[3-(1-methyl-3-indolyl)-2,5-dioxo -3-pyrrolin-4-yl]-1-indolyl]propyl]ammoniummethylsulfonat, Smp. 180-185°C.

### Beispiel 82

Eine Lösung von 500 mg 3-[1-[3-[(t-Butoxycarbonyl)(methyl)amino]propyl] -3-indolyl]-4-(1-methyl-3-indolyl)furan-2,5-dion in 1 ml DMF und 2 ml einer 33%igen wässrigen Ammoniaklösung wird 4 Stunden bei 140°C erhitzt. Das Lösungsmittel wird abgedampft und der Rückstand wird in 30 ml Aethylacetat aufgenommen. Unlösliches Material wird abfiltriert und eine gesättigte Lösung von Salzsäure in Aethylacetat wird dem Filtrat zugesetzt. Das Lösungsmittel wird abgedampft und der Rückstand wird an Silicagel mit Dichlormethan/Methanol/Essigsäure/Wasser (60:18:2:3) chromatographiert. Kristallisieren aus Methanol/Aethylacetat ergibt 75 mg 3-(1-Methyl-3-indolyl)-4-[1-[3-(methylamino)propyl] -3-indolyl]-1H-pyrrol-2,5-dionhydrochlorid, Smp. 273-275°C.

Das Ausgangsfurandion wird wie folgt hergestellt:
a) 3 g 1-(3-Brompropyl)indol werden mit einer 33%igen Lösung von Methylamin in Aethanol behandelt. Die entstandene Lösung wird 6 Stunden gerührt. Das Lösungsmittel wird abgedampft und der Rückstand wird in 50 ml Dichlormethan gelöst und mit gesättigter Natriumbicarbonatlösung gewaschen. Die organische Phase wird getrocknet und eingedampft. Man erhält 3,20 g 1-(3-Methylaminopropyl)indol.
b) 2,67 g Di(t-butyl)dicarbonat und 1,24 g Triäthylamin werden einer Lösung von 2,3 g 1-(3-Methylaminopropyl)indol in 40 ml Dichlormethan bei 0°C zugesetzt. Nach 4 Stunden wird das Gemisch mit gesättigter Natriumbicarbonatlösung gewaschen, getrocknet und eingedampft. Man erhält 3,68 g 1-[3-[(t-Butoxycarbonyl)(methyl)amino]propyl]indol.
c) 1,14 ml Oxalylchlorid werden einer Lösung von 3,6 g des Produkts von b) in 40 ml Diäthyläther bei 0°C zugesetzt. Die entstandene Lösung wird 1 Stunde bei 0°C gerührt und das Lösungsmittel wird dann abgedampft. Der Rückstand wird in 120 ml Dichlormethan gelöst und mit 3,44 ml Triäthylamin und 2,36 g 1-Methylindol-3-ylessigsäure behandelt. Nach 18 Stunden Rühren bei Raumtemperatur wird das Lösungsmittel abgedampft und der Rückstand wird an Silicagel mit Aethylacetat/Petroleumäther (1:2) gereinigt. Abdampfen der Lösungsmittel ergibt 1,4 g des erwünschten Furandions, Smp. 73-80°C.

### Beispiel 83

75 mg Methansulfonylchlorid werden einer Lösung von 170 mg des Produkts von Beispiel 21 in 8 ml Pyridin zugesetzt. Die entstandene Lösung wird 4 Stunden gerührt und das Lösungsmittel wird dann abgedampft. Der Rückstand wird an Silicagel mit Aethylacetat/Petroleumäther (1:1) gereinigt. Umkristallisieren aus Aethylacetat/Hexan ergibt 40 mg 3-[1-(3-Chlorpropyl)-3-indolyl]-4-(1-methyl-3-indolyl) -1H-pyrrol-2,5-dion, Smp. 254-256°C.

### Beispiel 84

In zu Beispiel 26 analoger Weise werden hergestellt:
3-[1-(2-Aminoäthyl)-3-indolyl]-4-(1-methyl-3-indolyl)-1H-pyrrol-2,5-dionhydrochlorid, Smp. 245-247°C;
3-[1-(4-Aminobutyl)-3-indolyl]-4-(1-methyl-3-indolyl)-1H-pyrrol-2,5-dionhydrochlorid, Smp. 190-192°C; und
3-[1-(5-Aminopentyl)-3-indolyl]-4-(1-methyl-3-indolyl)-1H-pyrrol-2,5-dion, Smp. 180-182°C.

### Beispiel 85

Eine Lösung von 1,6 g 3-[1-(2-Acetoxyäthyl)-3-indolyl]-4-(1-methyl -3-indolyl)furan-2,5-dion in 4 ml DMF und 8 ml einer 33%igen wässrigen Ammoniaklösung wird 4 Stunden bei 160°C erhitzt. Der Niederschlag wird abfiltriert und getrocknet. Man erhält 1,04 g 3-[1-(2-Hydroxyäthyl)-3-indolyl]-4-(1-methyl-3-indolyl) -1H-pyrrol-2,5-dion, Smp. 250-252°C.

Das Ausgangsfurandion wird wie folgt hergestellt:
a) 11,7 g Indol in 500 ml DMF werden mit einer Dispersion von 4 g Natriumhydrid in Mineralöl behandelt. Nach 1 Stunde wird das Gemisch in einem Eisbad abgekühlt und 10 ml Aethylenoxid werden zugesetzt. Das Gemisch wird auf Raumtemperatur erwärmen gelassen und dann 2 Stunden gerührt. Das Lösungsmittel wird abgedampft und der Rückstand wird mit 50 ml Wasser behandelt und mit 2M Salzsäure neutralisiert. Das Produkt wird in Dichlormethan extrahiert, das Lösungsmittel wird eingedampft und der Rückstand wird mit Aethylacetat/Petroleumäther chromatographiert. Man erhält 7,6 g 1-(2-Hydroxyäthyl)indol.
b) Eine eiskalte Lösung von 4,6 g 1-(2-Hydroxyäthyl)indol in 10 ml Diäthyläther wird mit 1 ml Pyridin und 4 ml Essigsäureanhydrid behandelt. Nach 2 Stunden werden 50 ml Wasser zugesetzt, das Gemisch wird mit 100 ml Dichlormethan extrahiert und das Dichlormethanextrakt wird getrocknet. Das Lösungsmittel wird abgedampft. Man erhält 5,7 g 1-(2-Acetoxyäthyl)indol.
c) 2,57 ml Oxalylchlorid werden einer Lösung von 5,7 g 1-(2-Acetoxyäthyl)indol in 70 ml Dichlormethan bei 0°C zugesetzt. Die entstandene Lösung wird 2 Stunden gerührt und das Lösungsmittel wird dann abgedampft. Dem Rückstand, aufgelöst in 210 ml Dichlormethan, werden 7,7 ml Triäthylamin und 5,29 g 1-Methylindol-3-essigsäure unter Stickstoff zugesetzt. Nach 18 Stunden Rühren wird das Lösungsmittel abgedampft und der Rückstand wird an Silicagel mit Aethylacetat/Petroleumäther (1:2) gereinigt. Kristallisieren aus Aethylacetat/Hexan ergibt 1,87 g 3-[1-(2-Acetoxyäthyl)-3-indolyl]-4-(1-methyl -3-indolyl)furan-2,5-dion, Smp. 198-199°C.

### Beispiel 86

200 mg 3-(1-Methyl-3-indolyl)-4-(4-pyridyl)furan-2,5-dion werden mit 5 ml DMF und 5 ml einer 33%igen wässrigen Ammoniaklösung behandelt. Die entstandene Lösung wird 17 Stunden bei 140°C erhitzt. Nach Abkühlen wird die Suspension mit Wasser verdünnt. Das Produkt wird abfiltriert, mit Wasser gewaschen und getrocknet. Man erhält 144 mg 3-(1-Methyl-3-indolyl)-4-(4-pyridyl)-1H-pyrrol-2,5-dion, Smp. 332-334°C.

Das Ausgangsfurandion wird wie folgt hergestellt:
5 g 4-Pyridylessigsäure-hydrochlorid und 3,72 g Diisopropyläthylamin in 50 ml Dichlormethan werden bei 0°C zuerst mit 6,37 g 1-Methylindol-3-glyoxylchlorid in 50 ml Dichlormethan und dann mit 7,5 g Diisopropyläthylamin behandelt. Das Gemisch wird auf Raumtemperatur erwärmen gelassen und dann 65 Stunden gerührt. Das Lösungsmittel wird abgedampft und der Rückstand wird in Dichlormethan aufgenommen und über Silicagel mit Aethylacetat chromatographiert. Die das Produkt enthaltenden Fraktionen werden eingeengt. Kristallisieren aus Aethylacetat/Hexan ergibt 940 mg des Furandions, Smp. 217-219°C.

### Beispiel 87

In zu Beispiel 86 analoger Weise werden hergestellt:
3-(1-Methyl-3-indolyl)-4-(3-pyridyl)-1H-pyrrol-2,5-dion, Smp. 278-279°C; und
3-(1-Methyl-3-indolyl)-4-(2-pyridyl)-1H-pyrrol-2,5-dion, Smp. 242-244°C.

### Beispiel 88

135 mg 3-(1-Methyl-3-indolyl)-4-(3-pyrrolyl)furan-2,5-dion werden mit 5 ml DMF und 5 ml einer 33%igen wässrigen Ammoniaklösung behandelt. Die Lösung wird 4 Stunden bei 140°C erhitzt. Die abgekühlte Lösung wird mit Wasser verdünnt und mit Dichlormethan extrahiert. Die Dichlormethanextrakte werden getrocknet und eingeengt. Chromatographie des Rückstandes an Silicagel mit Aethylacetat/Hexan (1:1) gefolgt durch Kristallisieren aus Aethylacetat/Hexan ergeben 50 mg 3-(1-Methyl-3-indolyl)-4-(3-pyrrolyl)-1H-pyrrol-2,5-dion, Smp. 240-241°C.

Das Ausgangsfurandion wird wie folgt hergestellt:
a) 1 g 1-(Benzolsulfonyl)-3-pyrrolylessigsäure in 25 ml Dichlormethan werden bei 0°C mit einer Lösung von 837 mg 1-Methylindol-3-glyoxylchlorid in 25 ml Dichlormethan und dann mit 975 mg Diisopropyläthylamin behandelt. Das Gemisch wird auf Raumtemperatur erwärmen gelassen und dann 22 Stunden gerührt. Nach Einengen und Chromatographie des Rückstandes an Silicagel mit Aethylacetat/Hexan (1:1) erhält man 540 mg 3-[1-(Benzolsulfonyl)-3-pyrrolyl]-4-(1-methyl -3-indolyl)furan-2,5-dion.
b) 255 mg des Produkts von a) in 10 ml Aethanol werden mit 2,5 ml einer 2,5M Natriumhydroxidlösung behandelt. Die Lösung wird 17 Stunden bei Raumtemperatur belassen. Nach Verdünnen mit 10 ml Wasser wird das Gemisch mit Diäthyläther extrahiert. Die wässrige Lösung wird mit konzentrierter Salzsäure angesäuert und mit Aethylacetat extrahiert. Die Aethylacetatextrakte werden getrocknet und eingeengt. Man erhält 140 mg des Furandions, m/e 292 (M⁺).

### Beispiel 89

1,455 g des Produkts von Beispiel 58 in 45 ml Aethanol werden mit 364 mg Thioharnstoff behandelt und das Gemisch wird 18 Stunden unter Rückfluss erhitzt. Nach Abkühlen wird der Niederschlag abfiltriert und mit Aethanol und Diäthyläther gewaschen. Der Feststoff wird getrocknet. Man erhält 1,33 g 3-[1-[3-(Amidinothio)propyl]-3-indolyl]-4-(1-methyl -3-indolyl)-1H-pyrrol-2,5-dionmethansulfonat, Smp. 236-238°C (Zers.).

### Beispiel 90

In zu Beispiel 89 analoger Weise werden hergestellt:
3-[1-[2-(Amidinothio)äthyl]-3-indolyl]-4-(1-methyl-3-indolyl)-1H-pyrrol-2,5-dionmethansulfonat, m.p. 238-240°C (Zers.);
3-[1-[4-(Amidinothio)butyl]-3-indolyl]-4-(1-methyl-3-indolyl)-1H-pyrrol-2,5-dionmethansulfonat, m.p. 150°C (Zers.); und
3-[1-[5-(Amidinothio)pentyl]-3-indolyl]-4-(1-methyl-3-indolyl)-1H-pyrrol-2,5-dionmethansulfonat, m.p. 130°C (Zers.).

### Beispiel 91

450 mg des Produkts von Beispiel 58 in 20 ml DMSO werden mit 116 mg Natriumcyanid behandelt. Das Gemisch wird 8 Stunden bei 50°C erhitzt, dann abgekühlt und in Wasser geschüttet. Das Gemisch wird mit Aethylacetat extrahiert und die Extrakte werden getrocknet. Konzentration und Chromatographie an Silicagel mit Toluol/Essigsäure (9:1) ergeben ein Feststoff, der mit Diäthyläther vermischt, abfiltriert und getrocknet wird. Man erhält 69 mg 3-[1-(3-Cyanopropyl)-3-indolyl]-4-(1-methyl-3-indolyl)-1H-pyrrol-2,5-dion, Smp. 245-247°C.

### Beispiel 92

3-[1-(4-Cyanobutyl)-3-indolyl]-4-(1-methyl-3-indolyl)-1H-pyrrol-2,5-dion, Smp. 195-198°C wird in zu Beispiel 91 analoger Weise hergestellt.

### Beispiel 93

100 mg des Produkts von Beispiel 91 in 20 ml Aethanol werden mit gasförmigem Chlorwasserstoff behandelt, bis eine gesättigte Lösung erhalten wird. Nach 6 Stunden wird das Lösungsmittel abgedampft und der Rückstand wird in 50 ml Aethanol gelöst. Die Lösung wird auf 0°C abgekühlt, mit Ammoniakgas gesättigt und dann auf Raumtemperatur erwärmen gelassen. Nach 17 Stunden wird das Lösungsmittel abgedampft. Der Rückstand wird in Wasser gelöst und mit Aethylacetat extrahiert. Die wässrige Lösung wird lyophilisiert. Man erhält 84 mg 3-[1-(3-Amidinophenyl)-3-indolyl]-4-(1-methyl-3-indolyl) -1H-pyrrol-2,5-dion-hydrochlorid, Smp. 175-177°C.

### Beispiel 94

100 mg des Produkts von Beispiel 58 in 10 ml DMSO werden mit 120 mg 5-Mercapto-1-methyltetrazol-natriumsalz behandelt. Die Lösung wird 6 Stunden bei 55°C erhitzt, dann abgekühlt und in Wasser geschüttet. Die wässrige Phase wird mit Aethylacetat extrahiert. Die Aethylacetatextrakte werden getrocknet und eingeengt. Chromatographie an Silicagel mit Dichlormethan/Aethylacetat (4:1) ergibt 48 mg 3-(1-Methyl-3-indolyl)-4-[1-[3-(1-methyl -5-tetrazolylthio)propyl]-3-indolyl]-1H-pyrrol-2,5-dion, Smp. 95-97°C.

### Beispiel 95

600 mg des Produkts von Beispiel 21 in 60 ml Dichlormethan werden mit 237 mg Pyridin behandelt. Das Gemisch wird bei 5-10°C einer Lösung von 855 mg Trifluormethansulfonsäureanhydrid in 15 ml Dichlormethan zugesetzt. Nach 1 Stunde bei 5-10°C wird das Gemisch an 944 mg 1,2-Diaminoäthan in 20 ml Dichlormethan zugesetzt. Das Gemisch wird 15 Minuten bei Raumtemperatur gerührt und dann mit einer Natriumbicarbonatlösung gewaschen. Die Dichlormethanlösung wird getrocknet und eingeengt. Chromatographie des Rückstandes an Silicagel mit Chloroform/Methanol/Essigsäure/Wasser (60:18:2:3) ergibt ein gummiartiges Produkt, das in 50 ml Aethanol gelöst wird. Die Lösung wird mit 25 ml 1M Salzsäure behandelt und eingeengt. Der Rückstand wird mit Diäthyläther vermischt, abfiltriert und getrocknet. Man erhält 221 mg 3-[1-[3-(2-Aminoäthylamino)propyl]-3-indolyl]-4-(1-methyl -3-indolyl)-1H-pyrrol-2,5-dion, Smp. 190-193°C.

### Beispiel 96

100 mg des Produkts von Beispiel 58 in 5 ml DMSO werden mit 30 mg Natriummethanthiolat behandelt. Die Lösung wird 30 Minuten gerührt und dann mit Wasser verdünnt. Der Niederschlag wird abfiltriert, mit Wasser gewaschen und getrocknet. Man erhält 52 mg 3-(1-Methyl-3-indolyl)-4-[1-[3-(methylthio)propyl] -3-indolyl]-1H-pyrrol-2,5-dion, Smp. 222-224°C.

### Beispiel 97

116 mg des Produkts von Beispiel 96 in 5 ml Dichlormethan werden bei 0°C mit 60 mg 85%iger Metachlorperbenzoesäure in 5 ml Dichlormethan behandelt. Die Lösung wird auf Raumtemperatur erwärmen gelassen und dann 1 Stunde gerührt. Die Lösung wird mit wässrigem Natriumbicarbonat gewaschen und getrocknet. Die Lösung wird eingeengt und der Rückstand wird aus Aethylacetat/Hexan kristallisiert. Man erhält 84 mg rac-3-(1-Methyl-3-indolyl)-4-[1-(3-methylsulfinylpropyl) -3-indolyl]-1H-pyrrol-2,5-dion, Smp. 140°C.

### Beispiel 98

90 mg des Produkts von Beispiel 97 in 5 ml Dichlormethan werden mit 60 mg einer 85%igen Metachlorperbenzoesäure in 5 ml Dichlormethan behandelt. Die Lösung wird 2 Stunden gerührt und dann mit wässrigem Natriumbicarbonat gewaschen. Die Lösung wird getrocknet und eingeengt. Chromatographie des Rückstandes an Silicagel mit Dichlormethan/Aethylacetat (1:1) und Umkristallisieren aus Aethylacetat/Hexan ergeben 25 mg 3-(1-Methyl-3-indolyl)-4-[1-(3-methylsulfonylpropyl) -3-indolyl]-1H-pyrrol-2,5-dion, Smp. 225-227°C.

### Beispiel 99

838 mg des Produkts von Beispiel 58 in 15 ml DMSO werden mit 600 mg Kaliumthiolacetat behandelt. Die Lösung wird 3 Stunden gerührt und dann mit Wasser verdünnt. Die Lösung wird mit Aethylacetat extrahiert und die Aethylacetatextrakte werden getrocknet. Nach Einengen und Chromatographie an Silicagel mit Aethylacetat erhält man 723 mg 3-[1-[3-(Acetylthio)propyl]-3-indolyl]-4-(1-methyl-3-indolyl)-1H-pyrrol-2,5-dion, Smp. 210-213°C.

### Beispiel 100

350 mg des Produkts von Beispiel 99 in 20 ml 50% Methanol/DMF werden mit 0,5 ml einer 33%igen wässrigen Ammoniaklösung behandelt. Das Gemisch wird 17 Stunden gerührt, dann mit 20 ml Natriumchloridlösung verdünnt und mit Aethylacetat extrahiert. Die Aethylacetatextrakte werden getrocknet und abgedampft. Chromatographie des Rückstandes an Silicagel mit Aethylacetat/Hexan (3:1) ergibt 266 mg 3-[1-(3-Mercaptopropyl)-3-indolyl]-4-(1-methyl -3-indolyl)-1H-pyrrol-2,5-dion, Smp. 155-157°C.

### Beispiel 101

0,71 g Trifluormethansulfonsäureanhydrid werden einer Lösung von 0,5 g des Produkts von Beispiel 21 in 10 ml Pyridin bei 0°C zugesetzt. Das Gemisch wird 3 Tage bei Raumtemperatur gerührt und dann abgedampft. Der Rückstand wird an Silicagel mit 10% Methanol in Dichlormethan chromatographiert. Man erhält 0,11 g 3-(1-Methyl-3-indolyl)-4-[1-[3-(1-pyridinio)propyl] -3-indolyl]-1H-pyrrol-2,5-diontrifluormethansulfonat, Smp. 87-88°C.

### Beispiel 102

Chromatographie der freien Base von Beispiel 26a) an Silicagel mit Dichlormethan/Methanol/Essigsäure/Wasser (60:18:2:3) ergibt das entsprechende Acetat, das in den Beispielen 64, 66 und 75 als Ausgangsmaterial verwendet wird.

Tabletten und Kapseln folgender Zusammensetzung werden in an sich bekannter Weise hergestellt:

| Beispiel A | |
|---|---|
| Bestandteile | Pro Tablette |
| Verbindung der Formel I | 5,0 mg |
| Lactose | 125,0 mg |
| Maisstärke | 75,0 mg |
| Talk | 4,0 mg |
| Magnesiumstearat | 1,0 mg |
| Tablettengewicht | 210,0 mg |

| Beispiel B | |
|---|---|
| Bestandteile | Pro Kapsel |
| Verbindung der Formel I | 10,0 mg |
| Lactose | 165,0 mg |
| Maisstärke | 20,0 mg |
| Talk | 5,0 mg |
| Kapselfüllgewicht | 200,0 mg |

## Patentansprüche (Patentansprüche für folgende(n) Vertragsstaat(en): AT, BE, CH, DE, FR, GB, IT, LI, LU, NL, SE)

1. Pyrrole der allgemeinen Formel 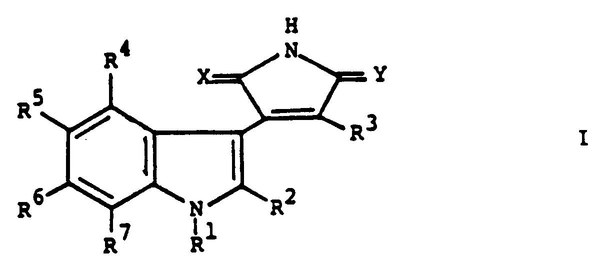 worin
R¹ Wasserstoff, Alkyl, Aryl, Aralkyl, Alkoxyalkyl, Hydroxyalkyl, Haloalkyl, Aminoalkyl, Monoalkylaminoalkyl, Dialkylaminoalkyl, Trialkylaminoalkyl, Aminoalkylaminoalkyl, Azidoalkyl, Acylaminoalkyl, Acylthioalkyl, Alkylsulfonylaminoalkyl, Arylsulphonylaminoalkyl, Mercaptoalkyl, Alkylthioalkyl, Alkylsulfinylalkyl, Alkylsulfonylalkyl, Alkylsulfonyloxyalkyl, Alkylcarbonyloxyalkyl, Cyanoalkyl, Amidinoalkyl, Isothiocyanatoalkyl, Glucopyranosyl, Carboxyalkyl, Alkoxycarbonylalkyl, Aminocarbonylalkyl, Hydroxyalkylthioalkyl, Mercaptoalkylthioalkyl, Arylthioalkyl oder Carboxyalkylthioalkyl oder eine Gruppe der Formel 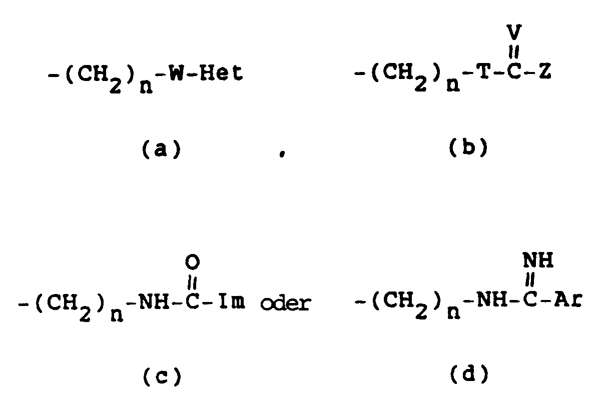 in der
Het eine heterocyclische Gruppe,
W NH, S oder eine Bindung,
T NH oder S,
V O, S, NH, NNO₂, NCN oder CHNO₂,
Z Alkylthio, Amino, Monoalkylamino oder Dialkylamino,
Im 1-Imidazolyl,
Ar Aryl, und
n eine Zahl von 2-6;
R² Wasserstoff, Alkyl, Aralkyl, Alkoxyalkyl, Hydroxyalkyl, Haloalkyl, Aminoalkyl, Monoalkylaminoalkyl, Dialkylaminoalkyl, Acylaminoalkyl, Alkylsulfonylaminoalkyl, Arylsulfonylaminoalkyl, Mercaptoalkyl, Alkylthioalkyl, Carboxyalkyl, Alkoxycarbonylalkyl, Aminocarbonylalkyl, Alkylthio oder Alkylsulfinyl;
R³ eine carbocyclische oder heterocyclische aromatische Gruppe;
R⁴, R⁵, R⁶ und R⁷ unabhängig voneinander Wasserstoff, Halogen, Alkyl, Hydroxy, Alkoxy, Aryloxy, Haloalkyl, Nitro, Amino, Acylamino, Monoalkylamino, Dialkylamino, Alkylthio, Alkylsulfinyl oder Alkylsulfonyl;
und eines von X und Y O und das andere O, S, (H,OH) oder (H,H) ist
mit der Bedingung, dass R¹ von Wasserstoff verschieden ist, falls R² Wasserstoff, R³ 3-Indolyl oder 6-Hydroxy-3-indolyl, R⁴, R⁵ und R⁷ Wasserstoff, R⁶ Wasserstoff oder Hydroxy und X und Y beide O sind, sowie falls R² Wasserstoff, R³ 3-Indolyl, R⁴, R⁵, R⁶ und R⁷ Wasserstoff, X (H,H) und Y O sind;
wobei eine heterocyclische Gruppe Het für eine gesättigte, teilweise ungesättigte oder eine aromatische 5- oder 6-gliedrige heterocyclische Gruppe steht, die gewünschtenfalls einen ankondensierten Benzolring enthält und unsubstituiert oder substituiert ist durch einen oder mehrere Substituenten aus der Gruppe von Halogen, Alkyl, Hydroxy, Alkoxy, Haloalkyl, Nitro, Amino, Acylamino, Mono- oder Dialkylamino, Alkylthio, Alkylsulfinyl und Alkylsufonyl,
eine heterocyclische aromatische Gruppe R³ wie weiter oben eine heterocyclische aromatische Gruppe Het definiert ist, und
eine carbocyclische aromatische Gruppe R³ für Phenyl oder Naphthyl steht, das gewünschtenfalls wie die obige Gruppe Het substituiert ist, und
Acyl allein oder in Kombination eine von einer Alkancarbonsäure mit bis zu 7 C-Atome oder von einer aromatischen Carbonsäure abgeleitete Gruppe ist, und
Alkyl allein oder in Kombination für eine geradkettige oder verzweigte Alkylgruppe mit bis zu 7 C-Atome steht,
sowie pharmazeutisch verwendbare Salze von sauren Verbindungen der Formel I mit Basen und von basischen Verbindungen der Formel I mit Säuren.

2. Verbindungen gemäss Anspruch 1, worin R¹ Wasserstoff, Alkyl, Aralkyl, Alkoxyalkyl, Hydroxyalkyl, Haloalkyl, Aminoalkyl, Monoalkylaminoalkyl, Dialkylaminoalkyl, Azidoalkyl, Acylaminoalkyl, Alkylsulfonylaminoalkyl, Arylsulfonylaminoalkyl, Mercaptoalkyl, Alkylthioalkyl, Glucopyranosyl, Carboxyalkyl, Alkoxycarbonylalkyl, Aminocarbonylalkyl, Hydroxyalkylthioalkyl, Mercaptoalkylthioalkyl, Arylthioalkyl oder Carboxyalkylthioalkyl.

3. Verbindungen gemäss Anspruch 2, worin R¹ Alkyl oder Aminoalkyl ist.

4. Verbindungen gemäss Anspruch 1, worin R¹ Isothiocyanatoalkyl oder eine Gruppe der Formel (b), in der T S, V NH und Z Amino sind oder in der T NH, V NH oder NNO₂ und Z Amino bedeuten.

5. Verbindungen nach einem der Ansprüche 1-4, worin R² Wasserstoff ist.

6. Verbindungen gemäss einem der Ansprüche 1-5, worin R³ durch Halogen, Alkyl, Alkoxy, Haloalkyl, Nitro, Amino, Alkylthio, Alkylsulfinyl oder Alkylsulfonyl monosubstituiertes Phenyl ist.

7. Verbindungen nach einem der Ansprüche 1-5, worin R³ eine Gruppe der Formel 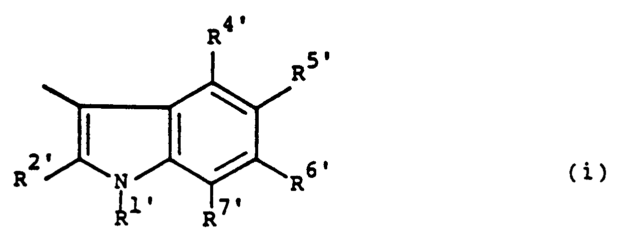 worin R^{1′}, R^{2′}, R^{4′}, R^{5′}, R^{6′} und R^{7′} eine der Bedeutungen von R¹, R², R⁴, R⁵, R⁶ bzw. R⁷ im Anspruch 1 haben, ist.

8. Verbindungen gemäss einem der Ansprüche 1-7, worin R⁴, R⁵, R⁶ und R⁷ Wasserstoff sind.

9. Verbindungen nach einem der Ansprüche 1-8, worin R¹ Methyl, 3-Aminopropyl, 3-Isothiocyanatopropyl oder eine Gruppe der Formel (b), in der T S, V NH, Z Amino und n die Zahl 3 ist oder in der T NH, V NH oder NNO₂, Z Amino und n die Zahl 3, R² Wasserstoff, R³ durch Chlor, Brom, Methyl, Methoxy, Trifluormethyl, Nitro, Amino, Methylsulfinyl oder Methylsulfonyl monosubstituiertes Phenyl oder eine Gruppe der Formel (i) nach Anspruch 7, in der R^{1′} Methyl und R^{2′}, R^{4′}, R^{5′}, R^{6′} und R^{7′} Wasserstoff, und R⁴, R⁵, R⁶ und R⁷ Wasserstoff sind.

10. Ein Pyrrol aus der Gruppe der folgenden:
3-(2-Chlorphenyl)-4-(1-methyl-3-indolyl)-1H-pyrrol-2,5-dion,
3-(1-Methyl-3-indolyl)-4-(2-nitrophenyl)-1H-pyrrol-2,5-dion,
3,4-Bis(1-methyl-3-indolyl)-1H-pyrrol-2,5-dion,
3-[1-(3-Aminopropyl)-3-indolyl-4-(1-methyl-3-indolyl)-1H-pyrrol-2,5-dion,
3-[1-[3-(Amidinothio)propyl]-3-indolyl]-4-(1-methyl-3-indolyl)-1H-pyrrol-2,5-dion,
3-(1-Methyl-3-indolyl)-4-[1-[3-(2-nitroguanidino)propyl]-3-indolyl]-1H-pyrrol-2,5-dion und
3-[1-(3-Isothiocyanatopropyl)-3-indolyl]-4-(1-methyl-3-indolyl)-1H-pyrrol-2,5-dion.

11. Verbindungen gemäss einem der Ansprüche 1-10 zur Verwendung als therapeutisch, insbesondere als antiinflammatorisch, immunologisch, bronchopulmonär und cardiovaskulär aktive Substanzen.

12. Ein Medikament, insbesondere ein antiinflammatorisches, immunologisches, bronchopulmonäres oder cardiovaskuläres Medikament, enthaltend eine Verbindung gemäss einem der Ansprüche 1-10 und ein therapeutisch inertes Trägermaterial.

13. Die Verwendung einer Verbindung gemäss einem der Ansprüche 1-10 bei der Herstellung eines Medikamentes gegen inflammatorische, immunologische, bronchopulmonäre oder cardiovaskuläre Krankheiten.

14. Verfahren zur Herstellung der Pyrrole nach einem der Ansprüche 1-10, dadurch gekennzeichnet, dass man
a) zur Herstellung einer Verbindung der Formel I, in der X und Y O sind, eine Verbindung der Formel 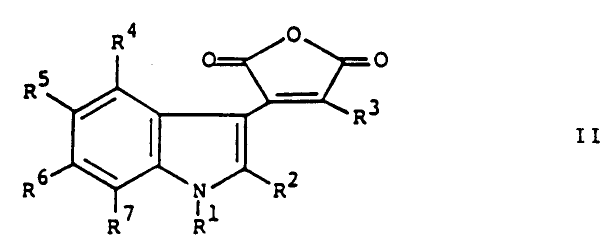 worin R¹ bis R⁷ die im Anspruch 1 angegebene Bedeutung haben,
mit Ammoniak unter Druck oder mit Hexamethyldisilazan und Methanol umsetzt, oder
(b) zur Herstellung einer Verbindung der Formel I, in der R¹ Wasserstoff und X und Y O sind, eine Verbindung der Formel 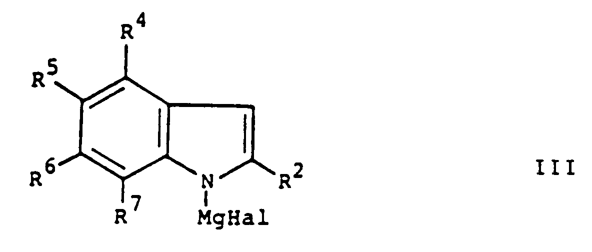 worin R² bis R⁷ die im Anspruch 1 angegebene Bedeutung haben und Hal Halogen ist,
mit einer Verbindung der Formel 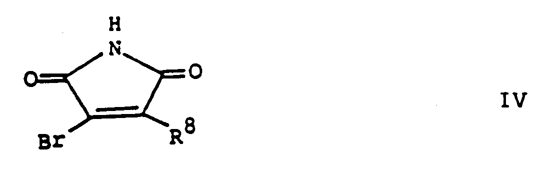 worin R⁸ die gleiche Bedeutung wie R³ in Anspruch 1 hat oder Brom ist,
umsetzt, oder
(c) zur Herstellung einer Verbindung der Formel I, in der R³ 1-Benzimidazolyl und X und Y O sind, eine Verbindung der Formel 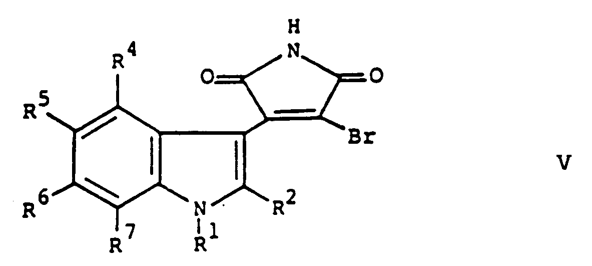 worin R¹ bis R⁷ die im Anspruch 1 angegebene Bedeutung haben,
mit einem Alkalimetallderivat des Benzimidazols umsetzt, oder
(d) zur Herstellung einer Verbindung der Formel I, in der eins von X und Y O und das andere S ist, eine Verbindung der Formel I, in der X und Y O sind, mit einem Sulfurierungsmittel umsetzt, oder
(e) zur Herstellung einer Verbindung der Formel I, in der eins von X und Y O und das andere (H,OH) ist, eine Verbindung der Formel I, in der X und Y O sind, mit einem komplexen Metallhydrid reduziert, oder
(f) zur Herstellung einer Verbindung der Formel I, in der eines von X und Y O und das andere (H,H) ist, eine Verbindung der Formel I, in der eins von X und Y O und das andere (H,OH) ist, katalytisch hydriert, oder
(g) zur Herstellung einer Verbindung der Formel I, in der R¹ Alkyl, Aralkyl, Alkoxyalkyl oder Hydroxyalkyl ist, eine Verbindung der Formel I, in der R¹ Wasserstoff ist, entsprechend N-substituiert, und
(h) erwünschtenfalls einen in einer Verbindung der Formel I vorhandenen reaktiven Substituenten funktionell abwandelt, und
(i) erwünschtenfalls eine saure Verbindung der Formel I in ein pharmazeutisch verwendbares Salz mit einer Base oder eine basische Verbindung der Formel I in ein pharmazeutisch verwendbares Salz mit einer Säure überführt.

15. Ein Verfahren zur Herstellung eines Medikaments, insbesondere eines antiinflammatorischen, immunologischen, bronchopulmonären oder cardiovaskulären Medikaments, welches Verfahren dadurch gekennzeichnet ist, dass man eine Verbindung der Formel I nach Anspruch 1 oder ein pharmazeutisch verwendbares Salz einer sauren Verbindung der Formel I mit einer Base oder einer basischen Verbindung der Formel I mit einer Säure in eine galenische Darreichungsform zusammen mit einem therapeutisch inerten Träger und erwünschtenfalls mit einem oder mehreren anderen therapeutisch wirksamen Substanzen bringt.

## Patentansprüche (Patentansprüche für folgende(n) Vertragsstaat(en): ES, GR)

1. Verfahren zur Herstellung von Pyrrolen der allgemeinen Formel 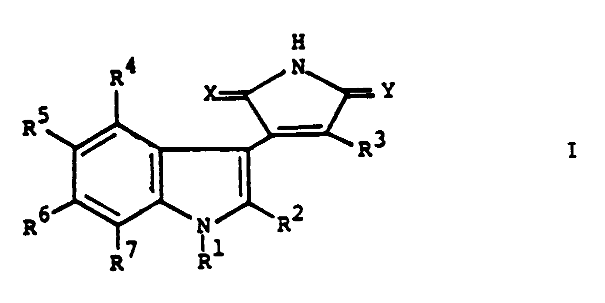 worin
R¹ Wasserstoff, Alkyl, Aryl, Aralkyl, Alkoxyalkyl, Hydroxyalkyl, Haloalkyl, Aminoalkyl, Monoalkylaminoalkyl, Dialkylaminoalkyl, Trialkylaminoalkyl, Aminoalkylaminoalkyl, Azidoalkyl, Acylaminoalkyl, Acylthioalkyl, Alkylsulfonylaminoalkyl, Arylsulphonylaminoalkyl, Mercaptoalkyl, Alkylthioalkyl, Alkylsulfinylalkyl, Alkylsulfonylalkyl, Alkylsulfonyloxyalkyl, Alkylcarbonyloxyalkyl, Cyanoalkyl, Amidinoalkyl, Isothiocyanatoalkyl, Glucopyranosyl, Carboxyalkyl, Alkoxycarbonylalkyl, Aminocarbonylalkyl, Hydroxyalkylthioalkyl, Mercaptoalkylthioalkyl, Arylthioalkyl oder Carboxyalkylthioalkyl oder eine Gruppe der Formel 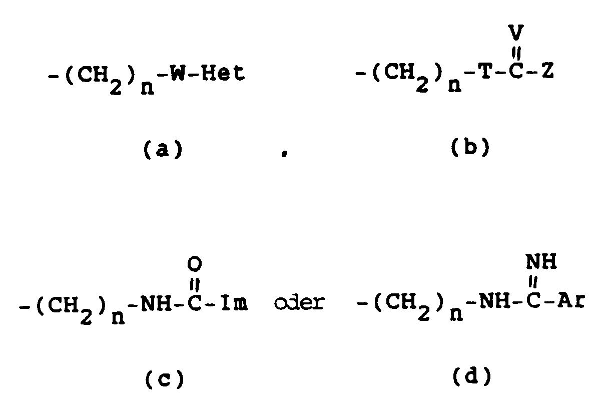 in der
Het eine heterocyclische Gruppe,
W NH, S oder eine Bindung,
T NH oder S,
V O, S, NH, NNO₂, NCN oder CHNO₂,
Z Alkylthio, Amino, Monoalkylamino oder Dialkylamino,
Im 1-Imidazolyl,
Ar Aryl, und
n eine Zahl von 2-6;
R² Wasserstoff, Alkyl, Aralkyl, Alkoxyalkyl, Hydroxyalkyl, Haloalkyl, Aminoalkyl, Monoalkylaminoalkyl, Dialkylaminoalkyl, Acylaminoalkyl, Alkylsulfonylaminoalkyl, Arylsulfonylaminoalkyl, Mercaptoalkyl, Alkylthioalkyl, Carboxyalkyl, Alkoxycarbonylalkyl, Aminocarbonylalkyl, Alkylthio oder Alkylsulfinyl;
R³ eine carbocyclische oder heterocyclische aromatische Gruppe;
R⁴, R⁵, R⁶ und R⁷ unabhängig voneinander Wasserstoff, Halogen, Alkyl, Hydroxy, Alkoxy, Aryloxy, Haloalkyl, Nitro, Amino, Acylamino, Monoalkylamino, Dialkylamino, Alkylthio, Alkylsulfinyl oder Alkylsulfonyl;
und eines von X und Y O und das andere O, S, (H,OH) oder (H,H) ist
mit der Bedingung, dass R¹ von Wasserstoff verschieden ist, falls R² Wasserstoff, R³ 3-Indolyl oder 6-Hydroxy-3-indolyl, R⁴, R⁵ und R⁷ Wasserstoff, R⁶ Wasserstoff oder Hydroxy und X und Y beide O sind, sowie falls R² Wasserstoff, R³ 3-Indolyl, R⁴, R⁵, R⁶ und R⁷ Wasserstoff, X (H,H) und Y O sind;
wobei eine heterocyclische Gruppe Het für eine gesättigte, teilweise ungesättigte oder eine aromatische 5- oder 6-gliedrige heterocyclische Gruppe steht, die gewünschtenfalls einen ankondensierten Benzolring enthält und unsubstituiert oder substituiert ist durch einen oder mehrere Substituenten aus der Gruppe von Halogen, Alkyl, Hydroxy, Alkoxy, Haloalkyl, Nitro, Amino, Acylamino, Mono- oder Dialkylamino, Alkylthio, Alkylsulfinyl und Alkylsufonyl,
eine heterocyclische aromatische Gruppe R³ wie weiter oben eine heterocyclische aromatische Gruppe Het definiert ist, und
eine carbocyclische aromatische Gruppe R³ für Phenyl oder Naphthyl steht, das gewünschtenfalls wie die obige Gruppe Het substituiert ist, und
Acyl allein oder in Kombination eine von einer Alkancarbonsäure mit bis zu 7 C-Atome oder von einer aromatischen Carbonsäure abgeleitete Gruppe ist, und
Alkyl allein oder in Kombination für eine geradkettige oder verzweigte Alkylgruppe mit bis zu 7 C-Atome steht,
sowie pharmazeutisch verwendbare Salze von sauren Verbindungen der Formel I mit Basen und von basischen Verbindungen der Formel I mit Säuren, dadurch gekennzeichnet, daß man
a) zur Herstellung einer Verbindung der Formel I, in der X und Y O sind, eine Verbindung der Formel 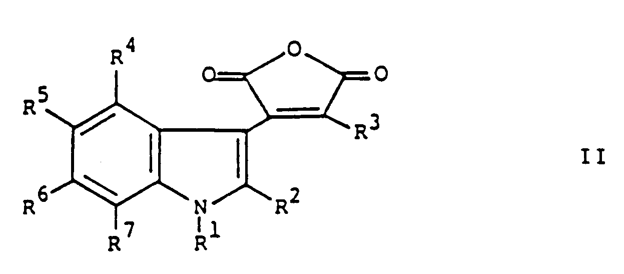 worin R¹ bis R⁷ die im Anspruch 1 angegebene Bedeutung haben,
mit Ammoniak unter Druck oder mit Hexamethyldisilazan und Methanol umsetzt, oder
(b) zur Herstellung einer Verbindung der Formel I, in der R¹ Wasserstoff und X und Y O sind, eine Verbindung der Formel 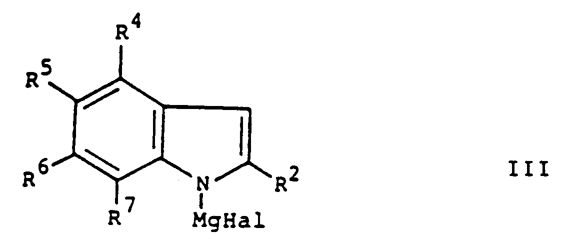 worin R² bis R⁷ die weiter oben angegebene Bedeutung haben und Hal Halogen ist,
mit einer Verbindung der Formel 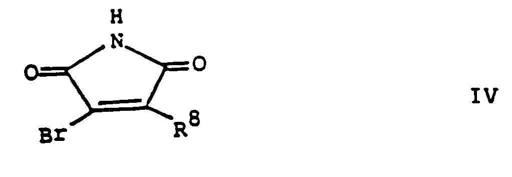 worin R⁸ die gleiche Bedeutung wie R³ hat oder Brom ist,
umsetzt, oder
(c) zur Herstellung einer Verbindung der Formel I, in der R³ 1-Benzimidazolyl und X und Y O sind, eine Verbindung der Formel 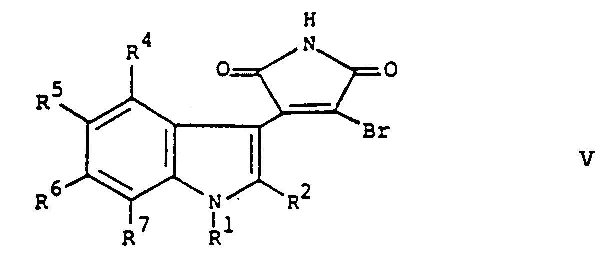 worin R¹ bis R⁷ die weiter oben angegebene Bedeutung haben,
mit einem Alkalimetallderivat des Benzimidazols umsetzt, oder
(d) zur Herstellung einer Verbindung der Formel I, in der eins von X und Y O und das andere S ist, eine Verbindung der Formel I, in der X und Y O sind, mit einem Sulfurierungsmittel umsetzt, oder
(e) zur Herstellung einer Verbindung der Formel I, in der eins von X und Y O und das andere (H,OH) ist, eine Verbindung der Formel I, in der X und Y O sind, mit einem komplexen Metallhydrid reduziert, oder
(f) zur Herstellung einer Verbindung der Formel I, in der eines von X und Y O und das andere (H,H) ist, eine Verbindung der Formel I, in der eins von X und Y O und das andere (H,OH) ist, katalytisch hydriert, oder
(g) zur Herstellung einer Verbindung der Formel I, in der R¹ Alkyl, Aralkyl, Alkoxyalkyl oder Hydroxyalkyl ist, eine Verbindung der Formel I, in der R¹ Wasserstoff ist, entsprechend N-substituiert, und
(h) erwünschtenfalls einen in einer Verbindung der Formel I vorhandenen reaktiven Substituenten funktionell abwandelt, und
(i) erwünschtenfalls eine saure Verbindung der Formel I in ein pharmazeutisch verwendbares Salz mit einer Base oder eine basische Verbindung der Formel I in ein pharmazeutisch verwendbares Salz mit einer Säure überführt.

2. Verfahren gemäss Anspruch 1, worin R¹ Wasserstoff, Alkyl, Aralkyl, Alkoxyalkyl, Hydroxyalkyl, Haloalkyl, Aminoalkyl, Monoalkylaminoalkyl, Dialkylaminoalkyl, Azidoalkyl, Acylaminoalkyl, Alkylsulfonylaminoalkyl, Arylsulfonylaminoalkyl, Mercaptoalkyl, Alkylthioalkyl, Glucopyranosyl, Carboxyalkyl, Alkoxycarbonylalkyl, Aminocarbonylalkyl, Hydroxyalkylthioalkyl, Mercaptoalkylthioalkyl, Arylthioalkyl oder Carboxyalkylthioalkyl ist.

3. Verfahren gemäss Anspruch 2, worin R¹ Alkyl oder Aminoalkyl ist.

4. Verfahren gemäss Anspruch 1, worin R¹ Isothiocyanatoalkyl oder eine Gruppe der Formel (b), in der T S, V NH und Z Amino sind oder in der T NH, V NH oder NNO₂ und Z Amino bedeuten.

5. Verfahren nach einem der Ansprüche 1-4, worin R² Wasserstoff ist.

6. Verfahren gemäss einem der Ansprüche 1-5, worin R³ durch Halogen, Alkyl, Alkoxy, Haloalkyl, Nitro, Amino, Alkylthio, Alkylsulfinyl oder Alkylsulfonyl monosubstituiertes Phenyl ist.

7. Verfahren nach einem der Ansprüche 1-5, worin R³ eine Gruppe der Formel 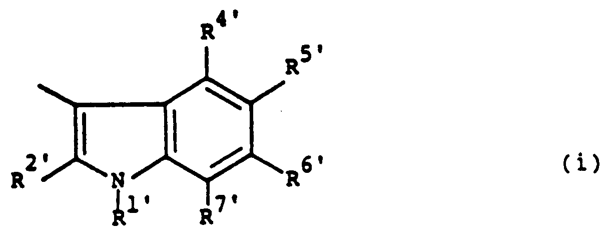 worin R^{1′}, R^{2′}, R^{4′}, R^{5′}, R^{6′} und R^{7′} eine der Bedeutungen von R¹, R², R⁴, R⁵, R⁶ bzw. R⁷ im Anspruch 1 haben, ist.

8. Verfahren gemäss einem der Ansprüche 1-7, worin R⁴, R⁵, R⁶ und R⁷ Wasserstoff sind.

9. Verfahren nach einem der Ansprüche 1-8, worin R¹ Methyl, 3-Aminopropyl, 3-Isothiocyanatopropyl oder eine Gruppe der Formel (b), in der T S, V NH, Z Amino und n die Zahl 3 ist oder in der T NH, V NH oder NNO₂, Z Amino und n die Zahl 3, R² Wasserstoff, R³ durch Chlor, Brom, Methyl, Methoxy, Trifluormethyl, Nitro, Amino, Methylsulfinyl oder Methylsulfonyl monosubstituiertes Phenyl oder eine Gruppe der Formel (i) nach Anspruch 7, in der R^{1′} Methyl und R^{2′}, R^{4′}, R^{5′}, R^{6′} und R^{7′} Wasserstoff, und R⁴, R⁵, R⁶ und R⁷ Wasserstoff sind.

10. Verfahren nach Anspruch 1 zur Herstellung eines Pyrrols aus der Gruppe der folgenden:
3-(2-Chlorphenyl)-4-(1-methyl-3-indolyl)-1H-pyrrol-2,5-dion,
3-(1-Methyl-3-indolyl)-4-(2-nitrophenyl)-1H-pyrrol-2,5-dion,
3,4-Bis(1-methyl-3-indolyl)-1H-pyrrol-2,5-dion,
3-[1-(3-Aminopropyl)-3-indolyl]-4-(1-methyl-3-indolyl)-1H-pyrrol-2,5-dion,
3-[1-[3-(Amidinothio)propyl]-3-indolyl]-4-(1-methyl-3-indolyl)-1H-pyrrol-2,5-dion,
3-(1-Methyl-3-indolyl)-4-[1-[3-(2-nitroguanidino)propyl]-3-indolyl]-1H-pyrrol-2,5-dion und
3-[1-(3-Isothiocyanatopropyl)-3-indolyl]-4-(1-methyl-3-indolyl)-1H-pyrrol-2,5-dion.

11. Verfahren nach Anspruch 1, dadurch gekennzeichnet, dass man
a) die Verbindung der Formel II mit wässrigem Ammoniak bzw. mit Hexamethyldisilazan und Methanol in Gegenwart eines inerten organischen Lösungsmittels bei einer Temperatur zwischen etwa 100 bis 150°C bzw. zwischen etwa 40 und 110°C umsetzt, oder
b) die Grignard-Reaktion zwischen den Verbindungen der Formeln III und IV in einem inerten organischen Lösungsmittel bei einer Temperatur bis zum Rückfluss durchführt, oder
c) die Verbindung der Formel V mit einem Alkalimetallderivat des Benzimidazols in einem inerten organischen Lösungsmittel bei einer Temperatur zwischen etwa 45 und 95°C umsetzt, oder
d) bei der Sulfurierung einer Verbindung der Formel I Phosphorpentasulfid oder das Lawesson- oder Davy-Reagenz in einem inerten organischen Lösungsmittel bei einer Temperatur bis zum Rückfluss einsetzt, oder
e) Lithiumaluminiumhydrid, Diisobutylaluminiumhydrid oder Natriumdihydro-bis(2-methoxyäthoxy)aluminat als komplexen Metallhydrid verwendet, oder
f) einen Palladium- oder Platinkatalysator als Hydrierungskatalysator verwendet, oder
g) die N-Substitution mittels eines Alkylenoxids, eines Aldehyddialkylacetals, oder eines Alkyl- oder Aralkylhalogenids bewerkstelligt.

12. Die Verwendung einer Verbindung gemäss einem der Ansprüche 1-10 bei der Herstellung eines Medikamentes gegen inflammatorische, immunologische, bronchopulmonäre oder cardiovaskuläre Krankheiten.

13. Ein Verfahren zur Herstellung eines Medikaments, insbesondere eines antiinflammatorischen, immunologischen, bronchopulmonären oder cardiovaskulären Medikaments, welches Verfahren dadurch gekennzeichnet ist, dass man eine Verbindung der Formel I nach Anspruch 1 oder ein pharmazeutisch verwendbares Salz einer sauren Verbindung der Formel I mit einer Base oder einer basischen Verbindung der Formel I mit einer Säure in eine galenische Darreichungsform zusammen mit einem therapeutisch inerten Träger und erwünschtenfalls mit einem oder mehreren anderen therapeutisch wirksamen Substanzen bringt.

## Claims (Claims for the following Contracting State(s): AT, BE, CH, DE, FR, GB, IT, LI, LU, NL, SE)

1. Pyrroles of the general formula 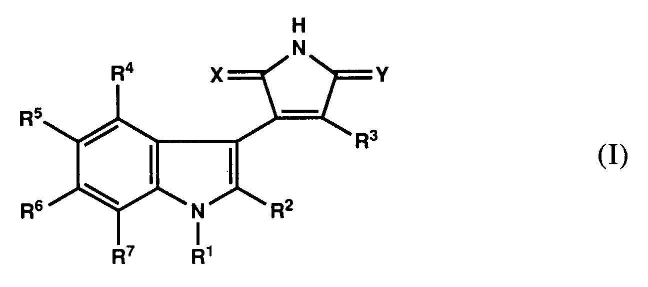 wherein
R¹ is hydrogen, alkyl, aryl, aralkyl, alkoxyalkyl, hydroxyalkyl, haloalkyl, aminoalkyl, monoalkylaminoalkyl, dialkylaminoalkyl, trialkylaminoalkyl, aminoalkylaminoalkyl, azidoalkyl, acylaminoalkyl, acylthioalkyl, alkylsulphonylaminoalkyl, arylsulphonylaminoalkyl, mercaptoalkyl, alkylthioalkyl, alkylsulphinylalkyl, alkylsulphonylalkyl, alkylsulphonyloxyalkyl, alkylcarbonyloxyalkyl, cyanoalkyl, amidinoalkyl, isothiocyanatoalkyl, glucopyranosyl, carboxyalkyl, alkoxycarbonylalkyl, aminocarbonylalkyl, hydroxyalkylthioalkyl, mercaptoalkylthioalkyl, arylthioalkyl or carboxyalkylthioalkyl or a group of the formula 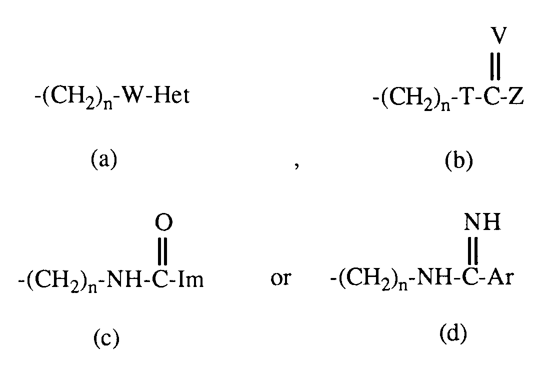 in which
Het is a heterocyclic group,
W is NH, S or a bond,
T is NH or S,
V is O, S, NH, NNO₂, NCN or CHNO₂,
Z is alkylthio, amino, monoalkylamino or dialkylamino,
Im is 1-imidazolyl,
Ar is aryl and
n is an integer of 2-6;
R² is hydrogen, alkyl, aralkyl, alkoxyalkyl, hydroxyalkyl, haloalkyl, aminoalkyl, monoalkylaminoalkyl, dialkylaminoalkyl, acylaminoalkyl, alkylsulphonylaminoalkyl, arylsulphonylaminoalkyl, mercaptoalkyl, alkylthioalkyl, carboxyalkyl, alkoxycarbonylalkyl, aminocarbonylalkyl, alkylthio or alkylsulphinyl;
R³ is a carbocyclic or heterocyclic aromatic group;
R⁴, R⁵, R⁶ and R⁷ are each independently hydrogen, halogen, alkyl, hydroxy, alkoxy, aryloxy, haloalkyl, nitro, amino, acylamino, monoalkylamino, dialkylamino, alkylthio, alkylsulphinyl or alkylsulphonyl;
and one of X and Y is O and the other is O, S, (H,OH) or (H,H);
with the proviso that R¹ is different from hydrogen when R² is hydrogen, R³ is 3-indolyl or 6-hydroxy-3-indolyl, R⁴, R⁵ and R⁷ are hydrogen, R⁶ is hydrogen or hydroxy and X and Y are both O and when R² is hydrogen, R³ is 3-indolyl, R⁴, R⁵, R⁶ and R⁷ are hydrogen, X is (H,H) and Y is O;
whereby a heterocyclic group Het stands for a saturated, partially unsaturated or aromatic 5- or 6-membered heterocyclic group which, if desired, contains a fused benzene ring and is unsubstituted or substituted by one or more substituents from the group of halogen, alkyl, hydroxy, alkoxy, haloalkyl, nitro, amino, acylamino, mono- or dialkylamino, alkylthio, alkylsulphinyl and alkylsulphonyl,
a heterocyclic aromatic group R³ is a heterocyclic aromatic group Het as defined above, and
a carboxylic aromatic group R³ stands for phenyl or naphthyl which, if desired, is substituted in the same manner as the Het group above, and
acyl alone or in combination is a group derived from an alkanecarboxylic acid with up to 7 C atoms or from an aromatic carboxylic acid, and
alkyl alone or in combination stands for a straight-chain or branched alkyl group with up to 7 C atoms,
as well as pharmaceutically usable salts of acidic compounds of formula I with bases and of basic compounds of formula I with acids.

2. Compounds according to claim 1, wherein R¹ is hydrogen, alkyl, aralkyl, alkoxyalkyl, hydroxyalkyl, haloalkyl, aminoalkyl, monoalkylaminoalkyl, dialkylaminoalkyl, azidoalkyl, acylaminoalkyl, alkylsulphonylaminoalkyl, arylsulphonylaminoalkyl, mercaptoalkyl, alkylthioalkyl, glucopyranosyl, carboxyalkyl, alkoxycarbonylalkyl, aminocarbonylalkyl, hydroxyalkylthioalkyl, mercaptoalkylthioalkyl, arylthioalkyl or carboxyalkylthioalkyl.

3. Compounds according to claim 2, wherein R¹ is alkyl or aminoalkyl.

4. Compounds according to claim 1, wherein R¹ is isothiocyanatoalkyl or a group of formula (b) in which T is S, V is NH and Z is amino or in which T is NH, V is NH or NNO₂ and Z is amino.

5. Compounds according to any one of claims 1 to 4, wherein R² is hydrogen.

6. Compounds according to any one of claims 1-5, wherein R³ is phenyl monosubstituted by halogen, alkyl, alkoxy, haloalkyl, nitro, amino, alkylthio, alkylsulphinyl or alkylsulphonyl.

7. Compounds according to any one of claims 1-5, wherein R³ is a group of the formula 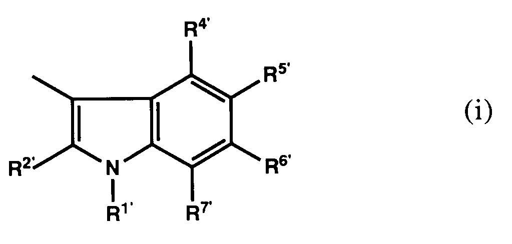 wherein R¹', R²', R⁴', R⁵', R⁶' and R⁷' have any of the meanings of R¹, R², R⁴, R⁵, R⁶ and R⁷, respectively, in claim 1.

8. Compounds according to any one of claims 1-7, wherein R⁴, R⁵, R⁶ and R⁷ are hydrogen.

9. Compounds according to any one of claims 1-8, wherein R¹ is methyl, 3-aminopropyl, 3-isothiocyanatopropyl or a group of formula (b) in which T is S, V is NH, Z is amino and n is the integer 3 or in which T is NH, V is NH or NNO₂, Z is amino and n is the integer 3, R² is hydrogen, R³ is phenyl monosubstituted by chlorine, bromine, methyl, methoxy, trifluoromethyl, nitro, amino, methylsulphinyl or methylsulphonyl or a group of formula (i) according to claim 7 in which R¹' is methyl and R²', R⁴', R⁵' R⁶' and R⁷' are hydrogen, and R⁴, R⁵, R⁶ and R⁷ are hydrogen.

10. A pyrrole from the following group:
3-(2-Chlorophenyl)-4-(1-methyl-3-indolyl)-1H-pyrrole-2,5-dione,
3-(1-methyl-3-indolyl)-4-(2-nitrophenyl)-1H-pyrrole-2,5-dione,
3,4-bis(1-methyl-3-indolyl)-1H-pyrrole-2,5-dione,
3-[1-(3-aminopropyl)-3-indolyl]-4-(1-methyl-3-indolyl)-1H-pyrrole-2,5-dione,
3-[1-[3-(amidinothio)propyl]-3-indolyl]-4-(1-methyl-3-indolyl)-1H-pyrrole-2,5-dione,
3-(1-methyl-3-indolyl)-4-[1-[3-(2-nitroguanidino)-propyl]-3-indolyl]-1H-pyrrole-2,5-dione and
3-[1-(3-isothiocyanatopropyl)-3-indolyl]-4-(1-methyl-3-indolyl)-1H-pyrrole-2,5-dione.

11. Compounds according to any one of claims 1-10 for use as therapeutically active substances, especially as antiinflammatory, immunological, bronchopulmonary and cardiovascular active substances.

12. A medicament, especially an antiinflammatory, immunological, bronchopulmonary or cardiovascular medicament, containing a compound according to any one of claims 1-10 and a therapeutically inert carrier material.

13. The use of a compound according to any one of claims 1-10 for the manufacture of a medicament against inflammatory, immunological, bronchopulmonary or cardiovascular disorders.

14. A process for the manufacture of the pyrroles according to any one of claims 1-10, characterized by
(a) for the manufacture of a compound of formula I in which X and Y are O, reacting a compound of the formula 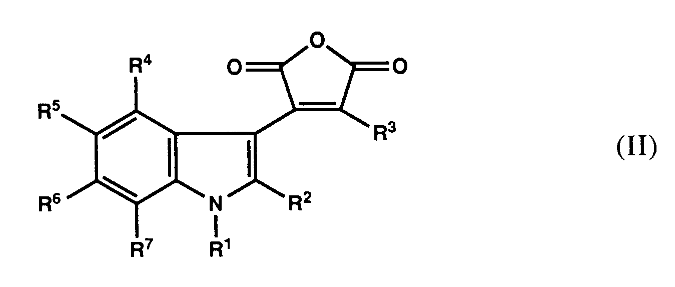 wherein R¹ to R⁷ have the significance given in claim 1,
with ammonia under pressure or with hexamethyldisilazane and methanol, or
(b) for the manufacture of a compound of formula I in which R¹ is hydrogen and X and Y are O, reacting a compound of the formula 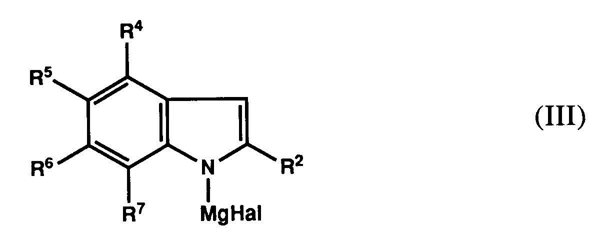 wherein R² to R⁷ have the significance given in claim 1 and Hal is halogen,
with a compound of the formula 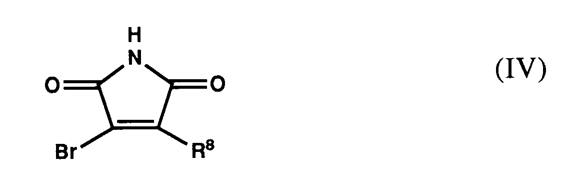 wherein R⁸ has the same significance as R³ in claim 1 or is bromine,
or
(c) for the manufacture of a compound of formula I in which R³ is 1 -benzimidazolyl and X and Y are O, reacting a compound of the formula 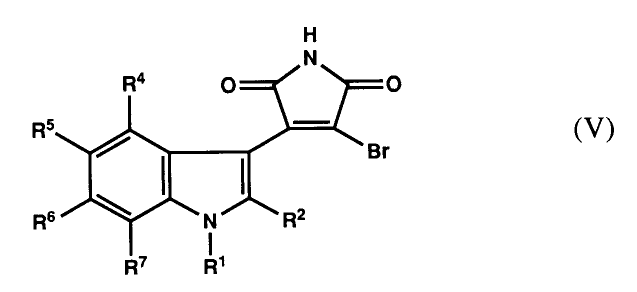 wherein R¹ to R⁷ have the significance given in claim 1,
with an alkali metal derivative of benzimidazole, or
(d) for the manufacture of a compound of formula I in which one of X and Y is O and the other is S, reacting a compound of formula I in which X and Y are O with a sulphurizing agent, or
(e) for the manufacture of a compound of formula I in which one of X and Y is O and the other is (H, OH), reducing a compound of formula I in which X and Y are O with a complex metal hydride, or
(f) for the manufacture of a compound of formula I in which one of X and Y is O and the other is (H,H), catalytically hydrogenating a compound of formula I in which one of X and Y is O and the other is (H,OH), or
(g) for the manufacture of a compound of formula I in which R¹ is alkyl, aralkyl, alkoxyalkyl or hydroxyalkyl, appropriately N-substituting a compound of formula I in which R¹ is hydrogen, and
(h) if desired, functionally modifying a reactive substituent present in a compound of formula I, and
(i) if desired, converting an acidic compound of formula I into a pharmaceutically usable salt with a base or converting a basic compound of formula I into a pharmaceutically usable salt with an acid.

15. A process for the manufacture of a medicament, especially an antiinflammatory, immunological, bronchopulmonary or cardiovascular medicament, which process is characterized by bringing a compound of formula I according to claim 1 or a pharmaceutically usable salt of an acidic compound of formula I with a base or of a basic compound of formula I with an acid into a galenical administration form together with a therapeutically inert carrier material and, if desired, one or more other therapeutically active substances.

## Claims (Claims for the following Contracting State(s): ES, GR)

1. A process for the manufacture of pyrroles of the general formula 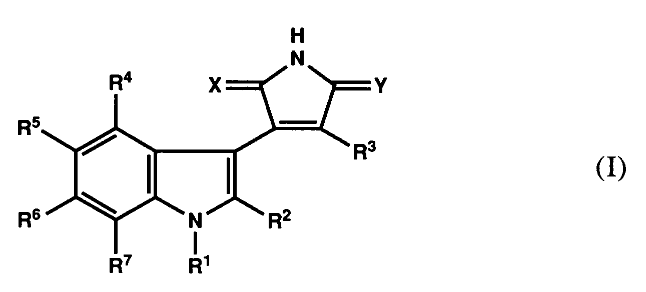 wherein
R¹ is hydrogen, alkyl, aryl, aralkyl, alkoxyalkyl, hydroxyalkyl, haloalkyl, aminoalkyl, monoalkylaminoalkyl, dialkylaminoalkyl, trialkylaminoalkyl, aminoalkylaminoalkyl, azidoalkyl, acylaminoalkyl, acylthioalkyl, alkylsulphonylaminoalkyl, arylsulphonylaminoalkyl, mercaptoalkyl, alkylthioalkyl, alkylsulphinylalkyl, alkylsulphonylalkyl, alkylsulphonyloxyalkyl, alkylcarbonyloxyalkyl, cyanoalkyl, amidinoalkyl, isothiocyanatoalkyl, glucopyranosyl, carboxyalkyl, alkoxycarbonylalkyl, aminocarbonylalkyl, hydroxyalkylthioalkyl, mercaptoalkylthioalkyl, arylthioalkyl or carboxyalkylthioalkyl or a group of the formula 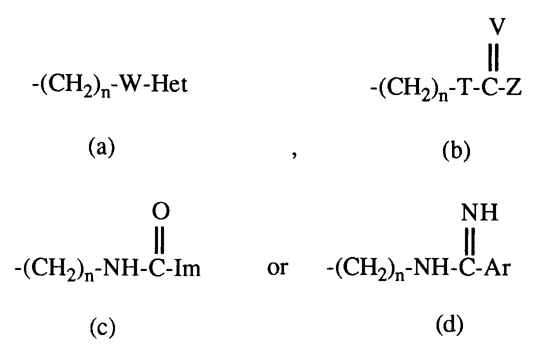 in which
Het is a heterocyclic group,
W is NH, S or a bond,
T is NH or S,
V is O, S, NH, NNO₂, NCN or CHNO₂,
Z is alkylthio, amino, monoalkylamino or dialkylamino,
Im is 1-imidazolyl,
Ar is aryl and
n is an integer of 2-6;
R² is hydrogen, alkyl, aralkyl, alkoxyalkyl, hydroxyalkyl, haloalkyl, aminoalkyl, monoalkylaminoalkyl, dialkylaminoalkyl, acylaminoalkyl, alkylsulphonylaminoalkyl, arylsulphonylaminoalkyl, mercaptoalkyl, alkylthioalkyl, carboxyalkyl, alkoxycarbonylalkyl, aminocarbonylalkyl, alkylthio or alkylsulphinyl;
R³ is a carbocyclic or heterocyclic aromatic group;
R⁴, R⁵, R⁶ and R⁷ are each independently hydrogen, halogen, alkyl, hydroxy, alkoxy, aryloxy, haloalkyl, nitro, amino, acylamino, monoalkylamino, dialkylamino, alkylthio, alkylsulphinyl or alkylsulphonyl;
and one of X and Y is O and the other is O, S, (H,OH) or (H,H);
with the proviso that R¹ is different from hydrogen when R² is hydrogen, R³ is 3-indolyl or 6-hydroxy-3-indolyl, R⁴, R⁵ and R⁷ are hydrogen, R⁶ is hydrogen or hydroxy and X and Y are both O and when R² is hydrogen, R³ is 3-indolyl, R⁴, R⁵, R⁶ and R⁷ are hydrogen, X is (H,H) and Y is O;
whereby a heterocyclic group Het stands for a saturated, partially unsaturated or aromatic 5- or 6-membered heterocyclic group which, if desired, contains a fused benzene ring and is unsubstituted or substituted by one or more substituents from the group of halogen, alkyl, hydroxy, alkoxy, haloalkyl, nitro, amino, acylamino, mono- or dialkylamino, alkylthio, alkylsulphinyl and alkylsulphonyl,
a heterocyclic aromatic group R³ is a heterocyclic aromatic group Het as defined above, and
a carboxylic aromatic group R³ stands for phenyl or naphthyl which, if desired, is substituted in the same manner as the Het group above, and
acyl alone or in combination is a group derived from an alkanecarboxylic acid with up to 7 C atoms or from an aromatic carboxylic acid, and
alkyl alone or in combination stands for a straight-chain or branched alkyl group with up to 7 C atoms,
as well as pharmaceutically usable salts of acidic compounds of formula I with bases and of basic compounds of formula I with acids, characterized by
a) for the manufacture of a compound of formula I in which X and Y are O, reacting a compound of the formula 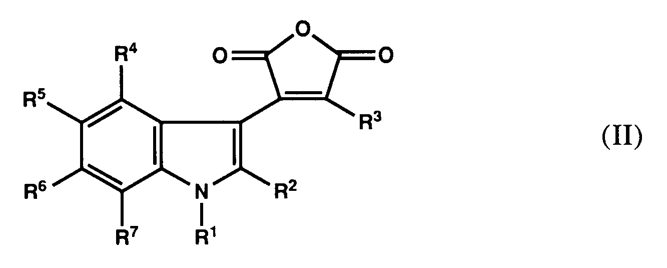 wherein R¹ to R⁷ have the significance given in claim 1,
with ammonia under pressure of with hexamethyldisilazane and methanol, or
(b) for the manufacture of a compound of formula I in which R¹ is hydrogen and X and Y are O, reacting a compound of the formula 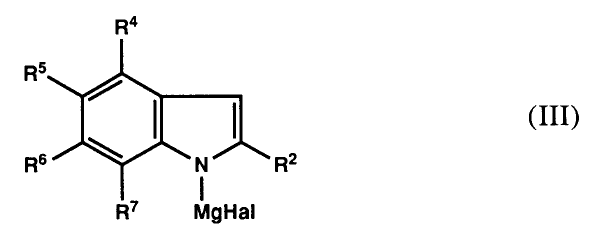 wherein R² to R⁷ have the significance given above and Hal is halogen,
with a compound of the formula 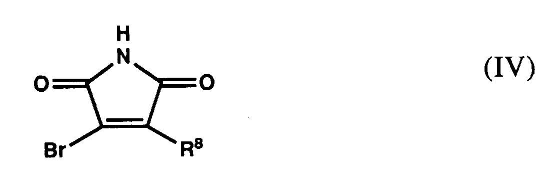 wherein R⁸ has the same significance as R³ or is bromine,
or
(c) for the manufacture of a compound of formula I in which R³ is 1-benzimidazolyl and X and Y are O, reacting a compound of the formula 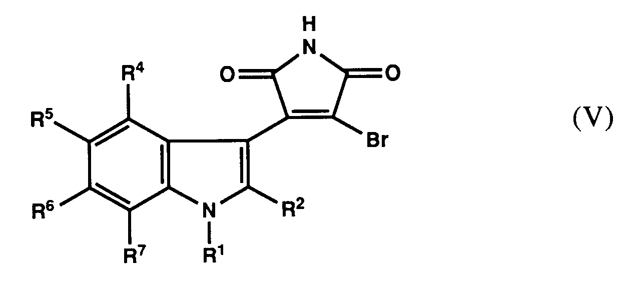 wherein R¹ to R⁷ have the significance given above,
with an alkali metal derivative of benzimidazole, or
(d) for the manufacture of a compound of formula I in which one of X and Y is O and the other is S, reacting a compound of formula I in which X and Y are O with a sulphurizing agent, or
(e) for the manufacture of a compound of formula I in which one of X and Y is O and the other is (H,OH), reducing a compound of formula I in which X and Y are O with a complex metal hydride, or
(f) for the manufacture of a compound of formula I in which one of X and Y is O and the other is (H,H), catalytically hydrogenating a compound of formula I in which one of X and Y is O and the other is (H,OH), or
(g) for the manufacture of a compound of formula I in which R¹ is alkyl, aralkyl, alkoxyalkyl or hydroxyalkyl, appropriately N-substituting a compound of formula I in which R¹ is hydrogen, and
(h) if desired, functionally modifying a reactive substituent present in a compound of formula I, and
(i) if desired, converting an acidic compound of formula I into a pharmaceutically usable salt with a base or converting a basic compound of formula I into a pharmaceutically usable salt with an acid.

2. A process according to claim 1, wherein R¹ is hydrogen, alkyl, aralkyl, alkoxyalkyl, hydroxyalkyl, haloalkyl, aminoalkyl, monoalkylaminoalkyl, dialkylaminoalkyl, azidoalkyl, acylaminoalkyl, alkylsulphonylaminoalkyl, arylsulphonylaminoalkyl, mercaptoalkyl, alkylthioalkyl, glucopyranosyl, carboxyalkyl, alkoxycarbonylalkyl, aminocarbonylalkyl, hydroxyalkylthioalkyl, mercaptoalkylthioalkyl, arylthioalkyl or carboxyalkylthioalkyl.

3. A process according to claim 2, wherein R¹ is alkyl or aminoalkyl.

4. A process according to claim 1, wherein R¹ is isothiocyanatoalkyl or a group of formula (b) in which T is S, V is NH and Z is amino or in which T is NH, V is NH or NNO₂ and Z is amino.

5. A process according to any one of claims 1 to 4, wherein R² is hydrogen.

6. A process according to any one of claims 1-5, wherein R³ is phenyl monosubstituted by halogen, alkyl, alkoxy, haloalkyl, nitro, amino, alkylthio, alkylsulphinyl or alkylsulphonyl.

7. A process according to any one of claims 1-5, wherein R³ is a group of the formula 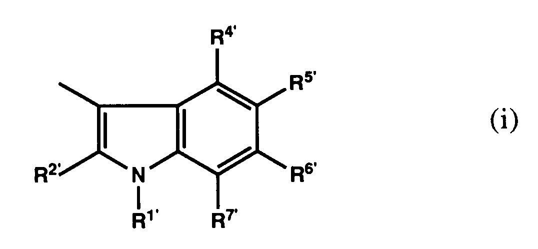 wherein R¹', R²', R⁴', R⁵', R⁶' and R⁷' have any of the meanings of R¹, R², R⁴, R⁵, R⁶ and R⁷, respectively, in claim 1.

8. A process according to any one of claims 1-7, wherein R⁴, R⁵, R⁶ and R⁷ are hydrogen.

9. A process according to any one of claims 1-8, wherein R¹ is methyl, 3-aminopropyl, 3-isothiocyanatopropyl or a group of formula (b) in which T is S, V is NH, Z is amino and n is the integer 3 or in which T is NH, V is NH or NNO₂, Z is amino and n is the integer 3, R² is hydrogen, R³ is phenyl monosubstituted by chlorine, bromine, methyl, methoxy, trifluoromethyl, nitro, amino, methylsulphinyl or methylsulphonyl or a group of formula (i) according to claim 7 in which R¹' is methyl and R²', R⁴', R⁵' R⁶' and R⁷' are hydrogen, and R⁴, R⁵, R⁶ and R⁷ are hydrogen.

10. A process according to claim 1 for the manufacture of a pyrrole from the following group:
3-(2-Chlorophenyl)-4-(1-methyl-3-indolyl)-1H-pyrrole-2,5-dione,
3-(1-methyl-3-indolyl)-4-(2-nitrophenyl)-1H-pyrrole-2,5-dione,
3,4-bis(1-methyl-3-indolyl)-1H-pyrrole-2,5-dione,
3-[1-(3-aminopropyl)-3-indolyl]-4-(1-methyl-3-indolyl)-1H-pyrrole-2,5-dione,
1-[1-[3-(amidinothio)propyl]-3-indolyl]-4-(1-methyl-3-indolyl)-1H-pyrrole-2,5-dione,
3-(1-methyl-3-indolyl)-4-[1-[3-(2-nitroguanidino)-propyl]-3-indolyl]-1H-pyrrole-2,5-dione and
3-[1-(3-isothiocyanatopropyl)-3-indolyl]-4-(1-methyl-3-indolyl)-1H-pyrrole-2,5-dione.

11. A process according to claim 1, characterized in that
a) the compound of formula II is reacted with aqueous ammonia or with hexamethyldisilazane and methanol in the presence of an inert organic solvent at a temperature between about 100 and 150°C and, respectively, between about 40 and 100°C, or
b) the Grignard reaction between the compounds of formulae III and IV is carried out in an inert organic solvent at a temperature up to reflux, or
c) the compound of formula V is reacted with an alkali metal derivative of benzimidazole in an inert organic solvent at a temperature between about 45 and 95°C, or
d) in the sulphurization of a compound of formula I, phosphorus pentasulphide or the Lawesson or Davy reagent is used in an inert organic solvent at a temperature up to reflux, or
e) lithium aluminium hydride, diisobutylaluminium hydride or sodium dihydro-bis(2-methoxyethoxy)aluminate is used as the complex metal hydride, or
f) a palladium or platinum catalyst is used as the hydrogenation catalyst, or
g) the N-substitution is carried out using an alkylene oxide, an aldehyde dialkyl acetal or an alkyl or aralkyl halide.

12. The use of a compound according to any one of claims 1-10 for the manufacture of a medicament against antiinflammatory, immunological, bronchopulmonary or cardiovascular disorders.

13. A process for the manufacture of a medicament, especially an antiinflammatory, immunological, bronchopulmonary or cardiovascular medicament, which process is characterized by bringing a compound of formula I according to claims 1 or a pharmaceutically usable salt of an acidic compound of formula I with a base or of a basic compound of formula I with an acid into a galenical administration from together with a therapeutically inert carrier material and, if desired, one or more other therapeutically active substances.

## Revendications (Revendications pour l'(les) Etat(s) contractant(s) suivant(s): AT, BE, CH, DE, FR, GB, IT, LI, LU, NL, SE)

1. Pyrroles de formule générale 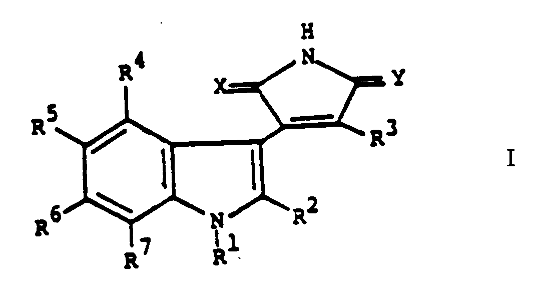 où
R¹ représente l'hydrogène, un alkyle, un aryle, un aralkyle, un alcoxyalkyle, un hydroxyalkyle, un haloalkyle, un aminoalkyle, un monoalkylaminoalkyle, un dialkylaminoalkyle, un trialkylaminoalkyle, un aminoalkylaminoalkyle, un azidoalkyle, un acylaminoalkyle, un acylthioalkyle, un alkylsulfonylaminoalkyle, un arylsulfonylaminoalkyle, un mercaptoalkyle, un alkylthioalkyle, un alkylsulfinylalkyle, un alkylsulfonylalkyle, un alkylsulfonyloxyalkyle, un alkylcarbonyloxyalkyle, un cyanoalkyle, un amidinoalkyle, un isothiocyanatoalkyle, un glucopyranosyle, un carboxyalkyle, un alcoxycarbonylalkyle, un aminocarbonylalkyle, un hydroxyalkylthioalkyle, un mercaptoalkylthioalkyle, un arylthioalkyle ou un carboxyalkylthioalkyle ou un groupe de formules 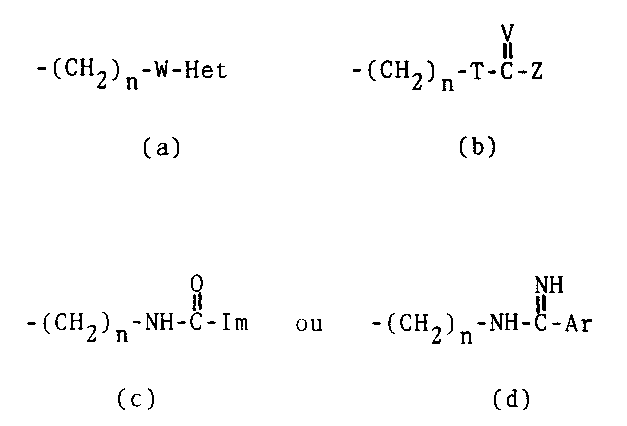 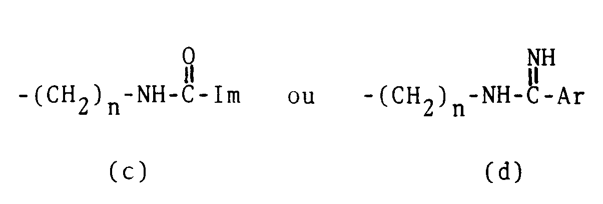 dans lesquelles
Het représente un groupe hétérocyclique,
W NH, S ou une liaison,
T NH ou S,
V O, S, NH, NNO₂, NCN ou CHNO₂,
Z un alkylthio, un amino, un monoalkylamino ou un dialkylamino,
Im le 1-imidazolyle,
Ar un aryle et
n un nombre allant de 2 à 6 ;
R² l'hydrogène, un alkyle, un aralkyle, un alcoxyalkyle, un hydroxyalkyle, un haloalkyle, un aminoalkyle, un monoalkylaminoalkyle, un dialkylaminoalkyle, un acylaminoalkyle, un alkylsulfonylaminoalkyle, un arylsulfonylaminoalkyle, un mercaptoalkyle, un alkylthioalkyle, un carboxyalkyle, un alcoxycarbonylalkyle, un aminocarbonylalkyle, un alkylthio ou un alkylsulfinyle ;
R³ représente un groupe aromatique carbocyclique ou hétérocyclique ;
R⁴, R⁵, R⁶ et R⁷ représentent, indépendamment les uns des autres, l'hydrogène, un halogène, un alkyle, un hydroxy, un alcoxy, un aryloxy, un haloalkyle, un nitro, un amino, un acylamino, un monoalkylamino, un dialkylamino, un alkylthio, un alkylsulfinyle ou un alkylsulfonyle ;
et l'un des symboles X et Y est O et l'autre O, S, (H,OH) ou (H,H),
sous réserve que R¹ est différent de l'hydrogène, lorsque R² est l'hydrogène, R³ est le 3-indolyle ou le 6-hydroxy-3-indolyle, R⁴, R⁵ et R⁷ sont l'hydrogène, R⁶ est l'hydrogène ou un hydroxy et X et Y sont tous les deux O ainsi que lorsque R² est l'hydrogène, R³ est le 3-indolyle, R⁴, R⁵, R⁶ et R⁷ sont l'hydrogène, X est (H,H) et Y est O ;
un groupe hétérocyclique Het signifiant un groupe hétérocyclique à 5 ou 6 chaînons, saturé, partiellement insaturé ou aromatique, contenant, si désiré, un noyau benzénique condensé et étant non substitué ou substitué par un ou plusieurs substituants choisis dans le groupe constitué par un halogène, un alkyle, un hydroxy, un alcoxy, un haloalkyle, un nitro, un amino, un acylamino, un mono- ou dialkylamino, un alkylthio, un alkylsulfinyle et un alkylsulfonyle, un groupe R³ aromatique hétérocyclique étant défini comme le groupe Het aromatique hétérocyclique ci-dessus et
un groupe R³ aromatique carbocyclique représentant le phényle ou le naphtyle, étant si désiré, substitué comme le groupe Het ci-dessus et
un acyle seul ou en combinaison étant un groupe dérivé d'un acide alcanoïque ayant jusqu'à 7 atomes de C ou d'un acide carboxylique aromatique et
un alkyle seul ou en combinaison représentant un groupe alkyle, à chaîne droite ou ramifiée, ayant jusqu'à 7 atomes de C,
ainsi que les sels pharmaceutiquement utilisables des composés acides de formule I avec les bases et des composés basiques de formule I avec les acides.

2. Composés selon le revendication 1 où R¹ représente l'hydrogène, un alkyle, un aralkyle, un alcoxyalkyle, un hydroxyalkyle, un haloalkyle, un aminoalkyle, un monoalkylaminoalkyle, un dialkylaminoalkyle, un azidoalkyle, un acylaminoalkyle, un alkylsulfonylaminoalkyle, un arylsulfonylaminoalkyle, un mercaptoalkyle, un alkylthioalkyle, un glucopyranosyle, un carboxyalkyle, un alcoxycarbonylalkyle, un aminocarbonylalkyle, un hydroxyalkylthioalkyle, un mercaptoalkylthioalkyle, un arylthioalkyle ou un carboxyalkylthioalkyle.

3. Composés selon la revendication 2 où R¹ est un alkyle ou un aminoalkyle.

4. Composés selon la revendication 1 où R¹ est un isothiocyanatoalkyle ou un groupe de formule (b) dans laquelle T est S, V est NH et Z est un amino ou dans laquelle T représente NH, V représente NH ou NNO₂ et Z représente un amino.

5. Composés selon l'une des revendications 1 à 4 où R² est l'hydrogène.

6. Composés selon l'une des revendications 1 à 5 où R³ est un phényle monosubstitué par un halogène, un alkyle, un alcoxy, un haloalkyle, un nitro, un amino, un alkylthio, un alkylsulfinyle ou un alkylsulfonyle.

7. Composés selon l'une des revendications 1 à 5 où R³ est un groupe de formule 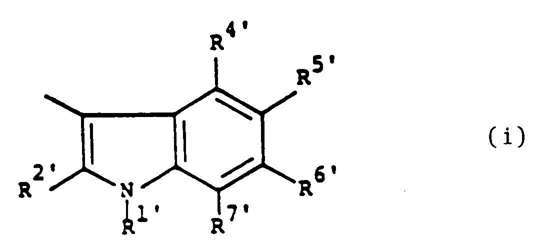 où R¹', R²', R⁴', R⁵', R⁶' et R⁷' ont l'une des significations de R¹, R², R⁴, R⁵, R⁶ ou R⁷ dans la revendication 1.

8. Composés selon l'une des revendications 1 à 7 où R⁴, R⁵, R⁶ et R⁷ sont l'hydrogène.

9. Composés selon l'une des revendications 1 à 8 où R¹ est le méthyle, le 3-aminopropyle, le 3-isothiocyanatopropyle ou un groupe de formule (b) dans laquelle T est S, V est NH, Z est un amino et n est le nombre 3 ou dans laquelle T est NH, V est NH ou NNO₂, Z est un amino et n est le nombre 3, R² est l'hydrogène, R³ est un phényle monosubstitué par le chlore, le brome, le méthyle, le méthoxy, le trifluorométhyle, le nitro, l'amino, le méthylsulfinyle ou le méthylsulfonyle ou un groupe de formule (i) selon la revendication 7 dans laquelle R¹' est le méthyle et R²', R⁴', R⁵', R⁶' et R⁷' sont l'hydrogène et R⁴, R⁵, R⁶ et R⁷ sont l'hydrogène.

10. Un pyrrole provenant du groupe des composés suivants :
la 3-(2-chlorophényl)-4-(1-méthyl-3-indolyl)-1H-pyrrol-2,5-dione,
la 3-(1-méthyl-3-indolyl)-4-(2-nitrophényl)-1H-pyrrol-2,5-dione,
la 3,4-bis(1-méthyl-3-indolyl)-1H-pyrrol-2,5-dione,
la 3-[1-(3-aminopropyl)-3-indolyl]-4-(1-méthyl-3-indolyl)-1H-pyrrol-2,5-dione,
la 3-[1-[3-(amidinothio)propyl]-3-indolyl]-4-(1-méthyl-3-indolyl)-1H-pyrrol-2,5-dione,
la 3-(1-méthyl-3-indolyl)-4-[1-[3-(2-nitroguanidino)-propyl]-3-indolyl]-1H-pyrrol-2,5-dione et
la 3-[1-(3-isothiocyanatopropyl)-3-indolyl]-4-(1-méthyl-3-indolyl)-1H-pyrrol-2,5-dione.

11. Composés selon l'une des revendications 1 à 10 destinés à être utilisés comme substance thérapeutiquement active, présentant, en particulier, une activité antiinflammatoire, immunologique, broncho-pulmonaire et cardio-vasculaire.

12. Un médicament, en particulier un médicament antiinflammatoire, immunologique, broncho-pulmonaire ou cardio-vasculaire, contenant un composé selon l'une des revendications 1 à 10 et un support thérapeutiquement inerte.

13. Utilisation d'un composé selon l'une des revendications 1 à 10 pour la préparation d'un médicament contre les maladies inflammatoire, immunologique, broncho-pulmonaire ou cardio-vasculaire.

14. Procédé pour la préparation des pyrroles selon l'une des revendications 1 à 10, caractérisé
(a) en ce que, pour la préparation d'un composé de formule I dans laquelle X et Y sont O, l'on fait réagir un composé de formule 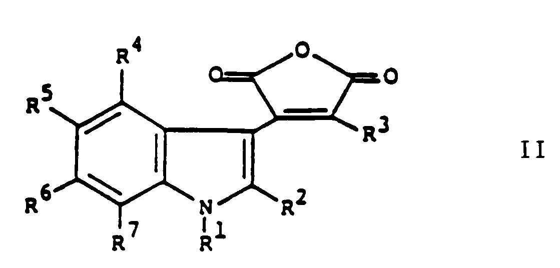 où R¹ à R⁷ ont la signification donnée dans la revendication 1,
avec l'ammoniac sous pression ou avec l'hexaméthyldisilazane et le méthanol ou
(b) en ce que, pour la préparation d'un composé de formule I dans laquelle R¹ est l'hydrogène et X et Y sont O, l'on fait réagir un composé de formule 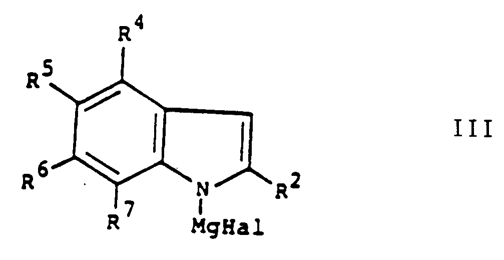 où R² à R⁷ ont la signification donnée dans la revendication 1 et Hal est un halogène,
avec un composé de formule 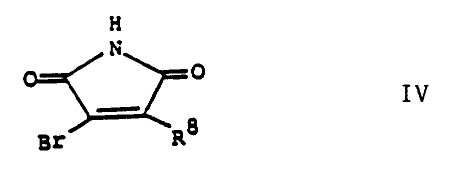 où R⁸ a la même signification que R³ dans la revendication 1 ou est le brome, ou
(c) en ce que, pour la préparation d'un composé de formule I dans laquelle R³ est le 1-benzimidazolyle et X et Y sont O, l'on fait réagir un composé de formule 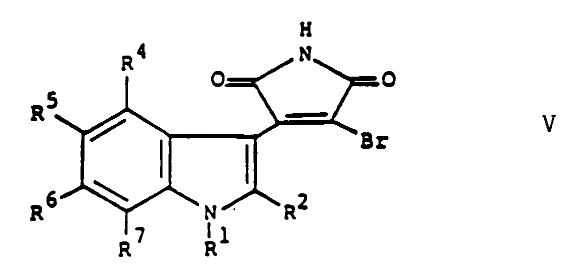 où R¹ à R⁷ ont la signification donnée dans la revendication 1,
avec un dérivé de métal alcalin ou benzimidazole, ou
(d) en ce que, pour la préparation d'un composé de formule I dans laquelle l'un des symboles X et Y est O et l'autre est S, l'on fait réagir un composé de formule I dans laquelle X et Y sont O, avec un agent de sulfuration, ou
(e) en ce que, pour la préparation d'un composé de formule I dans laquelle l'un des symboles X et Y est O et l'autre est (H,OH), l'on réduit un composé de formule I dans laquelle X et Y sont O, avec un hydrure métallique complexe, ou
(f) en ce que, pour la préparation d'un composé de formule I dans laquelle l'un des symboles X et Y est O et l'autre est (H,H), l'on hydrogène catalytiquement un composé de formule I dans laquelle l'un des symboles X et Y est O et l'autre est (H,OH), ou
(g) en ce que, pour la préparation d'un composé de formule I dans laquelle R¹ est un alkyle, un aralkyle, un alcoxyalkyle ou un hydroxyalkyle, l'on substitue en N de façon correspondante, un composé de formule I dans laquelle R¹ est l'hydrogène et
(h) en ce que, si désiré, l'on transforme fonctionnellement un substituant réactif présent dans un composé de formule I et
(i) en ce que, si désiré, l'on transforme un composé de formule I acide en un sel pharmaceutiquement utilisable avec une base ou un composé de formule I basique en un sel pharmaceutiquement utilisable avec un acide.

15. Un procédé pour la préparation d'un médicament, en particulier d'un médicament antiinflammatoire, immunologique, broncho-pulmonaire ou cardio-vasculaire, ledit procédé étant caractérisé en ce que l'on met sous une forme de présentation galénique un composé de formule I selon la revendication 1 ou un sel pharmaceutiquement utilisable d'un composé de formule I acide avec une base ou un composé de formule I basique avec un acide, associé à un support thérapeutiquement inerte et, si désiré, à une ou plusieurs autres substances thérapeutiquement actives.

## Revendications (Revendications pour l'(les) Etat(s) contractant(s) suivant(s): ES, GR)

1. Procédé pour la préparation des pyrroles de formule générale 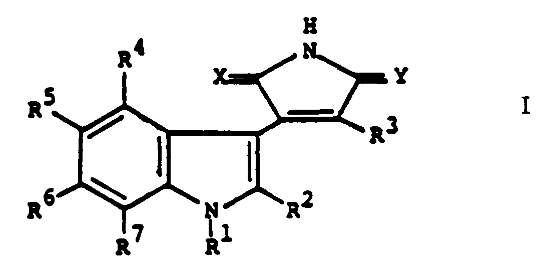 où
R¹ représente l'hydrogène, un alkyle, un aryle, un aralkyle, un alcoxyalkyle, un hydroxyalkyle, un haloalkyle, un aminoalkyle, un monoalkylaminoalkyle, un dialkylaminoalkyle, un trialkylaminoalkyle, un aminoalkylaminoalkyle, un azidoalkyle, un acylaminoalkyle, un acylthioalkyle, un alkylsulfonylaminoalkyle, un arylsulfonylaminoalkyle, un mercaptoalkyle, un alkylthioalkyle, un alkylsulfinylalkyle, un alkylsulfonylalkyle, un alkylsulfonyloxyalkyle, un alkylcarbonyloxyalkyle, un cyanoalkyle, un amidinoalkyle, un isothiocyanatoalkyle, un glucopyranosyle, un carboxyalkyle, un alcoxycarbonylalkyle, un aminocarbonylalkyle, un hydroxyalkylthioalkyle, un mercaptoalkylthioalkyle, un arylthioalkyle ou un carboxyalkylthioalkyle ou un groupe de formules 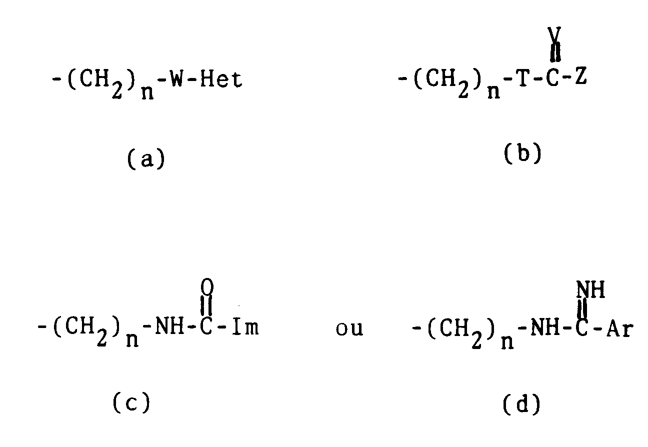 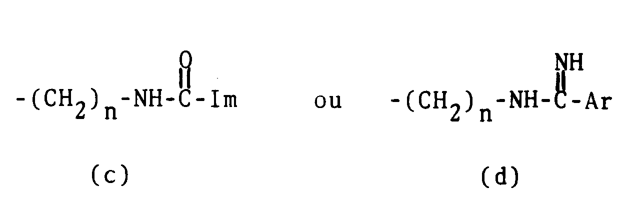 dans lesquelles
Het représente un groupe hétérocyclique,
W NH, S ou une liaison,
T NH ou S,
V O, S, NH, NNO₂, NCN ou CHNO₂,
Z un alkylthio, un amino, un monoalkylamino ou un dialkylamino,
Im le 1-imidazolyle,
Ar un aryle et
n un nombre allant de 2 à 6 ;
R² l'hydrogène, un alkyle, un aralkyle, un alcoxyalkyle, un hydroxyalkyle, un haloalkyle, un aminoalkyle, un monoalkylaminoalkyle, un dialkylaminoalkyle, un acylaminoalkyle, un alkylsulfonylaminoalkyle, un arylsulfonylaminoalkyle, un mercaptoalkyle, un alkylthioalkyle, un carboxyalkyle, un alcoxycarbonylalkyle, un aminocarbonylalkyle, un alkylthio ou un alkylsulfinyle ;
R³ représente un groupe aromatique carbocyclique ou hétérocyclique ;
R⁴, R⁵, R⁶ et R⁷ représentent, indépendamment les uns des autres, l'hydrogène, un halogène, un alkyle, un hydroxy, un alcoxy, un aryloxy, un haloalkyle, un nitro, un amino, un acylamino, un monoalkylamino, un dialkylamino, un alkylthio, un alkylsulfinyle ou un alkylsulfonyle ;
et l'un des symboles X et Y est O et l'autre O, S, (H,OH) ou (H,H),
sous réserve que R¹ est différent de l'hydrogène, lorsque R² est l'hydrogène, R³ est le 3-indolyle ou le 6-hydroxy-3-indolyle, R⁴, R⁵ et R⁷ sont l'hydrogène, R⁶ est l'hydrogène ou un hydroxy et X et Y sont tous les deux O ainsi que lorsque R² est l'hydrogène, R³ est le 3-indolyle, R⁴, R⁵, R⁶ et R⁷ sont l'hydrogène, X est (H,H) et Y est O ;
un groupe hétérocyclique Het signifiant un groupe hétérocyclique à 5 ou 6 chaînons, saturé, partiellement insaturé ou aromatique, contenant, si désiré, un noyau benzénique condensé et étant non substitué ou substitué par un ou plusieurs substituants provenant du groupe constitué par un halogène, un alkyle, un hydroxy, un alcoxy, un haloalkyle, un nitro, un amino, un acylamino, un mono- ou dialkylamino, un alkylthio, un alkylsulfinyle et un alkylsulfonyle,
un groupe R³ aromatique hétérocyclique étant défini comme le groupe Het aromatique hétérocyclique ci-dessus et
un groupe R³ aromatique carbocyclique représentant le phényle ou le naphtyle, étant si désiré, substitué comme le groupe Het ci-dessus et
un acyle seul ou en combinaison étant un groupe dérivé d'un acide alcanoïque ayant jusqu'à 7 atomes de C ou d'un acide carboxylique aromatique et
un alkyle seul ou en combinaison représentant un groupe alkyle, à chaîne droite ou ramifiée, ayant judqu'à 7 atomes de C,
ainsi que des sels pharmaceutiquement utilisables des composés acides de formule I avec les bases et des composés basiques de formule I avec les acides, caractérisé
(a) en ce que, pour la préparation d'un composé de formule I dans laquelle X et Y sont O, l'on fait réagir un composé de formule 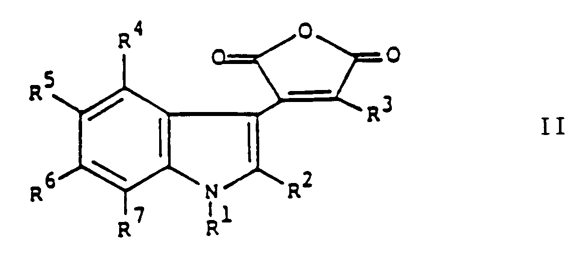 où R¹ à R⁷ ont la signification donnée ci-dessus,
avec l'ammoniac sous pression ou avec l'hexaméthyldisilazane et le méthanol, ou
(b) en ce que, pour la préparation d'un composé de formule I dans laquelle R¹ est l'hydrogène et X et Y sont O, l'on fait réagir un composé de formule 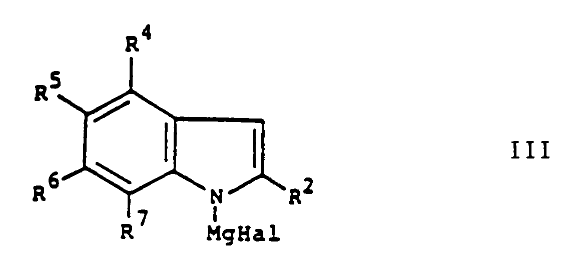 où R² à R⁷ ont la signification donnée ci-dessus et Hal est un halogène,
avec un composé de formule 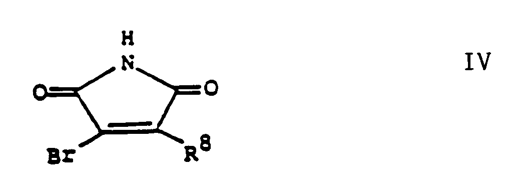 où R⁸ a la même signification que R³ ou est le brome, ou
(c) en ce que, pour la préparation d'un composé de formule I dans laquelle R³ est le 1-benzimidazolyle et X et Y sont O, l'on fait réagir un composé de formule 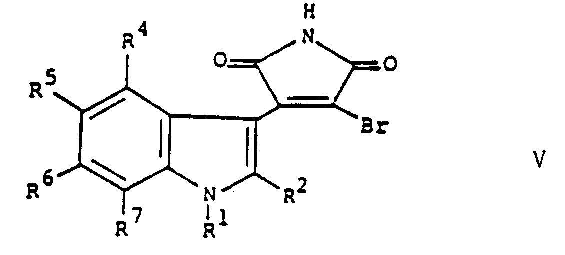 où R¹ à R⁷ ont la signification donnée ci-dessus,
avec un dérivé de métal alcalin du benzimidazole, ou
(d) en ce que, pour la préparation d'un composé de formule I dans laquelle l'un des symboles X et Y est O et l'autre est S, l'on fait réagir un composé de formule I dans laquelle X et Y sont O, avec un agent de sulfuration, ou
(e) en ce que, pour la préparation d'un composé de formule I dans laquelle l'un des symboles X et Y est O et l'autre est (H,OH), l'on réduit un composé de formule I dans laquelle X et Y sont O, avec un hydrure métallique complexe, ou
(f) en ce que, pour la préparation d'un composé de formule I dans laquelle l'un des symboles X et Y est O et l'autre est (H,H), l'on hydrogène catalytiquement un composé de formule I dans laquelle l'un des symboles X et Y est O et l'autre est (H,OH), ou
(g) en ce que, pour la préparation d'un composé de formule I dans laquelle R¹ est un alkyle, un aralkyle, un alcoxyalkyle ou un hydroxyalkyle, l'on substitue en N de façon correspondante, un composé de formule I dans laquelle R¹ est l'hydrogène, et
(h) en ce que, si désiré, l'on transforme fonctionnellement un substituant réactif présent dans un composé de formule I, et
(i) en ce que, si désiré, l'on transforme un composé de formule I acide en un sel pharmaceutiquement utilisable avec une base ou un composé de formule I basique en un sel pharmaceutiquement utilisable avec un acide.

2. Procédé selon la revendication 1 où R¹ représente l'hydrogène, un alkyle, un aralkyle, un alcoxyalkyle, un hydroxyalkyle, un haloalkyle, un aminoalkyle, un monoalkylaminoalkyle, un dialkylaminoalkyle, un azidoalkyle, un acylaminoalkyle, un alkylsulfonylaminoalkyle, un arylsulfonylaminoalkyle, un mercaptoalkyle, un alkylthioalkyle, un glucopyranosyle, un carboxyalkyle, un alcoxycarbonylalkyle, un aminocarbonylalkyle, un hydroxyalkylthioalkyle, un mercaptoalkylthioalkyle, un arylthioalkyle ou un carboxyalkylthioalkyle.

3. Procédé selon la revendication 2 où R¹ est un alkyle ou un aminoalkyle.

4. Procédé selon la revendication 1 où R¹ est un isothiocyanatoalkyle ou un groupe de formule (b) dans laquelle T est S, V est NH et Z est un amino ou dans laquelle T représente NH, V représente NH ou NNO₂ et Z représente un amino.

5. Procédé selon l'une des revendications 1 à 4 où R² est l'hydrogène.

6. Procédé selon l'une des revendications 1 à 5 où R³ est un phényle monosubstitué par un halogène, un alkyle, un alcoxy, un haloalkyle, un nitro, un amino, un alkylthio, un alkylsulfinyle ou un alkylsulfonyle.

7. Procédé selon l'une des revendications 1 à 5 où R³ est un groupe de formule 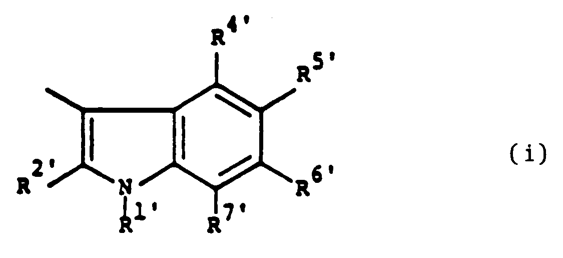 où R¹', R²', R⁴', R⁵', R⁶' et R⁷' ont l'une des significations de R¹, R², R⁴, R⁵, R⁶ ou R⁷ dans la revendication 1.

8. Procédé selon l'une des revendications 1 à 7 où R⁴, R⁵, R⁶ et R⁷ sont l'hydrogène.

9. Procédé selon l'une des revendications 1 à 8 où R¹ est le méthyle, le 3-aminopropyle, le 3-isothiocyanatopropyle ou un groupe de formule (b) dans laquelle T est S, V est NH, Z est un amino et n est le nombre 3 ou dans laquelle T est NH, V est NH ou NNO₂, Z est un amino et n est le nombre 3, R² est l'hydrogène, R³ est un phényle monosubstitué par le chlore, le brome, le méthyle, le méthoxy, le trifluorométhyle, le nitro, l'amino, le méthylsulfinyle ou le méthylsulfonyle ou un groupe de formule (i) selon la revendication 7 dans laquelle R¹' est le méthyle et R²', R⁴', R⁵', R⁶' et R⁷' sont l'hydrogène et R⁴, R⁵, R⁶ et R⁷ sont l'hydrogène.

10. Procédé selon la revendication 1 pour la préparation d'un pyrrole provenant du groupe des composés suivants :
la 3-(2-chlorophényl)-4-(1-méthyl-3-indolyl)-1H-pyrrol-2,5-dione,
la 3-(1-méthyl-3-indolyl)-4-(2-nitrophényl)-1H-pyrrol-2,5-dione,
la 3,4-bis(1-méthyl-3-indolyl)-1H-pyrrol-2,5-dione,
la 3-[1-(3-aminopropyl)-3-indolyl]-4-(1-méthyl-3-indolyl)-1H-pyrrol-2,5-dione,
la 3-[1-[3-(amidinothio)propyl]-3-indolyl]-4-(1-méthyl-3-indolyl)-1H-pyrrol-2,5-dione,
la 3-(1-méthyl-3-indolyl)-4-[1-[3-(2-nitroguanidino)-propyl]-3-indolyl]-1H-pyrrol-2,5-dione, et
la 3-[1-(3-isothiocyanatopropyl)-3-indolyl]-4-(1-méthyl-3-indolyl)-1H-pyrrol-2,5-dione.

11. Procédé selon la revendication 1, caractérisé
a) en ce que l'on fait réagir le composé de formule II avec de l'ammoniaque aqueux ou avec l'hexaméthyldisilazane et le méthanol en présence d'un solvant organique inerte à une température comprise entre environ 100 et environ 150°C ou entre environ 40 et environ 110°C ou
b) en ce que l'on effectue la réaction de Grignard entre les composés de formules III et IV dans un solvant organique inerte à une température allant jusqu'au reflux, ou
c) en ce que l'on fait réagir le composé de formule V avec un dérivé de métal alcalin du benzimidazole dans un solvant organique inerte à une température comprise entre environ 45 et environ 95°C, ou
d) en ce que l'on utilise, pour la sulfuration d'un composé de formule I, le pentasulfure de phosphore ou le réactif de Lawesson ou le réactif de Davy dans un solvant organique inerte à une température allant jusqu'au reflux, ou
e) en ce que l'on utilise l'hydrure de lithium et d'aluminium, l'hydrure de diisobutylaluminium ou le dihydro-bis(2-méthoxyéthoxy)aluminate de sodium comme hydrure métallique complexe, ou
f) en ce que l'on utilise un catalyseur de palladium ou de platine comme catalyseur d'hydrogénation, ou
g) en ce que l'on effectue la substitution en N à l'aide d'un oxyde d'alkylène, d'un acétal dialkylique aldéhyde ou d'un halogénure d'alkyle ou d'aralkyle.

12. Utilisation d'un composé selon l'une des revendications 1 à 10 pour la préparation d'un médicament contre les maladies inflammatoire, immunologique, broncho-pulmonaire ou cardio-vasculaire.

13. Procédé pour la préparation d'un médicament en particulier d'un médicament antiinflammatoire, immunologique, broncho-pulmonaire ou cardio-vasculaire, ledit procédé étant caractérisé en ce que l'on met sous une forme de présentation galénique un composé de formule I selon la revendication 1 ou un sel pharmaceutiquement utilisable d'un composé de formule I acide avec une base ou un composé de formule I basique avec un acide, associé à un support thérapeutiquement inerte et, si désiré, à une ou plusieurs autres substances thérapeutiquement actives.
